(19) **Europäisches Patentamt / European Patent Office / Office européen des brevets**

(11) **EP 2 445 937 B1**

(12) **EUROPEAN PATENT SPECIFICATION**

(45) Date of publication and mention
of the grant of the patent:
**24.05.2017 Bulletin 2017/21**

(51) Int Cl.:
***C07K 16/46*** (2006.01)   ***A61P 31/00*** (2006.01)
***C07K 16/18*** (2006.01)   ***A61K 39/00*** (2006.01)

(21) Application number: **10792563.8**

(22) Date of filing: **22.06.2010**

(86) International application number:
**PCT/US2010/039448**

(87) International publication number:
**WO 2010/151526 (29.12.2010 Gazette 2010/52)**

(54) **BISPECIFIC ANTIBODIES THAT BIND TO COMPLEMENT PROTEINS**

BISPEZIFISCHE ANTIKÖRPER DIE AN KOMPLEMENT-PROTEINEN BINDEN

ANTICORPS BISPÉCIFIQUES SE LIANT AUX PROTÉINES DU COMPLÉMENT

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **23.07.2009 US 228001 P
23.06.2009 US 219644 P**

(43) Date of publication of application:
**02.05.2012 Bulletin 2012/18**

(73) Proprietor: **ALEXION PHARMACEUTICALS, INC.
New Haven CT 06510 (US)**

(72) Inventor: **TAMBURINI, Paul, P.
Kensington, CT 06037 (US)**

(74) Representative: **D Young & Co LLP
120 Holborn
London EC1N 2DY (GB)**

(56) References cited:
**EP-A2- 0 300 740**   **WO-A1-2010/011697**
**WO-A1-2010/030789**   **WO-A1-2010/057084**
**WO-A2-2006/063150**   **WO-A2-2006/063150**

• HILLMEN PETER ET AL: "The complement inhibitor eculizumab in paroxysmal nocturnal hemoglobinuria", NEW ENGLAND JOURNAL OF MEDICINE, MASSACHUSETTS MEDICAL SOCIETY, BOSTON, MA, US, vol. 355, no. 12, 21 September 2006 (2006-09-21), pages 1233-1243, XP002482088, ISSN: 1533-4406, DOI: 10.1056/NEJMOA061648

• GELDERMAN K A ET AL: "Inhibiting complement regulators in cancer immunotherapy with bispecific mAbs", EXPERT OPINION ON BIOLOGICAL THERAPY, ASHLEY, LONDON, GB, vol. 5, no. 12, 1 January 2005 (2005-01-01), pages 1593-1601, XP009110815, ISSN: 1471-2598, DOI: 10.1517/14712598.5.12.1593

EP 2 445 937 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

    <110> Alexion Pharmaceuticals, Inc.

    <120> Bispecific Antibodies that Bind to Complement Proteins

    <130> ALXN-149-WO1

    <140> To be determined
    <141> Concurrently Herewith

    <150> 61/219,644
    <151> 2009-06-23

    <150> 61/228,001
    <151> 2009-07-23

    <160> 27

    <170> PatentIn version 3.5

    <210> 1
    <211> 74
    <212> PRT
    <213> Homo sapiens

    <400> 1

```
Thr Leu Gln Lys Lys Ile Glu Glu Ile Ala Ala Lys Tyr Lys His Ser
1               5               10              15

Val Val Lys Lys Cys Cys Tyr Asp Gly Ala Cys Val Asn Asn Asp Glu
            20              25              30

Thr Cys Glu Gln Arg Ala Ala Arg Ile Ser Leu Gly Pro Arg Cys Ile
        35              40              45

Lys Ala Phe Thr Glu Cys Cys Val Val Ala Ser Gln Leu Arg Ala Asn
    50              55              60

Ile Ser His Lys Asp Met Gln Leu Gly Arg
65              70
```

<210> 2
<211> 20
<212> PRT
<213> Homo sapiens

<400> 2

```
Cys Cys Tyr Asp Gly Ala Cys Val Asn Asn Asp Glu Thr Cys Glu Gln
1               5               10              15

Arg Ala Ala Arg
            20
```

<210> 3
<211> 18
<212> PRT
<213> Homo sapiens

<400> 3

```
Lys Cys Cys Tyr Asp Gly Ala Cys Val Asn Asn Asp Glu Thr Cys Glu
1               5               10              15

Gln Arg
```

<210> 4
<211> 10
<212> PRT
<213> Homo sapiens

<400> 4

```
Val Asn Asn Asp Glu Thr Cys Glu Gln Arg
1               5               10
```

<210> 5
<211> 6
<212> PRT
<213> Homo sapiens

<400> 5

                        Val Asn Asn Asp Glu Thr
                        1               5


<210> 6
<211> 9
<212> PRT
<213> Homo sapiens

<400> 6

                    Ala Ala Arg Ile Ser Leu Gly Pro Arg
                    1               5


<210> 7
<211> 19
<212> PRT
<213> Homo sapiens

<400> 7

        Cys Cys Tyr Asp Gly Ala Cys Val Asn Asn Asp Glu Thr Cys Glu Gln
        1               5                   10                  15


        Arg Ala Ala


<210> 8
<211> 18
<212> PRT
<213> Homo sapiens

<400> 8

        Cys Cys Tyr Asp Gly Ala Cys Val Asn Asn Asp Glu Thr Cys Glu Gln
        1               5                   10                  15


        Arg Ala


<210> 9
<211> 17
<212> PRT
<213> Homo sapiens

<400> 9

```
Cys Cys Tyr Asp Gly Ala Cys Val Asn Asn Asp Glu Thr Cys Glu Gln
1               5               10              15

Arg
```

<210> 10
<211> 16
<212> PRT
<213> Homo sapiens

<400> 10

```
Cys Cys Tyr Asp Gly Ala Cys Val Asn Asn Asp Glu Thr Cys Glu Gln
1               5               10              15
```

<210> 11
<211> 15
<212> PRT
<213> Homo sapiens

<400> 11

```
Cys Cys Tyr Asp Gly Ala Cys Val Asn Asn Asp Glu Thr Cys Glu
1               5               10              15
```

<210> 12
<211> 19
<212> PRT
<213> Homo sapiens

<400> 12

```
Cys Tyr Asp Gly Ala Cys Val Asn Asn Asp Glu Thr Cys Glu Gln Arg
1               5               10              15

Ala Ala Arg
```

<210> 13
<211> 18
<212> PRT
<213> Homo sapiens

<400> 13

```
Tyr Asp Gly Ala Cys Val Asn Asn Asp Glu Thr Cys Glu Gln Arg Ala
1               5               10              15

Ala Arg
```

<210> 14
<211> 19

<212> PRT
<213> Homo sapiens

<400> 14

```
Cys Tyr Asp Gly Ala Cys Val Asn Asn Asp Glu Thr Cys Glu Gln Arg
1               5                   10              15

Ala Ala Arg
```

<210> 15
<211> 655
<212> PRT
<213> Homo sapiens

<400> 15

```
Gln Glu Gln Thr Tyr Val Ile Ser Ala Pro Lys Ile Phe Arg Val Gly
1               5                   10              15

Ala Ser Glu Asn Ile Val Ile Gln Val Tyr Gly Tyr Thr Glu Ala Phe
                20                  25                  30

Asp Ala Thr Ile Ser Ile Lys Ser Tyr Pro Asp Lys Lys Phe Ser Tyr
                35                  40                  45

Ser Ser Gly His Val His Leu Ser Ser Glu Asn Lys Phe Gln Asn Ser
                50                  55                  60
```

```
Ala Ile Leu Thr Ile Gln Pro Lys Gln Leu Pro Gly Gly Gln Asn Pro
65                  70              75                  80

Val Ser Tyr Val Tyr Leu Glu Val Val Ser Lys His Phe Ser Lys Ser
                85              90                  95

Lys Arg Met Pro Ile Thr Tyr Asp Asn Gly Phe Leu Phe Ile His Thr
            100             105             110

Asp Lys Pro Val Tyr Thr Pro Asp Gln Ser Val Lys Val Arg Val Tyr
        115             120                 125

Ser Leu Asn Asp Asp Leu Lys Pro Ala Lys Arg Glu Thr Val Leu Thr
        130             135             140

Phe Ile Asp Pro Glu Gly Ser Glu Val Asp Met Val Glu Glu Ile Asp
145             150             155             160

His Ile Gly Ile Ile Ser Phe Pro Asp Phe Lys Ile Pro Ser Asn Pro
                165             170                 175

Arg Tyr Gly Met Trp Thr Ile Lys Ala Lys Tyr Lys Glu Asp Phe Ser
            180             185             190

Thr Thr Gly Thr Ala Tyr Phe Glu Val Lys Glu Tyr Val Leu Pro His
        195             200                 205

Phe Ser Val Ser Ile Glu Pro Glu Tyr Asn Phe Ile Gly Tyr Lys Asn
    210             215             220

Phe Lys Asn Phe Glu Ile Thr Ile Lys Ala Arg Tyr Phe Tyr Asn Lys
225             230             235             240

Val Val Thr Glu Ala Asp Val Tyr Ile Thr Phe Gly Ile Arg Glu Asp
            245             250             255

Leu Lys Asp Asp Gln Lys Glu Met Met Gln Thr Ala Met Gln Asn Thr
            260             265             270

Met Leu Ile Asn Gly Ile Ala Gln Val Thr Phe Asp Ser Glu Thr Ala
        275             280             285

Val Lys Glu Leu Ser Tyr Tyr Ser Leu Glu Asp Leu Asn Asn Lys Tyr
        290             295             300

Leu Tyr Ile Ala Val Thr Val Ile Glu Ser Thr Gly Gly Phe Ser Glu
305             310             315             320

Glu Ala Glu Ile Pro Gly Ile Lys Tyr Val Leu Ser Pro Tyr Lys Leu
```

|     |     |     | 325 |     |     |     |     | 330 |     |     |     |     | 335 |     |     |
| --- | --- | --- | --- | --- | --- | --- | --- | --- | --- | --- | --- | --- | --- | --- | --- |

Asn Leu Val Ala Thr Pro Leu Phe Leu Lys Pro Gly Ile Pro Tyr Pro
            340              345              350

Ile Lys Val Gln Val Lys Asp Ser Leu Asp Gln Leu Val Gly Gly Val
            355              360              365

Pro Val Ile Leu Asn Ala Gln Thr Ile Asp Val Asn Gln Glu Thr Ser
    370              375              380

Asp Leu Asp Pro Ser Lys Ser Val Thr Arg Val Asp Asp Gly Val Ala
385              390              395              400

Ser Phe Val Leu Asn Leu Pro Ser Gly Val Thr Val Leu Glu Phe Asn
            405              410              415

Val Lys Thr Asp Ala Pro Asp Leu Pro Glu Glu Asn Gln Ala Arg Glu
            420              425              430

Gly Tyr Arg Ala Ile Ala Tyr Ser Ser Leu Ser Gln Ser Tyr Leu Tyr
            435              440              445

Ile Asp Trp Thr Asp Asn His Lys Ala Leu Leu Val Gly Glu His Leu
    450              455              460

Asn Ile Ile Val Thr Pro Lys Ser Pro Tyr Ile Asp Lys Ile Thr His
465              470              475              480

Tyr Asn Tyr Leu Ile Leu Ser Lys Gly Lys Ile Ile His Phe Gly Thr
            485              490              495

Arg Glu Lys Phe Ser Asp Ala Ser Tyr Gln Ser Ile Asn Ile Pro Val
            500              505              510

Thr Gln Asn Met Val Pro Ser Ser Arg Leu Leu Val Tyr Tyr Ile Val
            515              520              525

Thr Gly Glu Gln Thr Ala Glu Leu Val Ser Asp Ser Val Trp Leu Asn
    530              535              540

Ile Glu Glu Lys Cys Gly Asn Gln Leu Gln Val His Leu Ser Pro Asp
545              550              555              560

Ala Asp Ala Tyr Ser Pro Gly Gln Thr Val Ser Leu Asn Met Ala Thr
            565              570              575

Gly Met Asp Ser Trp Val Ala Leu Ala Ala Val Asp Ser Ala Val Tyr
            580              585              590

```
Gly Val Gln Arg Gly Ala Lys Lys Pro Leu Glu Arg Val Phe Gln Phe
        595             600             605

Leu Glu Lys Ser Asp Leu Gly Cys Gly Ala Gly Gly Gly Leu Asn Asn
    610             615             620

Ala Asn Val Phe His Leu Ala Gly Leu Thr Phe Leu Thr Asn Ala Asn
625             630             635             640

Ala Asp Asp Ser Gln Glu Asn Asp Glu Pro Cys Lys Glu Ile Leu
            645             650             655
```

<210> 16
<211> 925
<212> PRT
<213> Homo sapiens

<400> 16

```
Leu His Met Lys Thr Leu Leu Pro Val Ser Lys Pro Glu Ile Arg Ser
1           5               10              15

Tyr Phe Pro Glu Ser Trp Leu Trp Glu Val His Leu Val Pro Arg Arg
        20              25              30

Lys Gln Leu Gln Phe Ala Leu Pro Asp Ser Leu Thr Thr Trp Glu Ile
    35              40              45

Gln Gly Ile Gly Ile Ser Asn Thr Gly Ile Cys Val Ala Asp Thr Val
    50              55              60

Lys Ala Lys Val Phe Lys Asp Val Phe Leu Glu Met Asn Ile Pro Tyr
65              70              75              80

Ser Val Val Arg Gly Glu Gln Ile Gln Leu Lys Gly Thr Val Tyr Asn
            85              90              95

Tyr Arg Thr Ser Gly Met Gln Phe Cys Val Lys Met Ser Ala Val Glu
            100             105             110

Gly Ile Cys Thr Ser Glu Ser Pro Val Ile Asp His Gln Gly Thr Lys
            115             120             125

Ser Ser Lys Cys Val Arg Gln Lys Val Glu Gly Ser Ser Ser His Leu
    130             135             140

Val Thr Phe Thr Val Leu Pro Leu Glu Ile Gly Leu His Asn Ile Asn
145             150             155             160
```

```
Phe Ser Leu Glu Thr Trp Phe Gly Lys Glu Ile Leu Val Lys Thr Leu
            165             170             175

Arg Val Val Pro Glu Gly Val Lys Arg Glu Ser Tyr Ser Gly Val Thr
            180             185             190

Leu Asp Pro Arg Gly Ile Tyr Gly Thr Ile Ser Arg Arg Lys Glu Phe
            195             200             205

Pro Tyr Arg Ile Pro Leu Asp Leu Val Pro Lys Thr Glu Ile Lys Arg
210             215             220

Ile Leu Ser Val Lys Gly Leu Leu Val Gly Glu Ile Leu Ser Ala Val
225             230             235             240

Leu Ser Gln Glu Gly Ile Asn Ile Leu Thr His Leu Pro Lys Gly Ser
            245             250             255

Ala Glu Ala Glu Leu Met Ser Val Val Pro Val Phe Tyr Val Phe His
            260             265             270

Tyr Leu Glu Thr Gly Asn His Trp Asn Ile Phe His Ser Asp Pro Leu
            275             280             285

Ile Glu Lys Gln Lys Leu Lys Lys Lys Leu Lys Glu Gly Met Leu Ser
    290             295             300

Ile Met Ser Tyr Arg Asn Ala Asp Tyr Ser Tyr Ser Val Trp Lys Gly
305             310             315             320

Gly Ser Ala Ser Thr Trp Leu Thr Ala Phe Ala Leu Arg Val Leu Gly
            325             330             335

Gln Val Asn Lys Tyr Val Glu Gln Asn Gln Asn Ser Ile Cys Asn Ser
            340             345             350

Leu Leu Trp Leu Val Glu Asn Tyr Gln Leu Asp Asn Gly Ser Phe Lys
            355             360             365

Glu Asn Ser Gln Tyr Gln Pro Ile Lys Leu Gln Gly Thr Leu Pro Val
    370             375             380

Glu Ala Arg Glu Asn Ser Leu Tyr Leu Thr Ala Phe Thr Val Ile Gly
385             390             395             400

Ile Arg Lys Ala Phe Asp Ile Cys Pro Leu Val Lys Ile Asp Thr Ala
            405             410             415

Leu Ile Lys Ala Asp Asn Phe Leu Leu Glu Asn Thr Leu Pro Ala Gln
```

```
                    420                      425                      430

        Ser Thr Phe Thr Leu Ala Ile Ser Ala Tyr Ala Leu Ser Leu Gly Asp
                435                 440                 445

        Lys Thr His Pro Gln Phe Arg Ser Ile Val Ser Ala Leu Lys Arg Glu
        450                 455                 460

        Ala Leu Val Lys Gly Asn Pro Pro Ile Tyr Arg Phe Trp Lys Asp Asn
        465                 470                 475                 480

        Leu Gln His Lys Asp Ser Ser Val Pro Asn Thr Gly Thr Ala Arg Met
                        485                 490                 495

        Val Glu Thr Thr Ala Tyr Ala Leu Leu Thr Ser Leu Asn Leu Lys Asp
                500                 505                 510

        Ile Asn Tyr Val Asn Pro Val Ile Lys Trp Leu Ser Glu Glu Gln Arg
                515                 520                 525

        Tyr Gly Gly Gly Phe Tyr Ser Thr Gln Asp Thr Ile Asn Ala Ile Glu
            530                 535                 540

        Gly Leu Thr Glu Tyr Ser Leu Leu Val Lys Gln Leu Arg Leu Ser Met
        545                 550                 555                 560

        Asp Ile Asp Val Ser Tyr Lys His Lys Gly Ala Leu His Asn Tyr Lys
                        565                 570                 575

        Met Thr Asp Lys Asn Phe Leu Gly Arg Pro Val Glu Val Leu Leu Asn
                580                 585                 590

        Asp Asp Leu Ile Val Ser Thr Gly Phe Gly Ser Gly Leu Ala Thr Val
                595                 600                 605

        His Val Thr Thr Val Val His Lys Thr Ser Thr Ser Glu Glu Val Cys
            610                 615                 620

        Ser Phe Tyr Leu Lys Ile Asp Thr Gln Asp Ile Glu Ala Ser His Tyr
        625                 630                 635                 640

        Arg Gly Tyr Gly Asn Ser Asp Tyr Lys Arg Ile Val Ala Cys Ala Ser
                        645                 650                 655

        Tyr Lys Pro Ser Arg Glu Glu Ser Ser Ser Gly Ser Ser His Ala Val
                    660                 665                 670

        Met Asp Ile Ser Leu Pro Thr Gly Ile Ser Ala Asn Glu Glu Asp Leu
                675                 680                 685
```

39

```
Lys Ala Leu Val Glu Gly Val Asp Gln Leu Phe Thr Asp Tyr Gln Ile
    690             695             700

Lys Asp Gly His Val Ile Leu Gln Leu Asn Ser Ile Pro Ser Ser Asp
705             710             715                 720

Phe Leu Cys Val Arg Phe Arg Ile Phe Glu Leu Phe Glu Val Gly Phe
                725             730             735

Leu Ser Pro Ala Thr Phe Thr Val Tyr Glu Tyr His Arg Pro Asp Lys
            740             745             750

Gln Cys Thr Met Phe Tyr Ser Thr Ser Asn Ile Lys Ile Gln Lys Val
        755             760             765

Cys Glu Gly Ala Ala Cys Lys Cys Val Glu Ala Asp Cys Gly Gln Met
    770             775             780

Gln Glu Glu Leu Asp Leu Thr Ile Ser Ala Glu Thr Arg Lys Gln Thr
785             790             795                 800

Ala Cys Lys Pro Glu Ile Ala Tyr Ala Tyr Lys Val Ser Ile Thr Ser
            805             810             815

Ile Thr Val Glu Asn Val Phe Val Lys Tyr Lys Ala Thr Leu Leu Asp
            820             825             830

Ile Tyr Lys Thr Gly Glu Ala Val Ala Glu Lys Asp Ser Glu Ile Thr
        835             840             845

Phe Ile Lys Lys Val Thr Cys Thr Asn Ala Glu Leu Val Lys Gly Arg
    850             855             860

Gln Tyr Leu Ile Met Gly Lys Glu Ala Leu Gln Ile Lys Tyr Asn Phe
865             870             875                 880

Ser Phe Arg Tyr Ile Tyr Pro Leu Asp Ser Leu Thr Trp Ile Glu Tyr
            885             890             895

Trp Pro Arg Asp Thr Thr Cys Ser Ser Cys Gln Ala Phe Leu Ala Asn
            900             905             910

Leu Asp Glu Phe Ala Glu Asp Ile Phe Leu Asn Gly Cys
        915             920             925
```

<210> 17

<211> 350
<212> PRT
<213> Homo sapiens

<400> 17

```
Met Asn Ser Phe Asn Tyr Thr Thr Pro Asp Tyr Gly His Tyr Asp Asp
1               5               10              15

Lys Asp Thr Leu Asp Leu Asn Thr Pro Val Asp Lys Thr Ser Asn Thr
        20              25              30

Leu Arg Val Pro Asp Ile Leu Ala Leu Val Ile Phe Ala Val Val Phe
        35              40              45

Leu Val Gly Val Leu Gly Asn Ala Leu Val Val Trp Val Thr Ala Phe
    50              55              60

Glu Ala Lys Arg Thr Ile Asn Ala Ile Trp Phe Leu Asn Leu Ala Val
65              70              75              80

Ala Asp Phe Leu Ser Cys Leu Ala Leu Pro Ile Leu Phe Thr Ser Ile
            85              90              95

Val Gln His His His Trp Pro Phe Gly Gly Ala Ala Cys Ser Ile Leu
            100             105             110

Pro Ser Leu Ile Leu Leu Asn Met Tyr Ala Ser Ile Leu Leu Leu Ala
        115             120             125

Thr Ile Ser Ala Asp Arg Phe Leu Leu Val Phe Lys Pro Ile Trp Cys
    130             135             140

Gln Asn Phe Arg Gly Ala Gly Leu Ala Trp Ile Ala Cys Ala Val Ala
145             150             155             160

Trp Gly Leu Ala Leu Leu Leu Thr Ile Pro Ser Phe Leu Tyr Arg Val
            165             170             175

Val Arg Glu Glu Tyr Phe Pro Pro Lys Val Leu Cys Gly Val Asp Tyr
            180             185             190

Ser His Asp Lys Arg Arg Glu Arg Ala Val Ala Ile Val Arg Leu Val
            195             200             205

Leu Gly Phe Leu Trp Pro Leu Leu Thr Leu Thr Ile Cys Tyr Thr Phe
    210             215             220

Ile Leu Leu Arg Thr Trp Ser Arg Arg Ala Thr Arg Ser Thr Lys Thr
225             230             235             240
```

42

```
Leu Lys Val Val Val Ala Val Val Ala Ser Phe Phe Ile Phe Trp Leu
            245             250             255

Pro Tyr Gln Val Thr Gly Ile Met Met Ser Phe Leu Glu Pro Ser Ser
            260             265             270

Pro Thr Phe Leu Leu Leu Asn Lys Leu Asp Ser Leu Cys Val Ser Phe
            275             280             285

Ala Tyr Ile Asn Cys Cys Ile Asn Pro Ile Ile Tyr Val Val Ala Gly
    290             295             300

Gln Gly Phe Gln Gly Arg Leu Arg Lys Ser Leu Pro Ser Leu Leu Arg
305             310             315             320

Asn Val Leu Thr Glu Glu Ser Val Val Arg Glu Ser Lys Ser Phe Thr
            325             330             335

Arg Ser Thr Val Asp Thr Met Ala Gln Lys Thr Gln Ala Val
            340             345             350
```

<210> 18
<211> 16
<212> PRT
<213> Homo sapiens

<400> 18

```
Ser Ile Val Gln His His His Trp Pro Phe Gly Gly Ala Ala Cys Ser
1               5               10              15
```

<210> 19
<211> 32
<212> PRT
<213> Homo sapiens

<400> 19

```
Arg Val Val Arg Glu Glu Tyr Phe Pro Pro Lys Val Leu Cys Gly Val
1               5               10              15

Asp Tyr Ser His Asp Lys Arg Arg Glu Arg Ala Val Ala Ile Val Arg
            20              25              30
```

<210> 20
<211> 19
<212> PRT
<213> Homo sapiens

<400> 20

43

```
Met Ser Phe Leu Glu Pro Ser Ser Pro Thr Phe Leu Leu Leu Asn Lys
1               5               10                  15

Leu Asp Ser
```

<210> 21
<211> 29
<212> PRT
<213> Homo sapiens

<400> 21

```
Met Asn Ser Phe Asn Tyr Thr Thr Pro Asp Tyr Gly His Tyr Asp Asp
1               5               10                  15

Lys Asp Thr Leu Asp Leu Asn Thr Pro Val Asp Lys Thr
            20              25
```

<210> 22
<211> 20
<212> PRT
<213> Homo sapiens

<400> 22

```
Asp Tyr Gly His Tyr Asp Asp Lys Asp Thr Leu Asp Leu Asn Thr Pro
1               5               10                  15

Val Asp Lys Thr
            20
```

<210> 23
<211> 337
<212> PRT
<213> Homo sapiens

<400> 23

```
Met Gly Asn Asp Ser Val Ser Tyr Glu Tyr Gly Asp Tyr Ser Asp Leu
1               5               10                  15

Ser Asp Arg Pro Val Asp Cys Leu Asp Gly Ala Cys Leu Ala Ile Asp
            20              25                  30

Pro Leu Arg Val Ala Pro Leu Pro Leu Tyr Ala Ala Ile Phe Leu Val
            35              40                  45

Gly Val Pro Gly Asn Ala Met Val Ala Trp Val Ala Gly Lys Val Ala
            50              55                  60
```

```
Arg Arg Arg Val Gly Ala Thr Trp Leu Leu His Leu Ala Val Ala Asp
65              70              75                      80

Leu Leu Cys Cys Leu Ser Leu Pro Ile Leu Ala Val Pro Ile Ala Arg
            85              90                      95

Gly Gly His Trp Pro Tyr Gly Ala Val Gly Cys Arg Ala Leu Pro Ser
            100             105             110

Ile Ile Leu Leu Thr Met Tyr Ala Ser Val Leu Leu Leu Ala Ala Leu
        115             120             125

Ser Ala Asp Leu Cys Phe Leu Ala Leu Gly Pro Ala Trp Trp Ser Thr
    130             135             140

Val Gln Arg Ala Cys Gly Val Gln Val Ala Cys Gly Ala Ala Trp Thr
145             150             155             160

Leu Ala Leu Leu Leu Thr Val Pro Ser Ala Ile Tyr Arg Arg Leu His
            165             170             175

Gln Glu His Phe Pro Ala Arg Leu Gln Cys Val Val Asp Tyr Gly Gly
            180             185             190

Ser Ser Ser Thr Glu Asn Ala Val Thr Ala Ile Arg Phe Leu Phe Gly
    195             200             205

Phe Leu Gly Pro Leu Val Ala Val Ala Ser Cys His Ser Ala Leu Leu
    210             215             220

Cys Trp Ala Ala Arg Arg Cys Arg Pro Leu Gly Thr Ala Ile Val Val
225             230             235             240

Gly Phe Phe Val Cys Trp Ala Pro Tyr His Leu Leu Gly Leu Val Leu
            245             250             255

Thr Val Ala Ala Pro Asn Ser Ala Leu Leu Ala Arg Ala Leu Arg Ala
            260             265             270

Glu Pro Leu Ile Val Gly Leu Ala Leu Ala His Ser Cys Leu Asn Pro
    275             280             285

Met Leu Phe Leu Tyr Phe Gly Arg Ala Gln Leu Arg Arg Ser Leu Pro
    290             295             300

Ala Ala Cys His Trp Ala Leu Arg Glu Ser Gln Gly Gln Asp Glu Ser
305             310             315             320

Val Asp Ser Lys Lys Ser Thr Ser His Asp Leu Val Ser Glu Met Glu
```

45

                    325                     330                     335

        Val

<210> 24
<211> 16
<212> PRT
<213> Homo sapiens

<400> 24

        Pro Ile Ala Arg Gly Gly His Trp Pro Tyr Gly Ala Val Gly Cys Arg
        1               5               10              15

<210> 25
<211> 32
<212> PRT
<213> Homo sapiens

<400> 25

        Arg Arg Leu His Gln Glu His Phe Pro Ala Arg Leu Gln Cys Val Val
        1               5               10              15

        Asp Tyr Gly Gly Ser Ser Ser Thr Glu Asn Ala Val Thr Ala Ile Arg
                    20              25              30

<210> 26
<211> 18
<212> PRT
<213> Homo sapiens

<400> 26

        Leu Thr Val Ala Ala Pro Asn Ser Ala Leu Leu Ala Arg Ala Leu Arg
        1               5               10              15

        Ala Glu

<210> 27
<211> 35
<212> PRT
<213> Homo sapiens

<400> 27

```
Met Gly Asn Asp Ser Val Ser Tyr Glu Tyr Gly Asp Tyr Ser Asp Leu
1               5               10              15

Ser Asp Arg Pro Val Asp Cys Leu Asp Gly Ala Cys Leu Ala Ile Asp
            20              25              30

Pro Leu Arg
            35
```

1

1

**Description**

Cross-Reference to Related Applications

This application claims the benefit of U.S. provisional patent application serial nos.: 61/219,644, filed on June 23, 2009, and 61/228,001, filed on July 23, 2009.

Sequence Listing

The instant application contains a Sequence Listing which has been submitted via EFS-Web and is hereby incorporated by reference in its entirety. Said ASCII copy, created on June 22, 2010, is named ALXN149WO1.txt, and is 25,951 bytes bytes in size.

Technical Field

The field of the invention is medicine, immunology, molecular biology, and protein chemistry.

Background

The complement system acts in conjunction with other immunological systems of the body to defend against intrusion of cellular and viral pathogens. There are at least 25 complement proteins, which are found as a complex collection of plasma proteins and membrane cofactors. The plasma proteins make up about 10% of the globulins in vertebrate serum. Complement components achieve their immune defensive functions by interacting in a series of intricate but precise enzymatic cleavage and membrane binding events. The resulting complement cascade leads to the production of products with opsonic, immunoregulatory, and lytic functions. A concise summary of the biologic activities associated with complement activation is provided, for example, in The Merck Manual, 16th Edition.

The complement cascade can progress via the classical pathway (CP), the lectin pathway, or the alternative pathway (AP). The lectin pathway is typically initiated with binding of mannose-binding lectin (MBL) to high mannose substrates. The AP can be antibody independent, and can be initiated by certain molecules on pathogen surfaces. The CP is typically initiated by antibody recognition of, and binding to, an antigenic site on a target cell. These pathways converge at the C3 convertase - the point where complement component C3 is cleaved by an active protease to yield C3a and C3b.

The AP C3 convertase is initiated by the spontaneous hydrolysis of complement component C3, which is abundant in the plasma fraction of blood. This process, also known as "tickover," occurs through the spontaneous cleavage of a thioester bond in C3 to form C3i or $C3(H_2O)$. Tickover is facilitated by the presence of surfaces that support the binding of activated C3 and/or have neutral or positive charge characteristics (e.g., bacterial cell surfaces). This formation of $C3(H_2O)$ allows for the binding of plasma protein Factor B, which in turn allows Factor D to cleave Factor B into Ba and Bb. The Bb fragment remains bound to C3 to form a complex containing $C3(H_2O)Bb$ - the "fluid-phase" or "initiation" C3 convertase. Although only produced in small amounts, the fluid-phase C3 convertase can cleave multiple C3 proteins into C3a and C3b and results in the generation of C3b and its subsequent covalent binding to a surface (e.g., a bacterial surface). Factor B bound to the surface-bound C3b is cleaved by Factor D to thus form the surface-bound AP C3 convertase complex containing C3b,Bb. (See, e.g., Muller-Eberhard (1988) Ann Rev Biochem 57:321-347.)

The AP C5 convertase - $(C3b)_2$,Bb - is formed upon addition of a second C3b monomer to the AP C3 convertase. (See, e.g., Medicus et al. (1976) J Exp Med 144:1076-1093 and Fearon et al. (1975) J Exp Med 142:856-863.) The role of the second C3b molecule is to bind C5 and present it for cleavage by Bb. (See, e.g., Isenman et al. (1980) J Immunol 124:326-331.) The AP C3 and C5 convertases are stabilized by the addition of the trimeric protein properdin as described in, e.g., Medicus et al. (1976), *supra.* However, properdin binding is not required to form a functioning alternative pathway C3 or C5 convertase. (See, e.g., Schreiber et al. (1978) Proc Natl Acad Sci USA 75: 3948-3952 and Sissons et al. (1980) Proc Natl Acad Sci USA 77: 559-562).

The CP C3 convertase is formed upon interaction of complement component C1, which is a complex of C1q, C1r, and C1s, with an antibody that is bound to a target antigen (e.g., a microbial antigen). The binding of the C1q portion of C1 to the antibody-antigen complex causes a conformational change in C1 that activates C1r. Active C1r then cleaves the C1-associated C1s to thereby generate an active serine protease. Active C1s cleaves complement component C4 into C4b and C4a. Like C3b, the newly generated C4b fragment contains a highly reactive thiol that readily forms amide or ester bonds with suitable molecules on a target surface (e.g., a microbial cell surface). C1s also cleaves complement component C2 into C2b and C2a. The complex formed by C4b and C2a is the CP C3 convertase, which is capable of processing C3 into C3a and C3b. The CP C5 convertase - C4b,C2a,C3b - is formed upon addition of a C3b monomer to the CP C3 convertase. (See, e.g., Müller-Eberhard (1988), *supra* and Cooper et al. (1970) J Exp Med 132:775-793.)

In addition to its role in C3 and C5 convertases, C3b also functions as an opsonin through its interaction with complement receptors present on the surfaces of antigen-presenting cells such as macrophages and dendritic cells. The opsonic function of C3b is generally considered to be one of the most important anti-infective functions of the complement system. Patients with genetic lesions that block C3b function are prone to infection by a broad variety of pathogenic organisms, while patients with lesions later in the complement cascade sequence, i.e., patients with lesions that block C5 functions, are found to be more prone only to *Neisseria* infection, and then only somewhat more prone.

The AP and CP C5 convertases cleave C5 into C5a and C5b. Cleavage of C5 releases C5a, a potent anaphylatoxin and chemotactic factor, and C5b, which allows for the formation of the lytic terminal complement complex, C5b-9. C5b combines with C6, C7, and C8 to form the C5b-8 complex at the surface of the target cell. Upon binding of several C9 molecules, the membrane attack complex (MAC, C5b-9, terminal complement complex - TCC) is formed. When sufficient numbers of MACs insert into target cell membranes the openings they create (MAC pores) mediate rapid osmotic lysis of the target cells.

While a properly functioning complement system provides a robust defense against infecting microbes, inappropriate regulation or activation of the complement pathways has been implicated in the pathogenesis of a variety of disorders including, e.g., rheumatoid arthritis (RA); lupus nephritis; asthma; ischemia-reperfusion injury; atypical hemolytic uremic syndrome (aHUS); dense deposit disease (DDD); paroxysmal nocturnal hemoglobinuria (PNH); macular degeneration (e.g., age-related macular degeneration (AMD)); hemolysis, elevated liver enzymes, and low platelets (HELLP) syndrome; thrombotic thrombocytopenic purpura (TTP), spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass and hemodialysis. (See, e.g., Holers et al. (2008) Immunological Reviews 223:300-316.) The down-regulation of complement activation has been demonstrated be effective in treating several disease indications in a variety of animal models. See, e.g., Rother et al. (2007) Nature Biotechnology 25(11):1256-1264, Wang et al. (1996) Proc. Natl. Acad Sci. USA 93:8563-8568; Wang et al. (1995) Proc. Natl. Acad. Sci. USA 92:8955-8959; Rinder et al. (1995) J. Clin. Invest. 96:1564-1572; Kroshus et al. (1995) Transplantation 60:1194-1202; Homeister et al. (1993) J. Immunol. 150:1055-1064; Weisman et al. (1990) Science 249:146-151; Amsterdam et al. (1995) Am. J. Physiol. 268:H448-H457; and Rabinovici et al. (1992) J Immunol 149:1744 1750.

Hilimen et al. (N Engl J Med. 2006 Sep 21;355(12):1233-43) investigates the use of the complement inhibitor eculizumab in paroxysmal nocturnal hemoglobinuria.

Summary

According to the present invention there is provided a bispecific antibody comprising: (a) a first antigen combining site that binds to C5a and (b) a second antigen combining site that binds to C5b, wherein the bispecific antibody bound to C5a inhibits the interaction between C5a and the C5a receptor; and wherein the bispecific antibody bound to C5b inhibits the assembly or lytic activity of the TCC; and wherein the bispecific antibody does not bind to full-length or mature C5.

The present disclosure relates to bispecific antibodies that bind to human complement component proteins. A bispecific antibody described herein can bind to two or more (e.g., two, three, four, five, or six or more) different epitopes. For example, a bispecific antibody described herein can bind to two or more (e.g., two, three, four, five, or six or more) different proteins, wherein at least two of the proteins are selected from C5a, C5b, a cellular receptor for C5a (e.g., C5aR1 or C5L2), and a component or an intermediate of the terminal complement complex such as C5b-6, C5b-7, C5b-8, or C5b-9. A bispecific antibody may bind to two different proteins selected from the group consisting of C5a, C5aR, C5b, C5L2, C5b-6, C5b-7, C5b-8, and C5b-9. The bispecific antibodies are useful for inhibiting terminal complement (e.g., the assembly and/or activity of the C5b=9 TCC) and/or C5a anaphylatoxin-mediated inflammation. Accordingly, the bispecific antibodies can be used in methods for treating a variety of complement pathway-associated disorders such as, but not limited to, atypical hemolytic uremic syndrome (aHUS); thombotic thrombocytopenic purpura (TTP); dense deposit disease (DDD); rheumatoid arthritis (RA); hemolysis, elevated liver enzymes, and low platelets (HELLP); age-related macular degeneration (AMD); myasthenia gravis (MG), cold agglutinin-disease (CAD), dermatomyositis, Graves' disease, Hashimoto's thyroiditis, type I diabetes, psoriasis, pemphigus, autoimmune hemolytic anemia (AIHA), idiopathic thrombocytopenic purpura (ITP), or any other complement-associated disorder described herein and/or known in the art.

The bispecific antibodies described herein feature a number of advantages, e.g., advantages over antibodies that bind to, and inhibit cleavage of, full-length or mature C5. Like such anti-C5 antibodies, the bispecific antibodies described herein are capable of inhibiting the downstream effects of one or both arms of C5 activation. That is, in some embodiments, the bispecific antibodies described herein can inhibit the C5a-mediated inflammatory response and the C5b (MAC)-dependent cell lysis that results from cleavage of C5. However, as the concentration of C5 in human plasma is approximately 0.37 μM (Rawal and Pangburn (2001) J Immunol 166(4):2635-2642), the use of high concentrations and/or frequent administration of anti-C5 antibodies is often necessary to effectively inhibit C5 in a human. Unlike C5, fragments C5a and C5b are present in blood at much lower concentrations and are often restricted to specific areas of local complement activation such as, e.g., the lungs in asthma patients, the joints of RA patients, or the drusen in the eyes of patients with AMD.

In addition, the disclosure sets forth in the working examples experimental data evidencing that while anti-C5 antibodies are highly effective at inhibiting complement *in vitro* and *in vivo* (see, e.g., Hillmen et al. (2004) N Engl J Med 350(6):552), the antibodies are particularly susceptible to target-mediated clearance because of the high concentration of C5 in blood. This discovery indicates that bispecific antibodies (e.g., bispecific antibodies that bind to C5a and a component of the MAC such as C5b) are very likely to have a longer half-life, as compared to anti-C5 antibodies, in blood due to a reduced contribution of antigen-mediated antibody clearance. As described above, fragments C5a and C5b are each present at a much lower concentration than C5 and are often produced at specific areas of complement activation. Thus, in view of their longer half-life, the bispecific antibodies described herein can be administered to a human at a much lower dose and/or less frequently than an anti-C5 antibody and effectively provide the same or greater inhibition of C5 in a human. The ability to administer a lower dose of the bispecific antibody, as compared to dose of an anti-C5 antibody, also allows for additional delivery routes such as, e.g., subcutaneous administration, intramuscular administration, intrapulmonary delivery, and administration via the use of biologically degradable microspheres.

The bispecific antibodies described herein also provide a number of advantages over the use of a combination of two different antibodies. For example, a bispecific antibody that binds to C5a and C5b, being one molecule, requires only one process development as compared to two different processes required to produce a separate anti-C5a antibody and an anti-C5b antibody. Moreover, administration of a cocktail of two different antibodies (e.g., an anti-C5a antibody and an anti-C5b antibody) would also likely require a significantly more complex clinical evaluation. For example, safety studies and pharmacokinetic (PK) and pharmacodynamic (PD) parameters for each separate antibody would need to be evaluated to ensure not only equivalence between the two antibodies, but also that neutralization of both of the antibodies' intended targets (e.g., C5a and C5b) is maintained over time. In addition, targeting only the active C5 fragments (C5a and C5b) or their receptors means that the clearance of the bispecific antibody is unlikely to be significantly influenced by the normal clearance or turnover of a native, highly abundant plasma C5 protein.

The disclosure features a bispecific antibody that binds to at least two of: C5a, C5b, a cellular receptor for C5a (e.g., C5aR1 or C5L2), a component of or an intermediate of the TCC; or the TCC (C5b-9) itself. The component of the TCC can be, e.g., C5b, C6, C7, C8, C9, or a biologically-active fragment thereof. The intermediate of the TCC can be, e.g., C5b-6, C5b-7, or C5b-8. The antibody may bind to C5b-9.

The disclosure also features a bispecific antibody that binds to: (a) C5a and C5aR1; (b) C5a and C5b; (c) C5b and C5aR1; (d) C5a and C5L2; (e) C5b and C5L2; (f) C5aR1 and C5L2; (g) C5b-6 and C5a; (h) C5b-6 and C5b; (i) C5b-6 and C5aR1; or (j) C5b-6 and C5L2.

The disclosure also features a bispecific antibody containing at least two (or consisting of two) different antigen combining sites, wherein at least one antigen combining site binds to C5a and at least one antigen combining site binds to C5b or C5aR. The disclosure also features an antibody containing at least two (or consisting of two) different antigen combining sites, wherein at least one antigen combining site binds to C5b and at least one antigen combining site binds to C5aR. The disclosure further features an antibody containing at least two different antigen combining sites, wherein (i) at least one antigen combining site binds to C5a or C5b; and (ii) at least one antigen combining site binds to a cellular receptor for C5a such as C5aR1 or C5L2. The disclosure also features an antibody containing at least two (or consisting of two) different antigen combining sites, wherein (i) at least one antigen combining site binds to C5a, C5b, or a cellular receptor for C5a; and (ii) at least one antigen combining site binds to C5b-6. Any of these antibodies may contain two or more than two (e.g., three, four, five, six, seven, eight, nine, or 10 or more) antigen combining sites.

The disclosure also features a bispecific antibody having binding specificity for at least two of C5a, C5aR1, C5b, C5L2, C6, C7, C8, C9, C5b-6, C5b-7, C5b-8, and C5b-9. The antibody may have binding specificity for at least two of C5a, C5b, C5aR1, and C5b-6.

The antibodies described herein may not bind to full-length or mature C5. The antibody may not bind to uncomplexed C5b, C6, C7, C8, or C9.

The disclosure also features a bispecific antibody containing or consisting of: (i) a first antigen combining site that binds to C5a; and (ii) a second antigen combining site that binds to a cellular receptor for C5a. The first antigen combining site can bind to desarginated C5a. The first antigen combining site can bind to a mammalian C5a (e.g., human C5a). The first antigen combining site can bind to a human C5a protein containing or consisting of an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:1. The first antigen combining site can bind to a fragment of a human C5a protein containing or consisting of the amino acid sequence depicted in any one of SEQ ID NOs:2-14. The first antigen combining site can bind to an epitope comprising at least 4 (e.g., at least 5, 6, 7, 8, 9, 10, 11, 12, 13, 14, 15, 16, 17, 18, 19, 20, 21, 22, 23, 24, or 25 or more) consecutive amino acids depicted in any one of SEQ ID NOs:1-14. The cellular receptor for C5a may be C5aR1.

C5aR1 can be a mammalian (e.g., a human) form of C5aR1. The second antigen combining site can bind to a human C5aR1 protein comprising an amino acid sequence that is at least 70 (e.g., at least 71, 72, 73, 74, 75,76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:17. The second antigen combining site can bind to a fragment of a human C5aR1 protein comprising the amino acid sequence depicted in any one of SEQ ID NOs:18-22. The second antigen combining site can bind to an epitope comprising at least 4 (e.g., at least 5, 6, 7, 8, 9, 10, 11, 12, 13, 14, 15, 16, 17, 18, 19, 20, 21, 22, 23, 24, or 25 or more) consecutive amino acids depicted in any one of SEQ ID NOs:17-22. The cellular receptor for C5a may be C5L2. C5L2 can be a mammalian (e.g., a human) form of C5L2. The second antigen combining site can bind to a human C5L2 protein comprising an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:23. The second antigen combining site may bind to a fragment of a human C5L2 at an epitope containing at least 4 (e.g., at least 5, 6, 7, 8, 9, 10, 11, 12, 13, 14, 15, 16, 17, 18, 19, 20, 21, 22, 23, 24, or 25 or more) consecutive amino acids of SEQ ID NO:23. The second antigen combining site may bind to a fragment of a human C5L2 protein comprising the amino acid sequence depicted in any one of SEQ ID NOs:24-27. The second antigen combining site may bind to an epitope comprising at least 4 (e.g., at least 5, 6, 7, 8, 9, 10, 11, 12, 13, 14, 15, 16, 17, 18, 19, 20, 21, 22, 23, 24, or 25 or more) consecutive amino acids depicted in anyone of SEQ ID NOs:24-27. The antibody may not bind to full-length or mature C5.

In an aspect, the disclosure features a bispecific antibody containing or consisting of: (i) a first antigen combining site that binds to C5a; and (ii) a second antigen combining site that binds to C5b. The first antigen combining site can bind to desarginated C5a. The first antigen combining site can bind to a mammalian C5a (e.g., human C5a). The first antigen combining site can bind to a human C5a protein containing or consisting of an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:1. The first antigen combining site can bind to a fragment of a human C5a protein containing or consisting of the amino acid sequence depicted in any one of SEQ ID NOs:2-14. The first antigen combining site can bind to an epitope comprising at least 4 (e.g., at least 5, 6, 7, 8, 9, 10, 11, 12, 13, 14, 15, 16, 17, 18, 19, 20, 21, 22, 23, 24, or 25 or more) consecutive amino acids depicted in any one of SEQ ID NOs:1-14. In some embodiments, the second antigen combining site binds to a mammalian (e.g., a human) form of C5b. The human C5b can contain or consist of an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:15 or 16. The second antigen combining site can bind to an epitope that contains, or consists of, at least 4 (e.g., at least 5, 6, 7, 8, 9, 10, 11, 12, 13,14, 15, 16, 17, 18, 19, 20, 21, 22, 23, 24, or 25 or more) consecutive amino acids depicted in SEQ ID NO:15 or 16. According to the present invention, the antibody does not bind to full-length or mature C5.

The disclosure also features a bispecific antibody that contains, or consists of: (i) a first antigen combining site that binds to C5b; and (ii) a second antigen combining site that binds to a cellular receptor for C5a.

The second antigen combining site may bind to a mammalian (e.g., a human) form of C5b. The human C5b can contain or consist of an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:15 or 16. The first antigen combining site can bind to an epitope that contains, or consists of, at least 4 (e.g., at least 5, 6, 7, 8, 9, 10, 11, 12, 13, 14, 15, 16, 17, 18, 19, 20, 21, 22, 23, 24, or 25 or more) consecutive amino acids depicted in SEQ ID NO:15 or 16.

The antibody may not bind to full-lengthor mature C5.

The cellular receptor for C5a may be C5aR1. C5aR1 can be a mammalian (e.g., a human) form of C5aR1. The second antigen combining site can bind to a human C5aR1 protein comprising an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:17. The second antigen combining site can bind to a fragment of a human C5aR1 protein comprising the amino acid sequence depicted in any one of SEQ ID NOs:18-22. The second antigen combining site can bind to an epitope comprising at least 4 (e.g., at least 5, 6, 7, 8, 9, 10, 11, 12, 13, 14, 15, 16, 17, 18, 19,20, 21, 22, 23,24, or 25 or more) consecutive amino acids depicted in any one of SEQ ID NOs:17-22. The cellular receptor for C5a may be C5L2. C5L2 can be a mammalian (e.g., a human) form of C5L2. The second antigen combining site can bind to a human C5L2 protein comprising an amino acid sequence that is at least 70 (e.g., at least 71, 72, 73, 74, 75, 76, 77, 78, 79, 80, 81, 82, 83, 84, 85, 86, 87, 88, 89, 90, 91, 92, 93, 94, 95, 96, 97, 98, 99, or even 100) % identical to the amino acid sequence depicted in SEQ ID NO:23. The second antigen combining site may bind to a fragment of a human C5L2 at an epitope containing at least 4 (e.g., at least 5, 6, 7, 8, 9, 10,11, 12, 13, 14, 15, 16, 17, 18, 19, 20, 21, 22, 23, 24, or 25 or more) consecutive amino acids of SEQ ID NO:23. The second antigen combining site can bind to a fragment of a human C5L2 protein comprising the amino acid sequence depicted in any one of SEQ ID NOs:24-27. The second antigen combining site can bind to an epitope comprising at least 4 (e.g., at least 5, 6, 7, 8, 9, 10, 11, 12, 13, 14, 15, 16, 17, 18, 19, 20, 21, 22, 23, 24, or 25 or more) consecutive amino acids depicted in any one of SEQ ID NOs:24-27.

The disclosure also provides a bispecific antibody that contains, or consists of: (i) a first antigen combining site that blinds to C5a, C5b, or a cellular receptor for C5a; and (ii) a second antigen combining site that binds to a component of the TCC, an intermediate of the TCC, or to C5b-9. TCC components, TCC intermediates, and suitable epitopes to which the first or second antigen combining site can bind are described herein. Where the first antigen combining site binds to C5b, the component of the TCC to which the second antigen combining sites binds may not be C5b. The first antigen combining site may bind to a first

epitope of C5b and the second antigen combining site may bind to a second epitope of C5b, wherein the first and second epitopes are not identical. For example, the first and second epitopes of C5b can be non-overlapping or can be composed of two different amino acid sequences.

A bispecific antibody described herein may contain a first and second antigen combining site that each bind to the same target (e.g., C5a, C5b, C5aR1, C5L2, C5b-6, C5b-7, C5b-8, C5b-9, C6, C7, C8, or C9), wherein each antigen combining site binds to a different epitope of the target. For example,

a bispecific antibody described herein can contain a first antigen combining site that binds to C5a and a second antigen combining site that binds to C5a at an epitope that is not identical to, or overlapping with, the epitope to which the first antigen combining site binds.

In some embodiments, a bispecific antibody described herein binds to the extracellular portion of the cellular receptor for C5a. For example, a bispecific antibody described herein can bind to the extracellular region of C5aR1 and/or C5L2.

A bispecific antibody described herein can inhibit the interaction between C5a and a cellular receptor for C5a (e.g., C5aR1 or C5L2).

A bispecific antibody described herein may inhibit the assembly or activity of the TCC. A bispecific antibody described herein may inhibit the C5a-dependent chemotaxis of a cell expressing a receptor for C5a.

A bispecific antibody described herein may inhibit the interaction between C5b and C6; C5b-6 and C7; C5b-7 and C8; or C5b-8 and C9.

A bispecific antibody described herein may inhibit complement-dependent lysis of a cell *in vitro*.

In some embodiments, a bispecific antibody described herein can bind to a cognate antigen with a $K_a$ of at least $10^8$ (e.g., at least $10^9$, $10^{10}$, or $10^{11}$) M$^{-1}$. For example, a bispecific antibody described herein can bind to C5a with a $K_a$ of at least $10^8$ M$^{-1}$. In another example, a bispecific antibody can bind to C5aR1 or C5L2 with a $K_a$ of at least $10^8$ M$^{-1}$. In yet another example, a bispecific antibody described herein can bind to C5b with a $K_a$ of at least $10^8$ M$^{-1}$. In yet another example, a bispecific antibody described herein can bind to C5b-6 with a $K_a$ of at least $10^8$ M$^{-1}$.

In some embodiments, a bispecific antibody described herein can further contain a third antigen combining site that binds to an antigen present in a terminal complement protein selected from the group consisting of C6, C7, C8, C9. In some embodiments, a bispecific antibody can contain an antigen combining site that binds to full-length or mature C5.

In some embodiments, a bispecific antibody described herein can be monoclonal, single-chain, humanized, recombinant, chimeric, chimerized, deimmunized, fully human, a diabody, an intrabody, or an F(ab')$_2$ fragment. The bispecific antibody can be a single chain diabody, a tandem single chain Fv fragment, a tandem single chain diabody, or a fusion protein comprising a single chain diabody and at least a portion of an immunoglobulin heavy chain constant region. The bispecific antibody can be a dual variable domain immunoglobulin (DVD-Ig) molecule. The bispecific antibody can contain, or be, two different monospecific antibodies that are associated with one another (e.g., covalently or non-covalently linked together).

In some embodiments, any bispecific antibody described herein can contain a heterologous moiety such as a sugar (e.g., an antibody can be glycosylated) or a detectable label. Detectable labels include, e.g., fluorescent labels, luminescent labels, heavy metal labels, radioactive labels, and enzymatic labels. A fluorescent label can be, e.g., selected from the group consisting of fluorescein, fluorescein isothiocyanate (FITC), green fluorescent protein (GFP), DyLight 488, phycoerythrin (PE), propidium iodide (PI), PerCP, PE-Alexa Fluor® 700, Cy5, allophycocyanin, and Cy7. An enzymatic label can be, e.g., horseradish peroxidase, alkaline phosphatase, or luciferase. A radioactive label can be, e.g., one selected from the group consisting of $^{32}$P, $^{33}$P, $^{14}$C, $^{125}$I, $^{131}$I, $^{35}$S, and $^3$H.

In another aspect, the disclosure features a pharmaceutical composition comprising a bispecific antibody of the invention and a pharmaceutically-acceptable carrier, excipient, or diluent.

The disclosure also features a method for inhibiting or preventing terminal complement in a subject. The method includes the step of administering to a subject in need thereof an antibody in an amount effective to inhibit terminal complement in the subject, wherein the antibody is any bispecific antibody described herein capable of inhibiting terminal complement. For example, the bispecific antibody can be one that binds to a component of the TCC (e.g., C5b, C6, C7, C8, or C9), an intermediate of the TCC (e.g., C5b-6, C5b-7, or C5b-8), or to C5b-9.

The disclosure also features a method for inhibiting or preventing C5a-dependent chemotaxis in a subject. The method includes the step of administering to a subject in need thereof an antibody in an amount effective to inhibit C5a-dependent chemotaxis in a subject. The antibody can be any bispecific antibody described herein capable of inhibiting C5a-dependent chemotaxis. For example, the bispecific antibody can be one that binds to C5a or C5aR1. The antibody can be administered as a pharmaceutical composition.

The disclosure also provides a method for treating or preventing a complement-associated disorder in a subject, which method include administering to a subject in need thereof an antibody in an amount effective to treat a complement-associated disorder in the subject. The antibody can be, e.g., a bispecific antibody described herein. For example, the antibody can be one that binds to: (i) C5a and C5b; (ii) C5b and a receptor for C5a; (iii) C5a and a component or intermediate of the TCC (or C5b-9); (iv) a receptor for C5a and a component or intermediate of the TCC (or C5b-9); or (v) C5b and a component or intermediate of the TCC (or C5b-9). The antibody can be administered as a pharmaceutical composition.

The subject may be one having, suspected of having, or at risk for developing a complement-associated disorder. The complement-associated disorder can be, e.g., an alternative complement pathway-associated disorder such as, e.g., rheumatoid arthritis, asthma, ischemia-reperfusion injury, atypical hemolytic uremic syndrome, thrombotic thrombocytopenic purpura, paroxysmal nocturnal hemoglobinuria, dense deposit disease, age-related macular degeneration, spontaneous fetal loss, Pauci-immune vasculitis, epidermolysis bullosa, recurrent fetal loss, multiple sclerosis, traumatic brain injury, or any other AP-associated disorder described herein or known in the art.

The subject may have, or is suspected of having, or at risk for developing a classical complement pathway-associated disorder such as, e.g., myasthenia gravis, cold agglutinin disease, dermatomyositis, Graves' disease, Hashimoto's thyroiditis, type I diabetes, psoriasis, pemphigus, autoimmune hemolytic anemia, idiopathic thrombocytopenic purpura, Goodpasture syndrome, antiphospholipid syndrome, catastrophic antiphospholipid syndrome, or any other CP-associated disorder described herein or known in the art of medicine.

Any of the methods described herein can further include the step of identifying the subject as having, suspected of having, or at risk for developing a complement-associated disorder. Any of the methods can include, after administering the antibody, monitoring the subject for an improvement in one or more symptoms of the complement-associated disorder.

The antibody may be intravenously administered to the subject. In some disclosures (e.g., where a respiratory condition is to be treated), the antibody can be administered to the subject by way of the lungs. The antibody may be administered subcutaneously or intramuscularly.

The subject may be a mammal. In some embodiments, the subject is a human.

The disclosure also features a method for producing any of the bispecific antibodies described herein. The method can include, e.g., culturing a cell that expresses a bispecific antibody under conditions that allow for the antibody to be expressed by the cell. The method can also include isolating the bispecific antibody from the cell or from the media in which the cell is cultured. The cultured cell can contain a nucleic acid expression vector containing a nucleotide sequence encoding the bispecific antibody. The vector can be integrated into the cell genome or can be episomal. The cell can be, e.g., a bacterial cell, a fungal cell (e.g., a yeast cell), an insect cell, or a mammalian cell. The cell can be a human cell.

Methods for producing a bispecific antibody described herein may involve chemically synthesizing the antibody.

The disclosure also features a population of cultured cells, a plurality of which express a bispecific antibody described herein. The population can include two or more pluralities of cells, each plurality including cells that express a different bispecific antibody.

As used herein, "associated with" in the context of an interaction between two or more atoms or molecular units (e.g., between two different monospecific antibodies or antigen-binding fragments of the monospecific antibodies), includes any covalent or non-covalent bonding, or physical admixture, of two or more atoms or molecular units. The chemical nature of covalent bonds (two atoms sharing one or more pairs of valence electrons) are known in the art and include, e.g., disulfide bonds or peptide bonds. A non-covalent bond is a chemical bond between atoms or molecules that does not involve the sharing of pairs of valence electrons. For example, non-covalent interactions include, e.g., hydrophobic interactions, hydrogen-bonding interactions, ionic bonding, Van der Waals bonding, or dipole-dipole interactions. Examples of such non-covalent interactions include, e.g., binding pair interactions (interactions of a first and second member of a binding pair such as the interaction between streptavidin and biotin). For example, a bispecific antibody can contain, or be, a first monospecific antibody and a second monospecific antibody non-covalently linked together by way of an avidin/streptavidin binding pair.

Unless otherwise defined, all technical and scientific terms used herein have the same meaning as commonly understood by one of ordinary skill in the art to which this disclosure pertains. In case of conflict, the present document, including definitions, will control. Preferred methods and materials are described below, although methods and materials similar or equivalent to those described herein can also be used in the practice or testing of the presently disclosed methods and compositions.

Other features and advantages of the present disclosure, e.g., methods for treating complement-associated disorders in a subject, will be apparent from the following description, the examples, and from the claims.

Brief Description of the Drawings

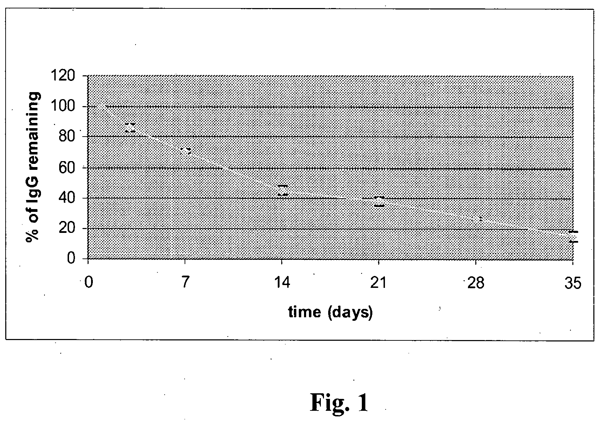

**Fig. 1** is a line graph depicting the time-dependent beta-phase clearance of a humanized anti-human C5 antibody in a human Fc receptor of neonate (FcRn) mouse model. The Y-axis represents the percentage of the initially administered amount of the humanized antibody that remains in the serum of the mice. The X-axis represents the time in days.

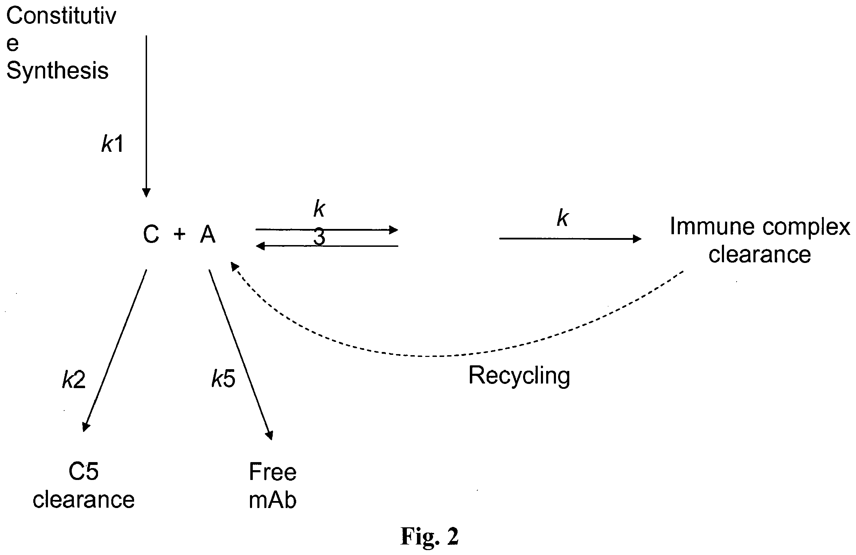

**Fig. 2** is a schematic diagram depicting the basic pathways for clearance of a humanized anti-human C5 antibody and its target antigen C5 in patients. Free antibody "A" refers to free (uncomplexed) antibody and "C" refers to uncomplexed or free C5. The antibody:C5 complex is represented by "CA." The rate constant for the association of the antibody and C5 is represented by "$k3$" and the rate constant for the dissociation of the complex is represented by "$k4$." The antibody:C5 complex can be eliminated by immune complex clearance having a rate constant represented by "$k6$." Free antibody is also eliminated as represented by a different rate constant "$k5$" C5 is constitutively expressed with a rate constant $k1$ and it is eliminated with a rate constant of "$k2$."

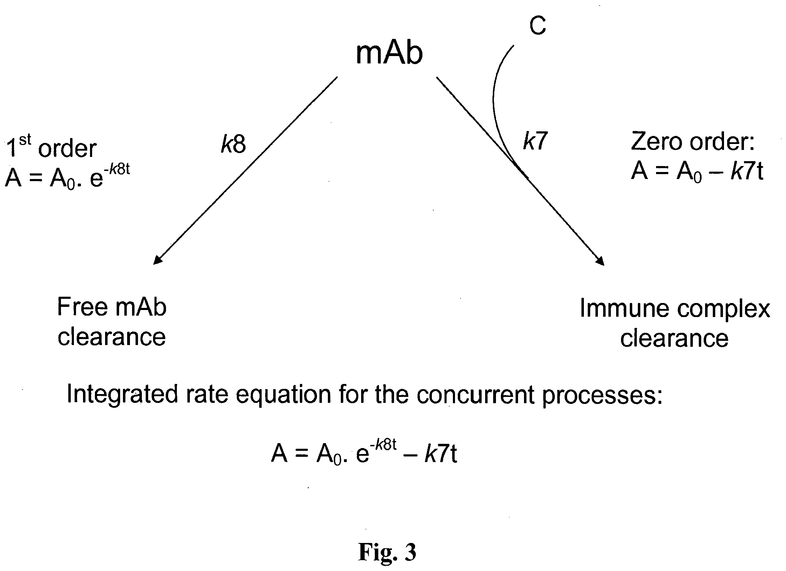

**Fig. 3** is a schematic diagram depicting a simplified series of pathways for clearance of a humanized anti-human C5 antibody in patients. "mAb" refers to the humanized antibody. "C" refers to free C5. In this simplified pathway, the immune complex clearance has a rate constant "$k7$." Free antibody clearance is governed by the first order equation $A = A_0 \times e^{(-k8t)}$ (Equation 2; see below), where "t" represents time and "$k8$" is the rate constant. Immune complex clearance is governed by the zero order equation $A = A_0 - k7t$ (Equation 3; see below), where $A_0$ is the concentration of the free antibody at time 0. The integrated rate equation for the concurrent processes is set forth as $A = A_0 \times e^{(-k8t)} - k7t$ (Equation 4; see below).

Detailed Description

The disclosure provides bispecific antibodies that are useful for inhibiting one or both of terminal complement (e.g., the assembly and/or activity of the C5b-9 TCC) and C5a anaphylatoxin-mediated inflammation. While in no way intended to be limiting, exemplary bispecific antibodies, conjugates, pharmaceutical compositions and formulations, and methods for using any of the foregoing are elaborated on below and are exemplified in the working Examples.

Antibodies

A bispecific antibody molecule described herein is one that binds to two or more (e.g., two, three, four, five, or six or more) different epitopes. For example, a bispecific antibody can bind to two or more (e.g., two, three, four, five, or six or more) different proteins, wherein at least two of the proteins are selected from C5a, C5b, a receptor for C5a (e.g., C5aR1 or C5L2), and a component or intermediate of the TCC such as C5b-6, C5b-7, C5b-8, or C5b-9. For example, the bispecific antibody can be one that binds to C5a and C5b. The bispecific antibody can be one that binds to C5aR and C5a. In another example, the bispecific antibody can be one that binds to C5aR1 and C5b. A bispecific antibody described herein may bind to two different proteins selected from the group consisting of C5a, C5aR1, C5L2, C5b, C5b-6, C5b-7, C5b-8, and C5b-9. The bispecific antibody may not bind to full-length, or mature C5.

The antibodies described herein can be used in a number of diagnostic and/or therapeutic applications. For example, a bispecific antibody described herein that binds to C5b, C5b-6, C5b-7, C5b-8, and/or C5b-9 is useful for inhibiting terminal complement *in vitro* or *in vivo.* An antibody that binds to one or both of C5a and C5aR1, may be useful for inhibiting a C5a anaphylatoxin-associated inflammatory response. Accordingly, the antibodies described herein are useful for preventing, treating, or ameliorating one or more symptoms of a variety of complement-associated conditions in a subject such as, but in no way limited to: PNH, hemolytic uremia syndrome (HUS), AMD, asthma, and sepsis.

At least one antigen combining site of a bispecific antibody described herein may bind to C5a, but not to full-length, native C5. Full-length C5 (e.g., the amino acid sequence of C5) is described in, e.g., Haviland et al. (1991) J Immunol 146(1):362-8. (See also Genbank Accession No. AAA51925.) Pro-C5 is a 1676 amino acid residue precursor protein. The first 18 peptides (numbered -18 to -1) constitute a signal peptide that is cleaved from the precursor protein. The remaining 1658 amino acid protein is cleaved in two places to form the alpha and beta chains. The first cleavage event occurs between amino acid residues 655 and 656. The second cleavage occurs between amino acid residues 659 to 660. The two cleavage events result in the formation of three distinct polypeptide fragments: (i) a fragment comprising amino acids 1 to 655, which is referred to as the beta chain; (ii) a fragment comprising amino acids 660 to 1658, which is referred to as the alpha chain; and (iii) a tetrapeptide fragment consisting of amino acids 656 to 659. The alpha chain and the beta chain polypeptide fragments are connected to each other via disulfide bond and constitute the mature C5 protein. The CP or AP C5 convertase activates mature C5 by cleaving the alpha chain between residues 733 and 734, which results in the liberation of C5a fragment (amino acids 660 to 733). The remaining portion of mature C5 is fragment C5b, which contains the residues 734 to 1658 of the alpha chain disulfide bonded to the beta chain.

*In vivo*, C5a is rapidly metabolized by a serum enzyme, carboxypeptidase B, to a 73 amino acid form termed "C5a des-Arg," which has lost the carboxyterminal arginine residue. Accordingly, in some embodiments, the antigen combining site that binds to C5a also binds to desarginated C5a. In some embodiments, the antigen combining site that binds to C5a does not bind to desarginated C5a.

In some embodiments, at least one antigen combining site of a bispecific antibody described herein can bind to a neoepitope present in C5a, i.e., an epitope that becomes exposed upon the liberation of C5a from the alpha chain fragment of mature C5. Antibodies that bind to C5a (e.g., a neo-epitope present in C5a) are known in the art as are methods for producing such antibodies. For example, an antibody that binds to C5a can have the binding specificity of a C5a neoepitope specific antibody described in any one of, e.g., PCT Publication No. WO 01/15731; Ames et al. (1994) J Immunol 152(9):4572-4581; Inoue (1989) Complement Inflamm 6(3):219-222; and U.S. Patent No. 6,866,845. In another example, a bispecific antibody that binds to C5a can have the binding specificity of a commercial C5a neoepitope-specific antibody such as, but not limited to, sc-52633 (Santa Cruz Biotechnology, Inc., Santa Cruz, California), I52-1486 (BD Pharmingen/BD Biosciences), ab11877 (Abcam, Cambridge, Massachusetts), and HM2079 (clone 2952; HyCult Biotechnology, the Netherlands). In some embodiments, the bispecific antibody that binds to C5a can crossblock the binding of any of the aforementioned C5a neoepitope-specific antibodies.

As used herein, the term "crossblocking antibody" refers to a subject bispecific antibody that lowers the amount of binding of a reference antibody to an epitope relative to the amount of binding of the reference antibody to the epitope in the absence of the subject antibody. Suitable methods for determining whether a subject bispecific antibody crossblocks the binding of a reference antibody to an epitope are known in the art.

In some embodiments, at least one antigen combining site of a bispecific antibody described herein can bind to a mammalian (e.g., human) form of C5a. For example, the antigen combining site can bind to a human C5a protein having the following amino acid sequence:

TLQKKIEEIAAKYKHSVVKKCCYDGACVNNDETCEQRAARISLGPRCIKAFTE    CCVVASQLRANISHKDMQLGR (SEQ ID NO:1). The antigen combining site can bind to human C5a at an epitope within or overlapping with the amino acid sequence: CCYDGACVNNDETCEQRAAR (SEQ ID NO:2); KCCYDGACVNNDETCEQR (SEQ ID NO:3); VNNDETCEQR (SEQ ID NO:4); VNNDET (SEQ ID NO:5); AARISLGPR (SEQ ID NO:6); CCYDGACVNNDETCEQRAA (SEQ ID NO:7); CCYDGACVNNDETCEQRA (SEQ ID NO:8); CCYDGACVNNDETCEQR (SEQ ID NO:9); CCYDGACVNNDETCEQ (SEQ ID NO:10); CCYDGACVNNDETCE (SEQ ID NO:11); CYDGACVNNDETCEQRAAR (SEQ ID NO:12); YDGACVNNDETCEQRAAR (SEQ ID NO:13); or CYDGACVNNDETCEQRAAR (SEQ ID NO:14). In some embodiments, the antigen combining site can bind to human C5a protein or fragment thereof containing an amino acid sequence that contains, or consists of, at least four (e.g., at least four, five, six, seven, eight, nine, 10, 11, 12, 13, 14, 15, 16, or 17 or more) consecutive amino acids depicted in any one of SEQ ID NOs:1-14. Additional C5a protein fragments to which an antibody described herein can bind and methods for generating suitable C5a-specific antigen combining sites are set forth in, e.g., U.S. Patent No. 4,686,100, the disclosure of which is incorporated herein by reference in its entirety.

The "antigen combining site" of an antibody, as used herein, refers to a surface of an antibody molecule that makes specific, physical contact with an antigen. The antigen combining site is typically composed of six hypervariable loops or complementarity determining regions (CDRs) within the hypervariable region of an immunoglobulin, three CDRs being from the light chain and three CDRs from the heavy chain of the immunoglobulin molecule. The exact boundaries of CDRs within the hypervariable regions of light and heavy chain polypeptides have been defined differently according to different methods. In some embodiments, the positions of the CDRs within a light or heavy chain variable domain can be as defined by Kabat et al. (1991) "Sequences of Proteins of Immunological Interest." NIH Publication No. 91-3242, U.S. Department of Health and Human Services, Bethesda, MD. In such cases, the CDRs can be referred to as "Kabat CDRs (e.g., "Kabat LCDR2" or "Kabat HCDR1"). In some embodiments, the positions of the CDRs of a light or heavy chain variable region can be as defined by Chothia et al. (1989) Nature 342:877-883. Accordingly, these regions can be referred to as "Chothia CDRs" (e.g., "Chothia LCDR2" or "Chothia HCDR3"), respectively.

In some embodiments, the binding of a bispecific antibody to C5a can inhibit the biological activity of C5a. Methods for measuring C5a activity include, e.g., chemotaxis assays, RIAs, or ELISAs (see, e.g., Ward and Zvaifler (1971) J Clin Invest. 50(3):606-16 and Wurzner et al. (1991) Compliment Inflamm. 8:328-340). In some embodiments, the binding of an antibody to C5a can inhibit the interaction between C5a and C5aR1. Suitable methods for detecting and/or measuring the interaction between C5a and C5aR1 (in the presence and absence of an antibody) are known in the art and described in, e.g., Mary and Boulay (1993) Eur J Haematol 51(5):282-287; Kaneko et al. (1995) Immunology 86(1):149-154: Giannini et al. (1995) J Biol Chem 270(32):19166-19172; and U.S. Patent No. 20060160726. For example, the binding of detectably labeled (e.g., radioactively labeled) C5a to C5aR1-expressing peripheral blood mononuclear cells can be evaluated in the presence and absence of an antibody. A decrease in the amount of detectably-labeled C5a that binds to C5aR1 in the presence of the antibody, as compared to the amount of binding in the absence of the antibody, is an indication that the antibody inhibits the interaction between C5a and C5aR1. In some embodiments, the binding of an antibody to C5a can inhibit the interaction between C5a and C5L2 (see below). Methods for detecting and/or measuring the interaction between C5a and C5L2 are known in the art and described in, e.g., Ward (2009) J Mol Med 87(4):375-378 and Chen et al. (2007) Nature 446(7132):203-207 (see below).

In some embodiments, at least one antigen combining site of a bispecific antibody described herein binds to C5b, but does not bind to full-length, native C5. The structure of C5b is described above and also detailed in, e.g., Müller-Eberhard (1985) Biochem Soc Symp 50:235-246; Yamamoto and Gewurz (1978) J Immunol 120(6):2008-2015; and Haviland et al. (1991), *supra.* C5b combines with C6, C7, and C8 to form the C5b-8 complex at the surface of the target cell. Protein complex intermediates formed during the series of combinations includes C5b-6 (including C5b and C6), C5b-7 (including C5b, C6, and C7), and C5b-8 (including C5b, C6, C7, and C8). Upon binding of several C9 molecules, the membrane attack complex (MAC, C5b-9 terminal complement complex (TCC)) is formed. When sufficient numbers of MACs insert into target cell membranes the openings they create (MAC pores) that mediate rapid osmotic lysis of the target cells.

In some embodiments, the binding of a bispecific antibody described herein to C5b can inhibit the interaction between C5b and C6. In some embodiments, the binding of the antibody to C5b can inhibit the assembly or activity of the C5b-9 MAC-TCC. In some embodiments, the binding of the bispecific antibody to C5b can inhibit complement-dependent cell lysis (e.g., *in vitro* and/or *in vivo*). Suitable methods for evaluating whether an antibody inhibits complement-dependent lysis include, e.g., hemolytic assays or other functional assays for detecting the activity of soluble C5b-9. For example, a reduction in the cell-lysing ability of complement in the presence of an antibody can be measured by a hemolysis assay described by Kabat and Mayer (eds.), "Experimental Immunochemistry, 2nd Edition," 135-240, Springfield, IL, CC Thomas (1961), pages 135-139, or a conventional variation of that assay such as the chicken erythrocyte hemolysis method as described in, e.g., Hillmen et al. (2004) N Engl J Med 350(6):552.

Antibodies that bind to C5b as well as methods for making such antibodies are known in the art. See, e.g., U.S. Patent No. 6,355,245. Commercially available anti-C5b antibodies are available from a number of vendors including, e.g., Hycult Biotechnology (catalogue number: HM2080; clone 568) and Abcam™ (ab46151 or ab46168).

In some embodiments, at least one antigen combining site of a bispecific antibody described herein can bind to a mammalian (e.g., human) form of C5b. For example, the antigen combining site can bind to a portion of a human C5b protein having the following amino acid sequence:

QEQTYVISAPKIFRVGASENIVIQVYGYTEAFDATISIKSYPDKKFSYSSGHVHL
SSENKFQNSAILTIQPKQLPGGQNPVSYVYLEVVSKHFSKSKRMPITYDNGFLF
IHTDKPVYTPDQSVKVRVYSLNDDLKPAKRETVLTFIDPEGSEVDMVEEIDHI
GIISFPDFKIPSNPRYGMWTIKAKYKEDFSTTGTAYFEVKEYVLPHFSVSIEPEY
NFIGYKNFKNFEITIKARYFYNKVVTEADVYITFGIREDLKDDQKEMMQTAM      QNTMLINGIAQVTFDSETAVKEL-
SYYSLEDLNNKYLYIAVTVIESTGGFSEEAE            IPGIKYVLSPYKLNLVATPLFLKPGIPYPIKVQVKDSLDQLVG-
GVPVILNAQTID                VNQETSDLDPSKSVTRVDDGVASFVLNLPSGVTVLEFNVKTDAPDLPEENQA
REGYRAIAYSSLSQSYLYIDWTDNHKALLVGEHLNIIVTPKSPYIDKITHYNYL
ILSKGKIIHFGTREKFSDASYQSINIPVTQNMVPSSRLLVYYIVTGEQTAELVSD SVWLNIEEKCGNQLQVHLSPDA-
DAYSPGQTVSLNMATGMDSWVALAAVDS         AVYGVQRGAKKPLERVFQFLEKSDLGCGAGGGLNNANVFH-
LAGLTFLTNAN ADDSQENDEPCKEIL (SEQ ID NO:15). The antigen combining site can bind to a portion of a human C5b protein having the following amino acid sequence:

LHMKTLLPVSKPEIRSYFPESWLWEVHLVPRRKQLQFALPDSLTTWEIQGIGIS
NTGICVADTVKAKVFKDVFLEMNIPYSVVRGEQIQLKGTVYNYRTSGMQFCV
KMSAVEGICTSESPVIDHQGTKSSKCVRQKVEGSSSHLVTFTVLPLEIGLHNIN
FSLETWFGKEILVKTLRVVPEGVKRESYSGVTLDPRGIYGTISRRKEFPYRIPL
DLVPKTEIKRILSVKGLLVGEILSAVLSQEGINILTHLPKGSAEAELMSVVPVFY
VFHYLETGNHWNIFHSDPLIEKQKLKKKLKEGMLSIMSYRNADYSYSVWKG
GSASTWLTAFALRVLGQVNKYVEQNQNSICNSLLWLVENYQLDNGSFKENS
QYQPIKLQGTLPVEARENSLYLTAFTVIGIRKAFDICPLVKIDTALIKADNFLLE
NTLPAQSTFTLAISAYALSLGDKTHPQFRSIVSALKREALVKGNPPIYRFWKD
NLQHKDSSVPNTGTARMVETTAYALLTSLNLKDINYVNPVIKWLSEEQRYGG
GFYSTQDTINAIEGLTEYSLLVKQLRLSMDIDVSYKHKGALHNYKMTDKNFL
GRPVEVLLNDDLIVSTGFGSGLATVHVTTVVHKTSTSEEVCSFYLKIDTQDIEA
SHYRGYGNSDYKRIVACASYKPSREESSSGSSHAVMDISLPTGISANEEDLKA   LVEGVDQLFTDYQIKDGH-
VILQLNSIPSS                  DFLCVRFRIFELFEVGFLSPATFTVYEYHRPDKQCTMFYSTSNIKIQKVCEGAA

CKCVEADCGQMQEELDLTISAETRKQTACKPEIAYAYKVSITSITVENVFVKY KATLLDIYKTGEAVAEKDSEITFIKKVTCTNAELVKGRQYLIMGKEALQIKYN FSFRYIYPLDSLTWIEY-WPRDTTCSSCQAFLANLDEFAEDIFLNGC (SEQ ID NO:16). In some embodiments, the antigen combining site can bind to human C5b protein or fragment thereof containing an amino acid sequence that contains, or consists of, at least four (e.g., at least four, five, six, seven, eight, nine, 10, 11, 12, 13, 14, 15, 16, 17, 18, 19, or 20 or more) consecutive amino acids depicted in SEQ ID NO:15 or SEQ ID NO:16.

Additional exemplary sub-fragments of human C5b or C5a to which a bispecific antibody described herein can bind are disclosed in, e.g., U.S. Patent No. 6,355,245.

At least one antigen combining site of a bispecific antibody described herein may bind to a receptor for human C5a. For example, at least one antigen combining site of the antibody can bind to C5aR (C5aR1 or CD88) or to C5L2 (GPR77).

At least one antigen combining site of a bispecific antibody described herein may bind to C5aR1 (e.g., to the extracellular region of C5aR1). C5aR1 has been described in detail in, e.g., Gerard and Gerard (1991) Nature 349(6310):614-617; Bao et al. (1992) Genomics 13:437-440; and U.S. Patent Application Publication No. 20050244406. For example, C5aR1 belongs to the family of seven transmembrane G-protein-coupled receptors and binds with high affinity to C5a. The C5a/C5aR1 interaction has a $K_d$ of about 1 nM. C5aR1 contains an extended N-terminal extracellular domain; the C5aR1 structure conforms to the seven transmembrane receptor family, with the extracellular N-terminus being followed by seven transmembrane helices connected by interhelical domains alternating as intracellular and extracellular loops, and ending with an intracellular C-terminal domain. C5aR1 can be found on many cell types including, e.g., smooth muscle cells, endothelial cells, and a variety of lymphocytes including, without limitation, neutrophils and macrophages. (See, e.g., Soruri et al. (2003) Immunol Lett 88(1):47-52: Zwirner et al. (1999) Mol Immunol 36(13-14):877-884; and Kiafard et al. (2007) Immunobiology 212(2):129-139. The binding of C5a to C5aR (C5aR1 or CD88) triggers a number of pro-inflammatory effects including, e.g., chemotaxis of several myeloid lineage cells (e.g., neutrophils, eosinophils, basophils, macrophages, and monocytes) and increased vascular permeability. High level activation of C5aR1 results in lymphocyte degranulation and activation of NADPH oxidase. C5a/C5aR1 have been implicated in the pathogenesis of a number of disorders including, e.g., sepsis, septic shock, SIRS (systemic/severe inflammatory response syndrome), MOF (multi organ failure), ARDS (acute respiratory distress syndrome), rheumatoid arthritis, and recurrent pregnancy loss. (See, e.g., Rittirsch et al. (2008) Nature Med 14:551-557; Girardi et al. (2003) J Clin Invest 112:1644-1654; Atkinson (2003) J Clin Invest 112:1639-1641; U.S. Patent No. 7,455,837; U.S. Patent Application Publication Nos. 20070065433, 20070274989, and 20070123466).

An exemplary amino acid sequence for human C5aR1 is provided herein and set forth in, e.g., Genbank Accession No. NP_001727.

At least one antigen combining site of a bispecific antibody described herein may bind to a mammalian (e.g., human) C5aR1. The human C5aR1 can have, e.g., the following amino acid sequence:

MNSFNYTTPDYGHYDDKDTLDLNTPVDKTSNTLRVPDILALVIFAVVFLVGV
LGNALVVWVTAFEAKRTINAIWFLNLAVADFLSCLALPILFTSIVQHHHWPFG
GAACSILPSLILLNMYASILLLATISADRFLLVFKPIWCQNFRGAGLAWIACAV
AWGLALLLTIPSFLYRVVREEYFPPKVLCGVDYSHDKRRERAVAIVRLVLGFL
WPLLTLTICYTFILLRTWSRRATRSTKTLKVVVAVVASFFIFWLPYQVTGIMM
SFLEPSSPTFLLLNKLDSLCVSFAYINCCINPIIYVVAGQGFQGRLRKSLPSLLR
NVLTEESVVRESKSFTRSTVDTMAQKTQAV (SEQ ID NO:17).

The antigen combining site may bind to human C5aR1 at an epitope that is within or overlapping with the amino acid sequence: SIVQHHHWPFGGAACS (SEQ ID NO:18); RVVREEYFPPKVLCGVDYSHDKRRERAVAIVR (SEQ ID NO:19); or MSFLEPSSPTFLLLNKLDS (SEQ ID NO:20).
The antigen combining site may bind to human C5aR1 protein or fragment thereof containing, or consisting of, at least four (e.g., at least four, five, six, seven, eight, nine, 10, 11, 12, 13, 14, 15, 16, or 17 or more) consecutive amino acids depicted in any one of SEQ ID NOs:17-20.

At least one antigen combining site of a bispecific antibody described herein may bind to the amino terminus of human C5aR1. For example, the antigen combining site can bind to an epitope that is within or overlapping with the amino acid sequence:

MNSFNYTTPDYGHYDDKDTLDLNTPVDKT (SEQ ID NO:21) or DYGHYDDKDTLDLNTPVDKT (SEQ ID NO:22),
The antigen combining site may bind
to a human C5aR1 protein containing, or consisting of, at least four (e.g., at least four, five, six, seven, eight, nine, 10, 11, 12, 13, 14, 15, 16, or 17 or more) consecutive amino acids depicted in any one of SEQ ID NO: 21 or 22.

An antigen combining site may bind to a human C5aR1 protein at an epitope within or overlapping with an amino acid sequence containing amino acids 24-30 of SEQ ID NO:17.

The binding of an antibody to C5aR1 may antagonize the activity of C5aR1. The binding of the bispecific antibody to C5aR1 may inhibit the interaction between C5a and C5aR1. Methods for detecting and/or measuring the interaction between C5a and C5aR1 are described herein. Methods for measuring the activity of C5aR1 (or inhibition thereof) are known in the art and include, e.g., a C5a-directed *in vitro* neutrophil chemotaxis assay as described in, e.g., U.S. Patent Application Publication No. 20050244406.

Exemplary C5aR1 antibodies, as well as methods for making the antibodies, are described in, e.g., U.S. Patent Application Publication No. 20050244406 and U.S. Patent No. 7,455,83 7.

At least one antigen combining site of a bispecific antibody described herein may bind to the C5a receptor C5L2. C5L2 is a high affinity receptor for C5a and is expressed on, e.g., granulocytes and immature dendritic cells. Unlike C5aR, C5L2 is not coupled to G proteins. (See, e.g., Monk et al. (2007) Br J Pharm 152:429-448 and Huber-Lang et al. (2005) J Immunol 174:1104-1110.)

At least one antigen combining site of a bispecific antibody described herein may bind to a mammalian (e.g., human) C5L2. The human C5L2 can have, e.g., the following amino acid sequence:

MGNDSVSYEYGDYSDLSDRPVDCLDGACLAIDPLRVAPLPLYAAIFLVGVPG
NAMVAWVAGKVARRRVGATWLLHLAVADLLCCLSLPILAVPIARGGHWPY
GAVGCRALPSIILLTMYASVLLLAALSADLCFLALGPAWWSTVQRACGVQVA
CGAAWTLALLLTVPSAIYRRLHQEHFPARLQCVVDYGGSSSTENAVTAIRFLF
GFLGPLVAVASCHSALLCWAARRCRPLGTAIVVGFFVCWAPYHLLGLVLTV
AAPNSALLARALRAEPLIVGLALAHSCLNPMLFLYFGRAQLRRSLPAACHWA
LRESQGQDESVDSKKSTSHDLVSEMEV (SEQ ID NO:23).

The antigen combining site may bind to human C5L2 protein or fragment thereof containing, or consisting of, at least four (e.g., at least four, five, six, seven, eight, nine, 10, 11, 12, 13, 14, 15, 16, or 17 or more) consecutive amino acids depicted in SEQ ID NO:23. The antigen combining site may bind to human C5L2 at an epitope that is within or overlapping with the amino acid sequence: PIARGGHWPYGAVGCR (SEQ ID NO:24); RRLHQEHFPARLQCVVDYGGSSSTENAVTAIR (SEQ ID NO:25); LTVAAPNSALLARALRAE (SEQ ID NO:26) or MGNDSVSYEYGDYSDLSDRPVDCLDGACLAIDPLR. (SEQ ID NO:27).

The antigen combining site may bind to human C5L2 protein or fragment thereof containing, or consisting of, at least four (e.g., at least four, five, six, seven, eight, nine, 10, 11, 12, 13, 14, 15, 16, or 17 or more) consecutive amino acids depicted in any one of SEQ ID NOs:24-27.

The binding of the bispecific antibody to CSL2 may inhibit the interaction between C5a and C5L2. Methods for detecting and/or measuring the interaction between C5a and C5L2 are described herein and in, e.g., Scola et al. (2007) J Biol Chem 282(6):3664-3671 and Cain and Monk (2002) J Biol Chem 277(9):7165-7169.

At least one antigen combining site of a bispecific antibody described herein may bind to a component of the terminal C5b-9 complement complex (TCC), e.g., C5b, C6, C7, C8, or C9. At least one antigen combining site of a bispecific antibody described herein may bind to a neoepitope present in an intermediate of the TCC or of the TCC itself, i.e., a neoepitope that is presented to solvent upon formation of an intermediate of the TCC or the TCC itself. As described above, intermediates of the TCC include, e.g., C5b-6, C5b-7, C5b-8, and C5b-9. Thus, the antigen combining site may bind to the C5b-6 intermediate, but not to uncomplexed C5b or to uncomplexed C6.

The antigen combining site may bind to the C5b-6 intermediate, but not bind to C5b-7, C5b-8, C5b-9, or any combination of the foregoing. The binding of the antibody to C5b-6 may inhibit the interaction between C5b-6 and C7. The binding of the antibody to C5b-6 may inhibit the formation of the TCC. Suitable methods for detecting the interaction between various members of the TCC and/or its intermediates (e.g., C5b-6 and C7) are described in, e.g., DiScipio (1992) J Biol Chem 267(24):17087-17094; Podack et al. (1980) J Exp Med 151:301-303; Podack et al. (1978) J Immunol 120:1841-1848; DiScipio et al. (1988) J Biol Chem 263:549-560. Methods for detecting the formation or activity of the TCC are well known in the art and include, e.g., hemolytic assays and use of C5b-9 neoepitope-specific antibodies.

Antibodies that bind to neoepitopes present on the C5b-6 intermediate, as well as methods for generating and identifying such antibodies, have been described in, e.g., Podack et al. (1978), *supra* and Mollnes et al. (1989) Complement Inflamm 6(3):223-235.

An antigen combining site of the bispecific antibody may bind to a neoepitope present in C5b-7, a complex containing C5b, C6, and C7.

An antigen combining site of the bispecific antibody may bind to a neoepitope present in C5b-8, a complex containing C5b, C6, C7, and C8. The foregoing antibodies, as well as methods for producing the antibodies, are described in, e.g., Mollnes et al. (1989), *supra.* The antibodies may be useful to inhibit the assembly and/or activity of the TCC.

The bispecific antibodies described herein may contain a third antigen combining site that binds to a terminal complement protein selected from the group consisting of C6, C7, C8, C9, and full-length, native C5.

The bispecific antibodies described herein may contain at least one antigen combining site that binds to: (i) C5a and C5 or (ii) C5b and C5. In other words, an antigen combining site that binds to C5a can also bind to full length or mature C5 and an antigen combining site that binds to C5b can also bind to full length or mature C5.

A bispecifc antibody may specifically bind to C5a, C5b, and/or C5aR. The term "specific binding" or "specifically binds" refers to two molecules forming a complex (e.g., a complex between a bispecific antibody and C5a, C5b, or C5aR) that is relatively stable under physiologic conditions. Typically, binding is considered specific when the association constant ($K_a$) is higher than $10^6$ M$^{-1}$. Thus, a bispecific antibody can specifically bind to a protein (e.g., C5a, C5b, or C5aR) with a $K_a$ of at least (or greater than) $10^6$ (e.g., at least or greater than $10^7$, $10^8$, $10^9$, $10^{10}$, $10^{11}$, $10^{12}$, $10^{13}$, $10^{14}$, or $10^{15}$ or higher) M$^{-1}$.

Methods for determining whether an antibody (e.g., a bispecific antibody described herein) binds to a protein antigen and/or the affinity for an antibody to a protein antigen are known in the art. For example, the binding of an antibody to a protein antigen can be detected and/or quantified using a variety of techniques such as, but not limited to, Western blot, dot blot, surface plasmon resonance method (e.g., BIAcore system; Pharmacia Biosensor AB, Uppsala, Sweden and Piscataway, N.J., Octet), or enzyme-linked immunosorbent assay (ELISA) assays. See, e.g., Harlow and Lane (1988) "Antibodies: A Laboratory Manual" Cold Spring Harbor Laboratory Press, Cold Spring Harbor, N.Y.; Benny K. C. Lo (2004) "Antibody Engineering: Methods and Protocols," Humana Press (ISBN: 1588290921); Borrebaek (1992) "Antibody Engineering, A Practical Guide," W.H. Freeman and Co., NY; Borrebaek (1995) "Antibody Engineering," 2nd Edition, Oxford University Press, NY, Oxford; Johne et al. (1993) J. Immunol. Meth 160:191-198; Jonsson et al. (1993) Ann. Biol. Clin. 51:19-26; and Jonsson et al. (1991) Biotechniques 11:620-627. See also, U.S. Patent No. 6,355,245.

As used herein, the term "bispecific antibody" refers to a whole or intact antibody molecule (e.g., IgM, IgG (including IgG1, IgG2, IgG3, and IgG4), IgA, IgD, or IgE) and any fragment thereof, which binds to two or more different proteins, at least two of which being C5a, C5b, or C5aR (see above). The term bispecific antibody includes, e.g., a chimerized or chimeric antibody, a humanized antibody, a deimmunized human antibody, and a fully human antibody. Bispecific antibodies also include, e.g., F(ab')$_2$ fragments or conjugates of two or more monospecific antibody fragments (e.g., two or more scFv, a Fab, an Fab', or an Fd immunoglobulin fragment). In addition, bispecific intrabodies, minibodies, triabodies, and diabodies (see, e.g., Todorovska et al. (2001) J Immunol Methods 248(1):47-66; Hudson and Kortt (1999) J Immunol Methods 231(1):177-189; Poljak (1994) Structure 2(12):1121-1123; Rondon and Marasco (1997) Annual Review of Microbiology 51:257-28 3). are also included in the definition of bispecific antibody and are compatible for use in the methods described herein. Also embraced by the term bispecific antibody are tandem single chain antibodies, single chain diabodies, tandem single chain diabodies, and fusion proteins containing single chain diabodies and at least a portion of a heavy chain constant region (e.g., a CH1 or a CH3 region of a heavy chain polypeptide) as described in, e.g., Kontermann (2005) Acta Pharmacologica Sinica 26(1): 1-9; Kufer et al. (2004) Trends Biotechnol 22:238-244; and Kriangkum et al. (2001) Biomol Eng 18:31-40,

Also embraced by the term "bispecific antibody" are antibodies containing at least one antigen combining site comprised of fewer than six canonical CDRs, e.g., a bispecific antibody where the binding specificity of one antigen combining site is determined by three, four or five CDRs, rather than six. Examples of antibodies wherein binding affinity and specificity are contributed primarily by one or the other variable domain are known in the art. Jeffrey et al. [(1993) Proc Natl Acad Sci USA 90:10310-10314] discloses an anti-digoxin antibody that binds to dioxin primarily by the antibody heavy chain. Accordingly, a skilled artisan can identify an antibody containing a single variable domain and that binds to, e.g., C5a, C5b, or C5aR in accordance with the disclosure.

In some embodiments, a bispecific antibody described herein can contain at least one antigen combining site from a "camelid" antibody. Camelid antibodies comprise a heavy chain, but lack a light chain. See, e.g., Muyldermans (2001) J Biotechnol 74:277-302. As such, the variable heavy chain region from a camelid antibody contains the minimal structural elements required to specifically bind to an antigen of interest. Camelid variable heavy chain regions have been found to bind to cognate antigens with high affinity. See, e.g., Desmyter et al. (2001) J Biol Chem 276:26285-90;

Dumoulin et al. (2003) Nature 424:783-788; Chan et al. (2008) Biochemistry (2008) 47(42):11041-54; and U.S. Patent No. 7,371,849.

A wide variety of bispecific antibody formats are known in the art of antibody engineering and methods for making the bispecific antibodies are well within the purview of those skilled in the art. Traditionally, the recombinant production of bispecific antibodies is based on the co-expression of two immunoglobulin heavy-chain/light-chain pairs, where the two heavy chains have different specificities (Milstein and Cuello (1983) Nature 305:537-539). Antibody variable domains with the desired binding specificities (antibody-antigen combining sites) can be fused to immunoglobulin constant domain sequences. The fusion can include an immunoglobulin heavy-chain constant domain, e.g., at least part of the hinge, CH2, and CH3 regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, e.g., Suresh et al. (1986) Methods in Enzymology 121:210; PCT Publication No. WO 96/27011; Brennan et al. (1985) Science 229:81; Shalaby et al., J. Exp. ed. (1992) 175:217-225; Kostelny et al. (1992) J Immunol 148(5) :1547-1553; Hollinger et al. (1993) Proc Natl Acad Sci USA 90:6444-6448; Gruber et al. (1994) J Immunol 152:5368; and Tutt et al. (1991) J Immunol 147:60. Bispecific antibodies also include cross-linked or heteroconjugate antibodies. Heteroconjugate antibodies may be made using any convenient cross-linking methods. Suitable cross-linking agents are well known in the art, and are disclosed in U.S. Pat. No. 4,676,980, along with a number of cross-linking techniques.

U.S. Patent No. 5,534,254 describes several different types of bispecific antibodies including, e.g., single chain Fv fragments linked together by peptide couplers, chelating agents, or chemical or disulfide couplings. In another example, Segal and Bast [(1995) Curr Protocols Immunol Suppl. 14:2.13.1-2.13.16] describes methods for chemically cross-linking two monospecific antibodies to thus form a bispecific antibody. As described above, a bispecific antibody described herein can be formed, e.g., by conjugating two single chain antibodies which are selected from, e.g.: (a) an antibody specific for a C5a; (b) an antibody specific for C5b; (c) an antibody specific for C5aR1; (d) an antibody specific for C5L2; (e) an antibody specific for C5b-6; (f) an antibody specific for C5b-7; (g) an antibody specific for C5b-8; and (h) an antibody specific for C5b-9. A bispecific antibody, may be a fusion protein containing a monoclonal antibody to C5a or C5b (or an antigen-binding fragment thereof) and an antibody or antigen-binding fragment thereof specific to C5aR1. In some embodiments, the bispecific antibody is a fusion protein containing a monoclonal antibody or fragment thereof specific to C5a and a second monoclonal antibody or fragment thereof that is specific to C5b. In

The bispecific antibody may be a fusion protein containing a monoclonal antibody or fragment thereof specific for a component or intermediate of the TCC and a monoclonal antibody that is specific for C5a or a receptor for C5a (e.g., C5aR1 or C5L2).

Various techniques for making and isolating bispecific antibody fragments directly from recombinant cell culture have also been described. For example, bispecific antibodies have been produced using leucine zippers. (See, e.g., Kostelny et al. (1992) J Immunol 148(5):1547-1553 and de Kruif and Logtenberg (1996) J Biol Chem 271(13):7630-7634) The leucine zipper peptides from the Fos and Jun proteins may be linked to the Fab' portions of two different antibodies by gene fusion. The antibody homodimers may be reduced at the hinge region to form monomers and then re-oxidized to form the antibody heterodimers.

In some embodiments, the bispecific antibody can be a tandem single chain (sc) Fv fragment, which contain two different scFv fragments covalently tethered together by a linker (e.g., a polypeptide linker). See, e.g., Ren-Heidenreich et al. (2004) Cancer 100:1095-1103 and Korn et al. (2004) J Gene Med 6:642-651. In some embodiments, the linker can contain, or be, all or part of a heavy chain polypeptide constant region such as a CH1 domain as described in, e.g., Grosse-Hovest et al. (2004) Proc Natl Acad Sci USA 101:6858-6863. In some embodiments, the two antibody fragments can be covalently tethered together by way of a polyglycine-serine or polyserine-glycine linker as described in, e.g., U.S. patent nos. 7,112,324 and 5,525,491, respectively. See also U.S. patent no. 5,258,498.

Methods for generating bispecific tandem scFv antibodies are described in, e.g., Maletz et al. (2001) Int J Cancer 93:409-416; Hayden et al. (1994) Ther Immunol 1:3-15; and Honemann et al. (2004) Leukemia 18:636-644. Alternatively, the antibodies can be "linear antibodies" as described in, e.g., Zapata et al. (1995) Protein Eng. 8(10):1057-1062. Briefly, these antibodies comprise a pairof tandem Fd segments ($V_H$-$C_H$1-$V_H$-$C_H$1) that form a pair of antigen binding regions.

A bispecific antibody can also be a diabody. Diabody technology described by, e.g., Hollinger et al. (1993) Proc Natl Acad Sci USA 90:6444-6448 has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. (See also, e.g., Zhu et al. (1996) Biotechnology 14:192-196 and Helfrich et al. (1998) Int J Cancer 76:232-239.) Bispecific single chain diabodies (scDb) as well as methods for generating scDb are described in, e.g., Brüsselbach et al. (1999) Tumor Targeting 4:115-123; Kipriyanov et al. (1999) J Mol Biol 293:41-56; and Nettlebeck et al. (2001) Mol Ther 3:882-891.

The disclosure also embraces variant forms of bispecific antibodies such as the tetravalent dual variable domain immunoglobulin (DVD-Ig) molecules described in Wu et al. (2007) Nat Biotechnol 25(11):1290-1297. The DVD-Ig molecules are designed such that two different light chain variable domains (VL) from two different parent antibodies are linked in tandem directly or via a short linker by recombinant DNA techniques, followed by the light chain constant domain. For example, the DVD-Ig light chain polypeptide can contain in tandem: (a) a VL from an antibody that binds to C5a; and (b) a VL from an antibody that binds to C5b. Similarly, the heavy chain comprises two different heavy chain variable domains (VH) linked in tandem, followed by the constant domain CH1 and Fc region. For example, the DVD-Ig heavy chain polypeptide can contain in tandem: (a) a VH from an antibody that binds to C5a; and (b) a VH from an antibody that binds to C5b. In this case, expression of the two chains in a cell results in a heterotetramer containing four antigen combining sites, two that specifically bind to C5a and two that specifically bind to C5b. It is understood that VL or VH from antibodies that bind to a receptor for C5a (e.g., C5aR1 or C5L2) or a component or intermediate of the TCC (e.g., C5b-6, C5b-7, C5b-8, or C5b-9) can also be used in the preparation of a DVD-Ig molecule in accordance with the disclosure. Methods for generating DVD-Ig molecules from two parent antibodies are further described in, e.g., PCT Publication Nos. WO 08/024188 and WO 07/024715.

Also embraced is the bispecific format described in, e.g., U.S. patent application publication no. 2007000490 9.

The antibodies (e.g., monoclonal antibodies) or fragments thereof that are used to form the bispecific antibody molecules described herein can be, e.g., chimeric antibodies, humanized antibodies, rehumanized antibodies, deimmunized antibodies, fully human antibodies, and antigen-binding fragments thereof. Chimeric antibodies are produced by recombinant processes well known in the art of antibody engineering and have a non-human mammal variable region and a human constant region. Humanized antibodies correspond more closely to the sequence of human antibodies than do chimeric antibodies. Humanized variable domains are constructed in which amino acid sequences of one or more CDRs of non-human origin are grafted to human framework regions (FRs) as described in, e.g., Jones et al. (1996) Nature 321: 522-525; Riechman et al. (1988) Nature 332:323-327 and U.S. Patent No. 5,530,101. The humanized antibody can be an antibody that contains one or more human framework regions that are not germline. For example, the humanized antibody can contain one or more framework regions that were subject to somatic hypermutation and thus no longer germline *per se.* (See, e.g., Abbas, Lichtman, and Pober (2000) "Cellular and Molecular Immunology," 4th Edition,W.B. Saunders Company (ISBN:0721682332)). In some embodiments, the humanized antibody contains human germline framework regions, e.g., human germline V$_H$ regions, human germline D regions, and human germline J regions (e.g., human germline J$_H$ regions). The MRC Center for Protein Engineering maintains the online VBase database system, which includes amino acid sequences for a large number of human germline framework regions. See, e.g., Welschof et al. (1995) J Immunol Methods 179:203-214; Chothia et al. (1992) J Mol Biol 227:776-798; Williams et al. (1996) J Mol Biol 264:220-232; Marks et al. (1991) Eur J Immunol 21:985-991; and Tomlinson et al. (1995) EMBO J. 14:4628-4638. Amino acid sequences for a repertoire of suitable human germline framework regions can also be obtained from the JOINSOLVER® Germline Databases (e.g., the JOINSOLVER® Kabat databases or the JOINSOLVER® IMGT databases) maintained in part by the U.S. Department of Health and Human Services and the National Institutes of Health. See, e.g., Souto-Carneiro et al. (2004) J Immunol. 172:6790-6802.

Fully human antibodies are antibodies having variable and constant regions (if present) derived from human germline immunoglobulin sequences. Human antibodies can include amino acid residues not encoded by human germline immunoglobulin sequences (e.g., mutations introduced by random or site-specific mutagenesis *in vitro* or by somatic mutation *in vivo*). However, the term "human antibody" does not include antibodies in which CDR sequences derived from the germline of another mammalian species, such as a mouse, have been grafted onto human framework sequences (i.e., humanized antibodies). Fully human or human antibodies may be derived from transgenic mice carrying human antibody genes (carrying the variable (V), diversity (D), joining (J), and constant (C) exons) or from human cells. For example, it is possible to produce transgenic animals (e.g., mice) that are capable, upon immunization, of producing a full repertoire of human antibodies in the absence of endogenous immunoglobulin production. (See, e.g., Jakobovits et al. (1993) Proc. Natl. Acad. Sci. USA 90:2551; Jakobovits et al. (1993) Nature 362:255-258; Bruggemann et al. (1993) Year in Immunol. 7:33; and Duchosal et al. (1992) Nature 355:258.) Transgenic mice strains can be engineered to contain gene sequences from unrearranged human immunoglobulin genes. The human sequences may code for both the heavy and light chains of human antibodies and would function correctly in the mice, undergoing rearrangement to provide a wide antibody repertoire similar to that in humans. The transgenic mice can be immunized with the target protein (e.g., C5a, C5b, or C5aR).

The wholly and partially human antibodies described above are less immunogenic than their entirely murine or non-human-derived antibody counterparts. All these molecules (or derivatives thereof) are therefore less likely to evoke an immune or allergic response. Consequently, they are better suited for *in vivo* administration in humans, especially when repeated or long-term administration is necessary, as may be needed for treatment with the bispecific antibodies described herein.

Methods for Producing a Bispecific Antibody

As described above, the bispecific antibodies can be produced using a variety of techniques known in the art of molecular biology and protein chemistry. For example, a nucleic acid encoding one or both of the heavy and light chain polypeptides of a bispecific antibody can be inserted into an expression vector that contains transcriptional and translational regulatory sequences, which include, e.g., promoter sequences, ribosomal binding sites, transcriptional start and stop sequences, translational start and stop sequences, transcription terminator signals, polyadenylation signals, and enhancer or activator sequences. The regulatory sequences include a promoter and transcriptional start and stop sequences. In addition, the expression vector can include more than one replication system such that it can be maintained in two different organisms, for example in mammalian or insect cells for expression and in a prokaryotic host for cloning and amplification.

Several possible vector systems are available for the expression of cloned heavy chain and light chain polypeptides from nucleic acids in mammalian cells. One class of vectors relies upon the integration of the desired gene sequences into the host cell genome. Cells which have stably integrated DNA can be selected by simultaneously introducing drug resistance genes such as *E. coli* gpt (Mulligan and Berg (1981) Proc. Natl. Acad. Sci. USA, 78:2072) or Tn5 neo (Southern and Berg (1982) Mol. Appl. Genet. 1:327). The selectable marker gene can be either linked to the DNA gene sequences to be expressed, or introduced into the same cell by co-transfection (Wigler et al. (1979) Cell 16:77). A second class of vectors utilizes DNA elements which confer autonomously replicating capabilities to an extrachromosomal plasmid. These vectors can be derived from animal viruses, such as bovine papillomavirus (Sarver et al. (1982) Proc. Natl. Acad. Sci. USA, 79:7147), polyoma virus (Deans et al. (1984) Proc. Natl. Acad. Sci. USA 81:1292), or SV40 virus (Lusky and Botchan (1981) Nature 293:79).

The expression vectors can be introduced into cells in a manner suitable for subsequent expression of the nucleic acid. The method of introduction is largely dictated by the targeted cell type, discussed below. Exemplary methods include $CaPO_4$ precipitation, liposome fusion, lipofectin, electroporation, viral infection, dextran-mediated transfection, polybrene-mediated transfection, protoplast fusion, and direct microinjection.

Appropriate host cells for the expression of bispecific antibodies include yeast, bacteria, insect, plant, and mammalian cells. Of particular interest are bacteria such as *E. coli,* fungi such as *Saccharomyces cerevisiae* and *Pichia pastoris,* insect cells such as SF9, mammalian cell lines (e.g., human cell lines), as well as primary cell lines (e.g., primary mammalian cells).

In some embodiments, a bispecific antibody can be expressed in, and purified from, transgenic animals (e.g., transgenic mammals). For example, a bispecific antibody that binds to C5a and C5b can be produced in transgenic non-human mammals (e.g., rodents, sheep or goats) and isolated from milk as described in, e.g., Houdebine (2002) Curr Opin Biotechnol 13(6):625-629; van Kuik-Romeijn et al. (2000) Transgenic Res 9(2):155-159; and Pollock et al. (1999) J Immunol Methods 231(1-2):147-157.

The bispecific antibodies described herein can be produced from cells by culturing a host cell transformed with the expression vector containing nucleic acid encoding the antibodies, under conditions, and for an amount of time, sufficient to allow expression of the proteins. Such conditions for protein expression will vary with the choice of the expression vector and the host cell, and will be easily ascertained by one skilled in the art through routine experimentation. For example, antibodies expressed in *E. coli* can be refolded from inclusion bodies (see, e.g., Hou et al. (1998) Cytokine 10:319-30). Bacterial expression systems and methods for their use are well known in the art (see Current Protocols in Molecular Biology, Wiley & Sons, and Molecular Cloning - A Laboratory Manual - 3rd Ed., Cold Spring Harbor Laboratory Press, New York (2001)). The choice of codons, suitable expression vectors and suitable host cells will vary depending on a number of factors, and may be easily optimized as needed. A bispecific antibody described herein can be expressed in mammalian cells or in other expression systems including but not limited to yeast, baculovirus, and *in vitro* expression systems (see, e.g., Kaszubska et al. (2000) Protein Expression and Purification 18:213-220).

Following expression, the bispecific antibodies can be isolated. The term "purified" or "isolated" as applied to any of the proteins described herein (e.g., a bispecific antibody) refers to a polypeptide that has been separated or purified from components (e.g., proteins or other naturally-occurring biological or organic molecules) which naturally accompany it, e.g., other proteins, lipids, and nucleic acid in a prokaryote expressing the proteins. Typically, a polypeptide is purified when it constitutes at least 60 (e.g., at least 65, 70, 75, 80, 85, 90, 92, 95, 97, or 99) %, by weight, of the total protein in a sample.

A bispecific antibody can be isolated or purified in a variety of ways known to those skilled in the art depending on what other components are present in the sample. Standard purification methods include electrophoretic, molecular, immunological, and chromatographic techniques, including ion exchange, hydrophobic, affinity, and reverse-phase HPLC chromatography. For example, a bispecific antibody that binds to C5a and C5aR can be purified using a standard anti-antibody column or, e.g., a protein-A or protein-G column. Ultrafiltration and diafiltration techniques, in conjunction with protein concentration, are also useful. See, e.g., Scopes (1994) "Protein Purification, 3rd edition," Springer-Verlag, New York City, New York. The degree of purification necessary will vary depending on the desired use. In some instances, no purification of the expressed antibody thereof will be necessary.

Methods for determining the yield or purity of a purified antibody are known in the art and include, e.g., Bradford assay, UV spectroscopy, Biuret protein assay, Lowry protein assay, amido black protein assay, high pressure liquid chromatography (HPLC), mass spectrometry (MS), and gel electrophoretic methods (e.g., using a protein stain such as Coomassie Blue or colloidal silver stain).

In some embodiments, endotoxin can be removed from the bispecific antibodies preparations. Methods for removing endotoxin from a protein sample are known in the art and exemplified in the working examples. For example, endotoxin can be removed from a protein sample using a variety of commercially available reagents including, without limitation, the ProteoSpin™ Endotoxin Removal Kits (Norgen Biotek Corporation), Detoxi-Gel Endotoxin Removal Gel (Thermo Scientific; Pierce Protein Research Products), MiraCLEAN® Endotoxin Removal Kit (Mirus), or Acrodisc™ - Mustang® E membrane (Pall Corporation).

Methods for detecting and/or measuring the amount of endotoxin present in a sample (both before and after purification) are known in the art and commercial kits are available. For example, the concentration of endotoxin in a protein sample can be determined using the QCL-1000 Chromogenic kit (BioWhittaker), the limulus amebocyte lysate (LAL)-based kits such as the Pyrotell®, Pyrotell®-T, Pyrochrome®, Chromo-LAL, and CSE kits available from the Associates of Cape Cod Incorporated.

Modification of the Bispecific Antibodies

The bispecific antibodies can be modified following their expression and purification. The modifications can be covalent or non-covalent modifications. Such modifications can be introduced into the bispecific antibodies by, e.g., reacting targeted amino acid residues of the polypeptide with an organic derivatizing agent that is capable of reacting with selected side chains or terminal residues. Suitable sites for modification can be chosen using any of a variety of criteria including, e.g., structural analysis or amino acid sequence analysis of the bispecific antibodies.

In some embodiments, the bispecific antibodies can be conjugated to a heterologous moiety. The heterologous moiety can be, e.g., a heterologous polypeptide, a therapeutic agent (e.g., a toxin or a drug), or a detectable label such as, but not limited to, a radioactive label, an enzymatic label, a fluorescent label, or a luminescent label. Suitable heterologous polypeptides include, e.g., an antigenic tag (e.g., FLAG, polyhistidine, hemagglutinin (HA), glutathione-S-transferase (GST), or maltose-binding protein (MBP)) for use in purifying the antibodies. Heterologous polypeptides also include polypeptides that are useful as diagnostic or detectable markers, for example, luciferase, green fluorescent protein (GFP), or chloramphenicol acetyl transferase (CAT). Where the heterologous moiety is a polypeptide, the moiety can be incorporated into a bispecific antibody described herein as a fusion protein.

Suitable radioactive labels include, e.g., $^{32}P$, $^{33}P$, $^{14}C$, $^{125}I$, $^{131}I$, $^{35}S$, and $^3H$. Suitable fluorescent labels include, without limitation, fluorescein, fluorescein isothiocyanate (FITC), green fluorescence protein (GFP), DyLight 488, phycoerythrin (PE), propidium iodide (PI), PerCP, PE-Alexa Fluor® 700, Cy5, allophycocyanin, and Cy7. Luminescent labels include, e.g., any of a variety of luminescent lanthanide (e.g., europium or terbium) chelates. For example, suitable europium chelates include the europium chelate of diethylene triamine pentaacetic acid (DTPA) or tetraazacyclododecane-1,4,7,10-tetraacetic acid (DOTA). Enzymatic labels include, e.g., alkaline phosphatase, CAT, luciferase, and horseradish peroxidase.

Two proteins (e.g., a bispecific antibody and a heterologous moiety) can be cross-linked using any of a number of known chemical cross linkers. Examples of such cross linkers are those which link two amino acid residues via a linkage that includes a "hindered" disulfide bond. In these linkages, a disulfide bond within the cross-linking unit is protected (by hindering groups on either side of the disulfide bond) from reduction by the action, for example, of reduced glutathione or the enzyme disulfide reductase. One suitable reagent, 4-succinimidyloxycarbonyl-$\alpha$-methyl-$\alpha$ (2-pyridyldithio) toluene (SMPT), forms such a linkage between two proteins utilizing a terminal lysine on one of the proteins and a terminal cysteine on the other. Heterobifunctional reagents that cross-link by a different coupling moiety on each protein can also be used. Other useful cross-linkers include, without limitation, reagents which link two amino groups (e.g., N-5-azido-2-nitrobenzoyloxysuccinimide), two sulfhydryl groups (e.g., 1,4-bis-maleimidobutane), an amino group and a sulfhydryl group (e.g., m-maleimidobenzoyl-N-hydroxysuccinimide ester), an amino group and a carboxyl group (e.g., 4-[p-azidosalicylamido]butylamine), and an amino group and a guanidinium group that is present in the side chain of arginine (e.g., p-azidophenyl glyoxal monohydrate).

In some embodiments, a radioactive label can be directly conjugated to the amino acid backbone of the bispecific antibody. Alternatively, the radioactive label can be included as part of a larger molecule (e.g., $^{125}I$ in meta-[$^{125}I$]iodophenyl-N-hydroxysuccinimide ([$^{125}I$]mIPNHS) which binds to free amino groups to form meta-iodophenyl (mIP) derivatives of relevant proteins (see, e.g., Rogers et al. (1997) J. Nucl. Med. 38:1221-1229) or chelate (e.g., to DOTA or DTPA) which is in turn bound to the protein backbone. Methods of conjugating the radioactive labels or larger molecules/chelates containing them to the bispecific antibodies described herein are known in the art. Such methods involve incubating the proteins with the radioactive label under conditions (e.g., pH, salt concentration, and/or temperature) that facilitate binding of the radioactive label or chelate to the protein (see, e.g., U.S. Patent No. 6,001,329).

Methods for conjugating a fluorescent label (sometimes referred to as a "fluorophore") to a protein (e.g., a bispecific antibody) are known in the art of protein chemistry. For example, fluorophores can be conjugated to free amino groups (e.g., of lysines) or sulfhydryl groups (e.g., cysteines) of proteins using succinimidyl (NHS) ester or tetrafluorophenyl (TFP) ester moieties attached to the fluorophores. In some embodiments, the fluorophores can be conjugated to a heterobifunctional cross-linker moiety such as sulfo-SMCC. Suitable conjugation methods involve incubating a bispecific antibody protein with the fluorophore under conditions that facilitate binding of the fluorophore to the protein. See, e.g., Welch and Redvanly (2003) "Handbook of Radiopharmaceuticals: Radiochemistry and Applications," John Wiley and Sons (ISBN 0471495603).

In some embodiments, the bispecific antibodies can be modified, e.g., with a moiety that improves the stabilization and/or retention of the antibodies in circulation, e.g., in blood, serum, or other tissues. For example, the bispecific antibody can be PEGylated as described in, e.g., Lee et al. (1999) Bioconjug. Chem 10(6): 973-8; Kinstler et al. (2002) Advanced Drug Deliveries Reviews 54:477-485; and Roberts et al. (2002) Advanced Drug Delivery Reviews 54:459-476. The stabilization moiety can improve the stability, or retention of, the antibody by at least 1.5 (e.g., at least 2, 5, 10, 15, 20, 25, 30, 40, or 50 or more) fold.

In some embodiments, the bispecific antibodies described herein can be glycosylated. In some embodiments, a bispecific antibody described herein can be subjected to enzymatic or chemical treatment, or produced from a cell, such that the antibody has reduced or absent glycosylation. Methods for producing antibodies with reduced glycosylation are known in the art and described in, e.g., U.S. patent no. 6,933,368; Wright et al. (1991) EMBO J 10(10):2717-2723; and Co et al. (1993) Mol Immunol 30:1361.

Pharmaceutical Compositions

Compositions containing a bispecific antibody described herein can be formulated as a pharmaceutical composition, e.g., for administration to a subject for the treatment or prevention of a complement-associated disorder. The pharmaceutical compositions will generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, e.g., an acid addition salt or a base addition salt (see e.g., Berge et al. (1977) J Pharm Sci 66:1-19).

The compositions can be formulated according to standard methods. Pharmaceutical formulation is a well-established art, and is further described in, e.g., Gennaro (2000) "Remington: The Science and Practice of Pharmacy," 20th Edition, Lippincott, Williams & Wilkins (ISBN: 0683306472); Ansel et al. (1999) "Pharmaceutical Dosage Forms and Drug Delivery Systems," 7th Edition, Lippincott Williams & Wilkins Publishers (ISBN: 0683305727); and Kibbe (2000) "Handbook of Pharmaceutical Excipients American Pharmaceutical Association," 3rd Edition (ISBN: 091733096X). In some embodiments, a composition can be formulated, for example, as a buffered solution at a suitable concentration and suitable for storage at 2-8°C (e.g., 4°C). In some embodiments, a composition can be formulated for storage at a temperature below 0°C (e.g., -20°C or -80°C). In some embodiments, the composition can be formulated for storage for up to 2 years (e.g., one month, two months, three months, four months, five months, six months, seven months, eight months, nine months, 10 months, 11 months, 1 year, 1½ years, or 2 years) at 2-8°C (e.g., 4°C). Thus, in some embodiments, the compositions described herein are stable in storage for at least 1 year at 2-8°C (e.g., 4°C).

The pharmaceutical compositions can be in a variety of forms. These forms include, e.g., liquid, semi-solid and solid dosage forms, such as liquid solutions (e.g., injectable and infusible solutions), dispersions or suspensions, tablets, pills, powders, liposomes and suppositories. The preferred form depends, in part, on the intended mode of administration and therapeutic application. For example, compositions containing a bispecific antibody intended for systemic or local delivery can be in the form of injectable or infusible solutions. Accordingly, the compositions can be formulated for administration by a parenteral mode (e.g., intravenous, subcutaneous, intraperitoneal, or intramuscular injection). "Parenteral administration," "administered parenterally," and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid and intrasternal injection and infusion (see below).

The compositions can be formulated as a solution, microemulsion, dispersion, liposome, or other ordered structure suitable for stable storage at high concentration. Sterile injectable solutions can be prepared by incorporating an antibody described herein in the required amount in an appropriate solvent with one or a combination of ingredients enumerated above, as required, followed by filtered sterilization. Generally, dispersions are prepared by incorporating an antibody described herein into a sterile vehicle that contains a basic dispersion medium and the required other ingredients from those enumerated above. In the case of sterile powders for the preparation of sterile injectable solutions, methods for preparation include vacuum drying and freeze-drying that yield a powder of an antibody described herein plus any additional desired ingredient (see below) from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition a reagent that delays absorption, for example, monostearate salts, and gelatin.

In certain embodiments, an antibody described herein can be prepared with a carrier that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are known in the art. See, e.g., J.R. Robinson (1978) "Sustained and Controlled Release Drug Delivery Systems," Marcel Dekker, Inc., New York.

In some embodiments, an antibody described herein can be formulated in a composition suitable for intrapulmonary administration (e.g., for administration via nebulizer) to a mammal such as a human. Methods for preparing such compositions are well known in the art and described in, e.g., U.S. Patent Application Publication No. 20080202513; U.S. Patent Nos. 7,112,341 and 6,019,968; and PCT Publication Nos. WO 00/061178 and WO 06/12225 7.

Dry powder inhaler formulations and suitable systems for administration of the formulations are described in, e.g., U.S. Patent Application Publication No. 20070235029, PCT Publication No. WO 00/69887; and U.S. Patent No. 5,997,848.

Nucleic acids encoding an antibody can be incorporated into a gene construct to be used as a part of a gene therapy protocol to deliver nucleic acids that can be used to express and produce agents within cells (see below). Expression constructs of such components may be administered in any therapeutically effective carrier, e.g. any formulation or composition capable of effectively delivering the component gene to cells *in vivo.* Approaches include insertion of the subject gene in viral vectors including recombinant retroviruses, adenovirus, adeno-associated virus, lentivirus, and herpes simplex vitus-1 (HSV-1), or recombinant bacterial or eukaryotic plasmids. Viral vectors can transfect cells directly; plasmid DNA can be delivered with the help of, for example, cationic liposomes (lipofectin) or derivatized (e.g., antibody conjugated), polylysine conjugates, gramicidin S, artificial viral envelopes or other such intracellular carriers, as well as direct injection of the gene construct or $CaPO_4$ precipitation (see, e.g., WO04/060407) carried out *in vivo.* (See also, *"Ex vivo* Approaches," below.) Examples of suitable retroviruses include pLJ, pZIP, pWE and pEM which are known to those skilled in the art (see, e.g., Eglitis et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:6141-6145; Huber et al. (1991) Proc. Natl. Acad. Sci. USA 88:8039-8043; Ferry et al. (1991) Proc. Natl. Acad. Sci. USA 88:8377-8381; Chowdhury et al. (1991) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad. Sci. USA 89:10892-10895; Hwu et al. (1993) J. Immunol. 150:4104-4115; U.S. Patent Nos. 4,868,116 and 4,980,286; PCT Publication Nos. WO89/07136, WO89/02468, WO89/05345, and WO92/07573). Another viral gene delivery system utilizes adenovirus-derived vectors (see, e.g., Berkner et al. (1988) BioTechniques 6:616; Rosenfeld et al. (1991) Science 252:431-434; and Rosenfeld et al. (1992) Cell 68:143-155). Suitable adenoviral vectors derived from the adenovirus strain Ad type 5 dl324 or other strains of adenovirus (e.g., Ad2, Ad3, Ad7, etc.) are known to those skilled in the art. Yet another viral vector system useful for delivery of the subject gene is the adeno-associated virus (AAV). See, e.g., Flotte et al. (1992) Am J Respir Cell Mol Biol 7:349-356; Samulski et al. (1989) J Virol. 63:3822-3828; and McLaughlin et al. (1989) J Virol 62:1963-1973.

In some embodiments, a bispecific antibody described herein can be formulated with one or more additional active agents useful for treating or preventing a complement-associated disorder (e.g., an AP-associated disorder or a CP-associated disorder) in a subject. Additional agents for treating a complement-associated disorder in a subject will vary depending on the particular disorder being treated, but can include, without limitation, an antihypertensive (e.g., an angiotensin-converting enzyme inhibitor) [for use in treating, e.g., HELLP syndrome], an anticoagulant, a corticosteroid (e.g., prednisone), or an immunosuppressive agent (e.g., vincristine or cyclosporine A). Examples of anticoagulants include, e.g., warfarin (Coumadin), heparin, phenindione, fondaparinux, idraparinux, and thrombin inhibitors (e.g., argatroban, lepirudin, bivalirudin, or dabigatran). A bispecific antibody described herein can also be formulated with a fibrinolytic agent (e.g., ancrod, ε-aminocaproic acid, antiplasmin-$a_1$, prostacyclin, and defibrotide) for the treatment of a complement-associated disorder. In some embodiments, a bispecific antibody can be formulated with a lipid-lowering agent such as an inhibitor of hydroxymethylglutaryl CoA reductase. In some embodiments, a bispecific antibody can be formulated with, or for use with, an anti-CD20 agent such as rituximab (Rituxan™; Biogen Idec, Cambridge, MA). In some embodiments, e.g., for the treatment of RA, the bispecific antibody can be formulated with one or both of infliximab (Remicade®; Centocor, Inc.) and methotrexate (Rheumatrex®, Trexall®). In some embodiments, a bispecific antibody described herein can be formulated with a non-steroidal anti-inflammatory drug (NSAID). Many different NSAIDS are available, some over the counter including ibuprofen (Advil ®, Motrin®, Nuprin ®) and naproxen (Alleve®) and many others are available by prescription including meloxicam (Mobic®), etodolac (Lodine®), nabumetone (Relafen®), sulindac (Clinoril®), tolementin (Tolectin®), choline magnesium salicylate (Trilasate®), diclofenac (Cataflam®, Voltaren®, Arthrotec®), Diflusinal (Dolobid®), indomethicin (Indocin®), Ketoprofen (Orudis®, Oruvail®), Oxaprozin (Daypro®), and piroxicam (Feldene®). In some embodiments a bispecific antibody can be formulated for use with an anti-hypertensive, an anti-seizure agent (e.g., magnesium sulfate), or an anti-thrombotic agent. Anti-hypertensives include, e.g., labetalol, hydralazine, nifedipine, calcium channel antagonists, nitroglycerin, or sodium nitroprussiate. (See, e.g., Mihu et al. (2007) J Gasrointestin Liver Dis 16(4):419-424.) Anti-thrombotic agents include, e.g., heparin, antithrombin, prostacyclin, or low dose aspirin.

In some embodiments, a bispecific antibody described herein can be formulated for administration to a subject along with intravenous gamma globulin therapy (IVIG), plasmapheresis, plasma replacement, or plasma exchange. In some embodiments, a bispecific antibody can be formulated for use before, during, or after, a kidney transplant.

When a bispecific antibody is to be used in combination with a second active agent, the agents can be formulated separately or together. For example, the respective pharmaceutical compositions can be mixed, e.g., just prior to administration, and administered together or can be administered separately, e.g., at the same or different times (see below).

As described above, a composition can be formulated such that it includes a therapeutically effective amount of a bispecific antibody described herein. In some embodiments, a composition can be formulated to include a sub-therapeutic amount of a bispecific antibody and a sub-therapeutic amount of one or more additional active agents such that the components in total are therapeutically effective for treating or preventing a complement-associated disorder (e.g., an alternative complement pathway-associated complement disorder or a classical complement pathway-associated disorder). In some embodiments, a composition can be formulated to include, e.g., a first bispecific antibody that binds to C5aR and C5a and a second bispecific antibody that binds to C5b and C5a in accordance with the disclosure, each at a sub-therapeutic dose, such that the antibodies in total are at a concentration that is therapeutically effective for treating a complement-associated disorder. Methods for determining a therapeutically effective dose of an agent such as a therapeutic antibody are known in the art and described herein.

Methods for Treatment

The above-described compositions (e.g., any of the bispecific antibodies described herein or pharmaceutical compositions thereof) are useful in, *inter alia*, methods for treating or preventing a variety of complement-associated disorders (e.g., AP-associated disorders or CP-associated disorders) in a subject. The compositions can be administered to a subject, e.g., a human subject, using a variety of methods that depend, in part, on the route of administration. The route can be, e.g., intravenous injection or infusion (IV), subcutaneous injection (SC), intraperitoneal (IP), intrapulmonary, intraocular, or intramuscular injection.

Administration can be achieved by, e.g., local infusion, injection, or by means of an implant. The implant can be of a porous, non-porous, or gelatinous material, including membranes, such as sialastic membranes, or fibers. The implant can be configured for sustained or periodic release of the composition to the subject. (See, e.g., U.S. Patent Application Publication No. 20080241223; U.S. Patent Nos. 5,501,856; 4,863,457; and 3,710,795; EP488401; and EP 430539).

The composition can be delivered to the subject by way of an implantable device based on, e.g., diffusive, erodible, or convective systems, e.g., osmotic pumps, biodegradable implants, electrodiffusion systems, electroosmosis systems, vapor pressure pumps, electrolytic pumps, effervescent pumps, piezoelectric pumps, erosion-based systems, or electromechanical systems.

A suitable dose of a bispecific antibody described herein, which dose is capable of treating or preventing a complement-associated disorder in a subject, can depend on a variety of factors including, e.g., the age, sex, and weight of a subject to be treated and the particular inhibitor compound used. For example, a different dose of an antibody that binds to C5a and C5b may be required to treat a subject with RA as compared to the dose of an antibody that binds to C5a and C5aR1 that is required to treat the same subject. Other factors affecting the dose administered to the subject include, e.g., the type or severity of the complement-associated disorder. For example, a subject having RA may require administration of a different dosage of an antibody that binds to C5a and C5b than a subject with AMD. Other factors can include, e.g., other medical disorders concurrently or previously affecting the subject, the general health of the subject, the genetic disposition of the subject, diet, time of administration, rate of excretion, drug combination, and any other additional therapeutics that are administered to the subject. It should also be understood that a specific dosage and treatment regimen for any particular subject will depend upon the judgment of the treating medical practitioner (e.g., doctor or nurse).

An antibody described herein can be administered as a fixed dose, or in a milligram per kilogram (mg/kg) dose. In some embodiments, the dose can also be chosen to reduce or avoid production of antibodies or other host immune responses against one or more of the active antibodies in the composition. While in no way intended to be limiting, exemplary dosages of an antibody include, e.g., 1-100 $\mu$g/kg, 0.5-50 $\mu$g/kg, 0.1-100 $\mu$g/kg, 0.5-25 $\mu$g/kg, 1-20 $\mu$g/kg, and 1-10 $\mu$g/kg, 1-100 mg/kg, 0.5-50 mg/kg, 0.1-100 mg/kg, 0.5-25 mg/kg, 1-20 mg/kg, and 1-10 mg/kg. Exemplary dosages of an antibody described herein include, without limitation, 0.1 $\mu$g/kg, 0.5 $\mu$g/kg, 1.0 $\mu$g/kg, 2.0 $\mu$g/kg, 4 $\mu$g/kg, and 8 $\mu$g/kg, 0.1 mg/kg, 0.5 mg/kg, 1.0 mg/kg, 2.0 mg/kg, 4 mg/kg, and 8 mg/kg.

A pharmaceutical composition can include a therapeutically effective amount of an antibody described herein. Such effective amounts can be readily determined by one of ordinary skill in the art based, in part, on the effect of the administered antibody, or the combinatorial effect of the antibody and one or more additional active agents, if more than one agent is used. A therapeutically effective amount of an antibody described herein can also vary according to factors such as the disease state, age, sex, and weight of the individual, and the ability of the antibody (and one or more additional active agents) to elicit a desired response in the individual, e.g., amelioration of at least one condition parameter, e.g., amelioration of at least one symptom of the complement-associated disorder. For example, a therapeutically effective amount of an antibody that binds to C5a and C5b can inhibit (lessen the severity of or eliminate the occurrence of) and/or prevent a particular disorder, and/or any one of the symptoms of the particular disorder known in the art or described herein. A therapeutically effective amount is also one in which any toxic or detrimental effects of the composition are outweighed by the therapeutically beneficial effects.

Suitable human doses of any of the "bispecific antibodies described herein can further be evaluated in, e.g., Phase I dose escalation studies. See, e.g., van Gurp et al. (2008) Am J Transplantation 8(8):1711-1718; Hanouska et al. (2007) Clin Cancer Res 13(2, part 1):523-531; and Hetherington et al. (2006) Antimicrobial Agents and Chemotherapy 50(10): 3499-3500.

The terms "therapeutically effective amount" or "therapeutically effective dose," or similar terms used herein are intended to mean an amount of an agent that will elicit the desired biological or medical response (e.g., an improvement in one or more symptoms of a complement-associated disorder). In some embodiments, a composition described herein contains a therapeutically effective amount of an antibody, which specifically binds to C5a and C5b. A composition described herein may contain a therapeutically effective amount of an antibody, which specifically binds to C5a and C5aR1. A composition described herein may contain a therapeutically effective amount of an antibody, which specifically binds to C5b and C5aR. A composition described herein may contain a therapeutically effective amount of an antibody that specifically binds to: (i) C5a or a cellular receptor for C5a and (ii) a component or intermediate of the TCC including, e.g., C5b-6, C5b-7, C5b-8, or C5b-9. In some embodiments, the composition contains any of the antibodies described herein and one or more (e.g., three, four, five, six, seven, eight, nine, 10, or 11 or more) additional therapeutic agents such that the composition as a whole is therapeutically effective. For example, a composition can contain a bispecific antibody described herein and an immunosuppressive agent, wherein the antibody and agent are each at a concentration that when combined are therapeutically effective for treating or preventing a complement-associated disorder in a subject.

Toxicity and therapeutic efficacy of such compositions can be determined by known pharmaceutical procedures in cell cultures or experimental animals (e.g., animal models of any of the complement-associated disorders described herein). These procedures can be used, e.g., for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. A bispecific antibody (e.g., an antibody that binds to C5a and C5b, an antibody that binds to C5a and C5aR, an antibody that binds to C5b and C5aR, an antibody that binds to C5a and a component or intermediate of the TCC) that exhibits a high therapeutic index is preferred. While compositions that exhibit toxic side effects may be used, care should be taken to design a delivery system that targets such compounds to the site of affected tissue and to minimize potential damage to normal cells and, thereby, reduce side effects.

The data obtained from the cell culture assays and animal studies can be used in formulating a range of dosage for use in humans. The dosage of such antibodies lies generally within a range of circulating concentrations of the bispecific antibodies that include the $ED_{50}$ with little or no toxicity. The dosage may vary within this range depending upon the dosage form employed and the route of administration utilized. For a bispecific antibody used as described herein (e.g., for treating or preventing a complement-associated disorder), the therapeutically effective dose can be estimated initially from cell culture assays. A dose can be formulated in animal models to achieve a circulating plasma concentration range that includes the $IC_{50}$ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography or by ELISA.

In some embodiments, the methods can be performed in conjunction with other therapies for complement-associated disorders: For example, the composition can be administered to a subject at the same time, prior to, or after, plasmapheresis, IVIG therapy, plasma replacement, or plasma exchange. See, e.g., Appel et al. (2005) JAm. Soc Nephrol. 16:1392-1404. In some embodiments, a bispecific antibody described herein is not administered in conjunction with IVIG. In some embodiments, the composition can be administered to a subject at the same time, prior to, or after, a kidney transplant.

A "subject," as used herein, can be any mammal. For example, a subject can be a human, a non-human primate (e.g., monkey, baboon, or chimpanzee), a horse, a cow, a pig, a sheep, a goat, a dog, a cat, a rabbit, a guinea pig, a gerbil, a hamster, a rat, or a mouse. In some embodiments, the subject is an infant (e.g., a human infant).

As used herein, a subject "in need of prevention," "in need of treatment," or "in need thereof," refers to one, who by the judgment of an appropriate medical practitioner (e.g., a doctor, a nurse, or a nurse practitioner in the case of humans; a veterinarian in the case of non-human mammals), would reasonably benefit from a given treatment (such as treatment with a composition comprising an antibody that binds to C5a and C5b, an antibody that binds to the C5a and C5aR, or an antibody that binds to C5b and C5aR.

As described above, the bispecific antibodies described herein can be used to treat a variety of complement-associated disorders such as, e.g., AP-associated disorders and/or CP-associated disorders. Such disorders include, without limitation, rheumatoid arthritis (RA); antiphospholipid antibody syndrome; lupus nephritis; asthma; ischemia-reperfusion injury; atypical hemolytic uremic syndrome (aHUS); typical or infectious hemolytic uremic syndrome (tHUS); dense deposit disease (DDD); paroxysmal nocturnal hemoglobinuria (PNH); neuromyelitis optica (NMO); multifocal motor neuropathy (MMN); multiple sclerosis (MS); Degos' disease; macular degeneration (e.g., age-related macular degeneration (AMD)); hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; and traumatic brain injury. (See, e.g., Holers (2008) Immunological Reviews 223:300-316 and Holers and Thurman (2004) Molecular Immunology 41:147-152.) In some embodiments, the complement-associated disorder is a complement-associated vascular disorder such as, but not limited to, a cardiovascular disorder, myocarditis, a cerebrovascular disorder, a peripheral (e.g., musculoskeletal) vascular disorder, a renovascular disorder, a mesenteric/enteric vascular disorder, revascularization to transplants and/or replants, vasculitis, Henoch-Schönlein purpura nephritis, systemic lupus erythematosus-associated vasculitis, vasculitis associated with rheumatoid arthritis, immune complex vasculitis, Takayasu's disease, dilated cardiomyopathy, diabetic angiopathy, Kawasaki's disease (arteritis), venous gas embolus (VGE), and restenosis following stent placement, rotational atherectomy, and percutaneous transluminal coronary angioplasty (PTCA). (See, e.g., U.S. patent application publication no. 20070172483.) Additional complement-associated disorders include, without limitation, MG, CAD, dermatomyositis, Graves' disease, atherosclerosis, Alzheimer's disease, Guillain-Barré Syndrome, graft rejection (e.g., transplant rejection, e.g., kidney, liver, heart, bone marrow, or skin transplant rejection), systemic inflammatory response sepsis, septic shock, spinal cord injury, glomerulonephritis, Hashimoto's thyroiditis, type I diabetes, psoriasis, pemphigus, AIHA, ITP, Goodpasture syndrome, antiphospholipid syndrome (APS), and catastrophic APS (CAPS).

As used herein, a subject "at risk for developing a complement-associated disorder" (e.g., an AP-associated disorder or a CP-associated disorder) is a subject having one or more (e.g., two, three, four, five, six, seven, or eight or more) risk factors for developing the disorder. Risk factors will vary depending on the particular complement-associated disorder, but are well known in the art of medicine. For example, risk factors for developing DDD include, e.g., a predisposition to develop the condition, i.e., a family history of the condition or a genetic predisposition to develop the condition such as, e.g., one or more mutations in the gene encoding complement factor H (CFH), complement factor H-related 5 (CFHR5), and/or complement component C3 (C3). Such DDD-associated mutations as well methods for determining whether a subject carries one or more of the mutations are known in the art and described in, e.g., Licht et al. (2006) Kidney Int. 70:42-50; Zipfel et al. (2006) "The role of complement in membranoproliferative glomerulonephritis," In: Complement and Kidney Disease, Springer, Berlin, pages 199-221; Ault et al. (1997) JBiol. Chew. 272:25168-75; Abrera-Abeleda et al. (2006) J Med. Genet 43:582-589; Poznansky et al. (1989) J Immunol. 143:1254-1258; Jansen et al. (1998) Kidney Int. 53:331-349; and Hegasy et al. (2002) Am J Pathol 161:2027-2034. Thus, a human at risk for developing DDD can be, e.g., one who has one or more DDD-associated mutations in the gene encoding CFH or one with a family history of developing the disease.

Risk factors for TTP are well known in the art of medicine and include, e.g., a predisposition to develop the condition, i.e., a family history of the condition or a genetic predisposition to develop the condition such as, e.g., one or more mutations in the ADAMTS13 gene. ADAMTS13 mutations associated with TTP are reviewed in detail in, e.g., Levy et al. (2001) Nature 413:488-494; Kokame et al. (2004) Semin. Hematol. 41:34-40; Licht et al. (2004) Kidney Int. 66:955-958; and Noris et al. (2005) J. Am. Soc. Nephrol. 16:1177-1183. Risk factors for TTP also include those conditions or agents that are known to precipitate TTP, or TTP recurrence, such as, but not limited to, cancer, bacterial infections (e.g., *Bartonella sp.* infections), viral infections (e.g., HIV and Kaposi's sarcoma virus), pregnancy, or surgery. See, e.g., Avery et al. (1998) American Journal of Hematology 58:148-149 and Tsai, *supra*). TTP, or recurrence of TTP, has also been associated with the use of certain therapeutic agents (drugs) including, e.g., ticlopidine, FK506, corticosteroids, tamoxifen, or cyclosporin A (see, e.g., Gordon et al. (1997) Seminars in Hematology 34(2):140-147). Hereinafter, such manifestations of TTP may be, where appropriate, referred to as, e.g., "infection-associated TTP," "pregnancy-associated TTP," or "drug-associated TTP." Thus, a human at risk for developing TTP can be, e.g., one who has one or more TTP-associated mutations in the ADAMTS13 gene. A human at risk for developing a recurrent form of TTP can be one, e.g., who has had TTP and has an infection, is pregnant, or is undergoing surgery.

Risk factors for aHUS are well known in the art of medicine and include, e.g., a predisposition to develop the condition, i.e., a family history of the condition or a genetic predisposition to develop the condition such as, e.g., one or more mutations in complement Factor H (CFH), membrane cofactor protein (MCP; CD46), C4b-binding protein, complement factor B (CFB), or complement factor I (CFI). (See, e.g., Warwicker et al. (1998) Kidney Int. 53:836-844; Richards et al. (2001) Am JHum Genet 68:485-490; Caprioli et al. (2001) Am Soc Nephrol 12:297-307; Neuman et al. (2003) JMed Genet 40:676-681; Richards et al. (2006) Proc Natl Acad Sci USA 100:12966-12971; Fremeaux-Bacchi et al. (2005) JAm Soc Nephrol 17:2017-2025; Esparza-Gordillo et al. (2005) Hum Mol Genet 14:703-712; Goicoechea de Jorge et al. (2007) Proc Natl Acad Sci USA 104(1):240-245; Blom et al. (2008) J Immunol. 180(9):6385-91; and Fremeaux-Bacchi et al. (2004) JMedical Genet 41:e84). (See also Kavanagh et al. (2006) *supra*.) Risk factors also include, e.g., infection with *Streptococcus pneumoniae,* pregnancy, cancer, exposure to anti-cancer agents (e.g., quinine, mitomycin C, cisplatin, or bleomycin), exposure to immunotherapeutic agents (e.g., cyclosporine, OKT3, or interferon), exposure to anti-platelet agents (e.g., ticlopidine or clopidogrel), HIV infection, transplantation, autoimmune disease, and combined methylmalonic aciduria and homocystinuria (cblC). See, e.g., Constantinescu et al. (2004) Am J Kidney Dis 43:976-982; George (2003) Curr Opin Hematol 10:339-344; Gottschall et al. (1994) Am J Hematol 47:283-289; Valavaara et al. (1985) Cancer 55:47-50; Miralbell et al. (1996) J Clin Oncol 14:579-585; Dragon-Durey et al. (2005) J Am Soc Nephrol 16:555-63; and Becker et al. (2004) Clin Infect Dis 39:S267-S275.

Risk factors for HELLP are well known in the art of medicine and include, e.g., multiparous pregnancy, maternal age over 25 years, Caucasian race, the occurrence of preeclampsia or HELLP in a previous pregnancy, and a history of poor pregnancy outcome. (See, e.g., Sahin et al. (2001) Nagoya Med J 44(3):145-152; Sullivan et al. (1994) Am J Obstet Gynecol 171:940-943; and Padden et al. (1999) Am Fam Physician 60(3):829-836.) For example, a pregnant, Caucasian woman who developed preeclampsia during a first pregnancy can be one at risk for developing HELLP syndrome during, or following, a second pregnancy.

Risk factors for CAD are well known in the art of medicine and include, e.g., conditions or agents that are known to precipitate CAD, or CAD recurrence, such as, but not limited to, neoplasms or infections (e.g., bacterial and viral infections). Conditions known to be associated with the development of CAD include, e.g., HIV infection (and AIDS), hepatitis C infection, *Mycoplasma pneumonia* infection, Epstein-Barr virus (EBV) infection, cytomegalovirus (CMV) infection, rubella, or infectious mononucleosis. Neoplasms associated with CAD include, without limitation, non-Hodgkin's lymphoma. Hereinafter, such manifestations of CAD may be, where appropriate, referred to as, e.g., "infection-associated CAD" or "neoplasm-associated CAD." Thus, a human at risk for developing CAD can be, e.g., one who has an HIV infection, rubella, or a lymphoma. See also, e.g., Gertz (2006) Hematology 1:19-23; Horwitz et al. (1977) Blood 50:195-202; Finland and Barnes (1958) AMA Arch Intern Med 191:462-466; Wang et al. (2004) Acta Paediatr Taiwan 45:293-295; Michaux et al. (1998) Ann Hematol 76:201-204; and Chang et al. (2004) Cancer Genet Cytogenet 152:66-69.

Risk factors for MG are well known in the art of medicine and include, e.g., a predisposition to develop the condition, i.e., a family history of the condition or a genetic predisposition to develop the condition such as familial MG. For example, some HLA types are associated with an increased risk for developing MG. Risk factors for MG include the ingestion or exposure to certain MG-inducing drugs such as, but not limited to, D-penicillamine. See, e.g., Drosos et al. (1993) Clin Exp Rheumatol. 11(4):387-91 and Kaeser et al. (1984) Acta Neurol Scand Suppl. 100:39-47. As MG can be episodic, a subject who has previously experienced one or more symptoms of having MG can be at risk for relapse. Thus, a human at risk for developing MG can be, e.g., one who has a family history of MG and/or one who has ingested or been administered an MG-inducing drug such as D-penicillamine.

As used herein, a subject "at risk for developing CAPS" is a subject having one or more (e.g., two, three, four, five, six, seven, or eight or more) risk factors for developing the disorder. Approximately 60% of the incidences of CAPS are preceded by a precipitating event such as an infection. Thus, risk factors for CAPS include those conditions known to precipitate CAPS such as, but not limited to, certain cancers (e.g., gastric cancer, ovarian cancer, lymphoma, leukemia, endometrial cancer, adenocarcinoma, and lung cancer), pregnancy, puerperium, transplantation, primary APS, rheumatoid arthritis (RA), systemic lupus erythematosus (SLE), surgery (e.g., eye surgery), and certain infections. Infections include, e.g., parvovirus B19 infection and hepatitis C infection. Hereinafter, such manifestations of CAPS may be referred to as, e.g., "cancer-associated CAPS," "transplantation-associated CAPS," "RA-associated CAPS," "infection-associated CAPS," or "SLE-associated CAPS." See, e.g., Soltész et al. (2000) Haematologia (Budep) 30(4):303-311; Ideguchi et al. (2007) Lupus 16(1):59-64; Manner et al. (2008) Am J Med. Sci. 335(5):394-7; Miesbach et al. (2006) Autoimmune Rev. 6(2):94-7; Gómez-Puerta et al. (2006) Autoimmune Rev. 6(2):85-8; Gómez-Puerta et al. (2006) Semin. Arthritis Rheum. 35(5):322-32; Kasamon et al. (2005) Haematologia 90(3):50-53; Atherson et al. (1998) Medicine 77(3):195-207; and Canpolat et al. (2008) Clin Pediatr 47(6):593-7. Thus, a human at risk for developing CAPS can be, e.g., one who has primary CAPS and/or a cancer that is known to be associated with CAPS.

From the above it will be clear that subjects "at risk for developing a complement-associated disorder" (e.g., an AP-associated disorder or a CP-associated disorder) are not all the subjects within a species of interest.

A subject "suspected of having a complement-associated disorder" (e.g., an alternative complement pathway-associated disorder) is one having one or more (e.g., two, three, four, five, six, seven, eight, nine, or 10 or more) symptoms of the disease. Symptoms of these disorders will vary depending on the particular disorder, but are known to those of skill in the art of medicine. For example, symptoms of DDD include, e.g.: one or both of hematuria and proteinuria; acute nephritic syndrome; drusen development and/or visual impairment; acquired partial lipodystrophy and complications thereof; and the presence of serum C3 nephritic factor (C3NeF), an autoantibody directed against C3bBb, the C3 convertase of the alternative complement pathway. (See, e.g., Appel et al. (2005), *supra).* Symptoms of aHUS include, e.g., severe hypertension, proteinuria, uremia, lethargy/fatigue, irritability, thrombocytopenia, microangiopathic hemolytic anemia, and renal function impairment (e.g., acute renal failure). Symptoms of TTP include, e.g., microthrombi, thrombocytopenia, fever, low ADAMTS13 metalloproteinase expression or activity, fluctuating central nervous system abnormalities, renal failure, microangiopathic hemolytic anemia, bruising, purpura, nausea and vomiting (e.g., resulting from ischemia in the GI tract or from central nervous system involvement), chest pain due to cardiac ischemia, seizures, and muscle and joint pain. Symptoms of RA can include, e.g., stiffness, swelling, fatigue, anemia, weight loss, fever, and often, crippling pain. Some common symptoms of rheumatoid arthritis include joint stiffness upon awakening that lasts an hour or longer; swelling in a specific finger or wrist joints; swelling in the soft tissue around the joints; and swelling on both sides of the joint. Swelling can occur with or without pain, and can worsen progressively or remain the same for years before progressing. Symptoms of HELLP are known in the art of medicine and include, e.g., malaise, epigastric pain, nausea, vomiting, headache, right upper quadrant pain, hypertension, proteinuria, blurred vision, gastrointestinal bleeding, hypoglycemia, paresthesia, elevated liver enzymes/liver damage, anemia (hemolytic anemia), and low platelet count, any of which in combination with pregnancy or recent pregnancy. (See, e.g., Tomsen (1995) Am J Obstet Gynecol 172:1876-1890; Sibai (1986) Am J Obstet Gynecol 162:311-316; and Padden (1999), *supra.)* Symptoms of PNH include, e.g., hemolytic anemia (a decreased number of red blood cells), hemoglobinuria (the presence of hemoglobin in the urine particularly evident after sleeping), and hemoglobinemia (the presence of hemoglobin in the bloodstream). PNH-afflicted subjects are known to have paroxysms, which are defined here as incidences of dark-colored urine, dysphagia, fatigue, erectile dysfunction, thrombosis, and recurrent abdominal pain.

Symptoms of CAPS are well known in the art of medicine and include, e.g., histopathological evidence of multiple small vessel occlusions; the presence of antiphospholipid antibodies (usually at high titer), vascular thromboses, severe multi-organ dysfunction, malignant hypertension, acute respiratory distress syndrome, disseminated intravascular coagulation, microangiopathic hemolytic anemia, schistocytes, and thrombocytopenia. CAPS can be distinguished from APS in that patients with CAPS generally present with severe multiple organ dysfunction or failure, which is characterized by rapid, diffuse small vessel ischemia and thromboses predominantly affecting the parenchymal organs. In contrast, APS is associated with single venous or arterial medium-to-large blood vessel occlusions. Symptoms of MG include, e.g., fatigability and a range of muscle weakness-related conditions including: ptosis (of one or both eyes), diplopia, unstable gait, depressed or distorted facial expressions, and difficulty chewing, talking, or swallowing. In some instances, a subject can present with partial or complete paralysis of the respiratory muscles. Symptoms of CAD include, e.g., pain, fever, pallor, anemia, reduced blood flow to the extremities (e.g., with gangrene), and renal disease or acute renal failure. In some embodiments, the symptoms can occur following exposure to cold temperatures.

From the above it will be clear that subjects "suspected of having a complement-associated disorder" are not all the subjects within a species of interest.

The methods described herein can include identifying the subject as one having, suspected of having, or at risk for developing, a complement-associated disorder in a subject. Suitable methods for identifying the subject are known in the art. For example, suitable methods (e.g., sequencing techniques or use of microarrays) for determining whether a human subject has a DDD-associated mutation in a CFH, CFHR5, or C3 gene are described in, e.g., Licht et al. (2006) Kidney Int. 70:42-50; Zipfel et al. (2006), *supra;* Ault et al. (1997) J Biol. Chem. 272:25168-75; Abrera-Abeleda et al. (2006) JMed Genet 43:582-589; Poznansky et al. (1989) J Immunol. 143:1254-1258; Jansen et al. (1998) Kidney Int. 53:331-349; and Hegasy et al. (2002) Am J Pathol 161:2027-2034. Methods for detecting the presence of characteristic DDD-associated electron-dense deposits are also well known in the art. For example, a medical practitioner can obtain a tissue biopsy from the kidney of a patient and subject the tissue to electron microscopy. The medical practitioner may also examine the tissue by immunofluorescence to detect the presence of C3 using an anti-C3 antibody and/or light microscopy to determine if there is membranoproliferative glomerulonephritis. See, e.g., Walker et al. (2007) Mod. Pathol. 20:605-616 and Habib et al. (1975) Kidney Int. 7:204-215. In some embodiments, the identification of a subject as one having DDD can include assaying a blood sample for the presence of C3NeF. Methods for detecting the presence of C3NeF in blood are described in, e.g., Schwertz et al. (2001) Pediatr Allergy Immunol. 12:166-172.

In some embodiments, the medical practitioner can determine whether there is increased complement activation in a subject's serum. Indicia of increased complement activation include, e.g., a reduction in CH50, a decrease in C3, and an increase in C3dg/C3d. See, e.g., Appel et al. (2005), *supra.* In some embodiments, a medical practitioner can examine a subject's eye for evidence of the development of drusen and/or other visual pathologies such as AMD. For example, a medical practitioner can use tests of retinal function such as, but not limited to, dark adaptation, electroretinography, and electrooculography (see, e.g., Colville et al. (2003) Am J Kidney Dis. 42:E2-5).

Methods for identifying a subject as one having, suspected of having, or at risk for developing, TTP are also known in the art. For example, Miyata et al. describe a variety of assays for measuring ADAMTS13 activity in a biological sample obtained from a subject (Curr Opin Hematol (2007) 14(3):277-283). Suitable ADAMTS13 activity assays, as well as phenotypically normal ranges of ADAMTS 13 activity in a human subject, are described in, e.g., Tsai (2003) J. Am. Soc. Nephrol 14:1072-1081; Furlan et al. (1998) New Engl J Med. 339:1578-1584; Matsumoto et al. (2004) Blood 103:1305-1310; and Mori et al. (2002) Transfusion 42:572-580. Methods for detecting the presence of inhibitors of ADAMTS13 (e.g., autoantibodies that bind to ADAMTS13) in a biological sample obtained from a subject are known in the art. For example, a serum sample from a patient can be mixed with a serum sample from a subject without TTP to detect the presence of anti-ADAMTS 13 antibodies. In another example, immunoglobulin protein can be isolated from patient serum and used in *in vitro* ADAMTS13 activity assays to determine if an anti-ADAMTS13 antibody is present. See, e.g., Dong et al. (2008) Am J Hematol. 83(10):815-817. In some embodiments, risk of developing TTP can be determined by assessing whether a patient carries one or more mutations in the ADAMTS13 gene. Suitable methods (e.g., nucleic acid arrays or DNA sequencing) for detecting a mutation in the ADAMTS 13 gene are known in the art and described in, e.g., Levy et al., *supra;* Kokame et al., *supra;* Licht et al., *supra;* and Noris et al., *supra.*

In addition, methods for identifying a subject as one having, suspected of having, or at risk for developing aHUS are known in the art. For example, laboratory tests can be performed to determine whether a human subject has thrombocytopenia, microangiopathic hemolytic anemia, or acute renal insufficiency. Thrombocytopenia can be diagnosed by a medical professional as one or more of: (i) a platelet count that is less than 150,000/mm$^3$ (e.g., less than 60,000/mm$^3$); (ii) a reduction in platelet survival time, reflecting enhanced platelet disruption in the circulation; and (iii) giant platelets observed in a peripheral smear, which is consistent with secondary activation of thrombocytopoiesis. Microangiopathic hemolytic anemia can be diagnosed by a medical professional as one or more of: (i) hemoglobin concentrations that are less than 10 mg/dL (e.g., less than 6.5 mg/dL); (ii) increased serum lactate dehydrogenase (LDH) concentrations (>460 U/L); (iii) hyperbilirubinemia, reticulocytosis, circulating free hemoglobin, and low or undetectable haptoglobin concentrations; and (iv) the detection of fragmented red blood cells (schistocytes) with the typical aspect of burr or helmet cells in the peripheral smear together with a negative Coombs test. (See, e.g., Kaplan et al. (1992) "Hemolytic Uremic Syndrome and Thrombotic Thrombocytopenic Purpura," Informa Health Care (ISBN 0824786637) and Zipfel (2005) "Complement and Kidney Disease," Springer (ISBN 3764371668).)

A subject can also be identified as having aHUS by evaluating blood concentrations of C3 and C4 as a measure of complement activation or dysregulation. In addition, as is clear from the foregoing disclosure, a subject can be identified as having genetic aHUS by identifying the subject as harboring one or more mutations in a gene associated with aHUS such as CFI, CFB, CFH, or MCP (*supra*). Suitable methods for detecting a mutation in a gene include, e.g., DNA sequencing and nucleic acid array techniques. (See, e.g., Breslin et al. (2006) Clin Am Soc Nephrol 1:88-99 and Goicoechea de Jorge et al. (2007) Proc Natl Acad Sci USA 104:240-245.)

Methods for diagnosing a subject as one having, suspected of having, or at risk for developing, RA are also known in the art of medicine. For example, a medical practitioner can examine the small joints of the hands, wrists, feet, and knees to identify inflammation in a symmetrical distribution. The practitioner may also perform a number of tests to exclude other types of joint inflammation including arthritis due to infection or gout. In addition, rheumatoid arthritis is associated with abnormal antibodies in the blood circulation of afflicted patients. For example, an antibody referred to as "rheumatoid factor" is found in approximately 80% of patients. In another example, anti-citrulline antibody is present in many patients with rheumatoid arthritis and thus it is useful in the diagnosis of rheumatoid arthritis when evaluating patients with unexplained joint inflammation. See, e.g., van Venrooij et al. (2008) Ann NY Acad Sci 1143:268-285 and Habib et al. (2007) Immunol Invest 37(8):849-857. Another antibody called "the antinuclear antibody" (ANA) is also frequently found in patients with rheumatoid arthritis. See, e.g., Benucci et al. (2008) Clin Rheumatol 27(1):91-95; Julkunen et al. (2005) Scan J Rheumatol 34(2):122-124; and Miyawaki et al. (2005) J Rheumatol 32(8):1488-1494.

A medical practitioner can also examine red blood cell sedimentation rate to help in diagnosing RA in a subject. The sedimentation rate can be used as a crude measure of the inflammation of the joints and is usually faster during disease flares and slower during remissions. Another blood test that can be used to measure the degree of inflammation present in the body is the C-reactive protein.

Furthermore, joint x-rays can also be used to diagnose a subject as having rheumatoid arthritis. As RA progresses, the x-rays can show bony erosions typical of rheumatoid arthritis in the joints. Joint x-rays can also be helpful in monitoring the progression of disease and joint damage over time. Bone scanning, a radioactive test procedure, can demonstrate the inflamed joints.

Methods for identifying a subject as one having, suspected of having, or at risk for developing, HELLP are known in the art of medicine. Hallmark symptoms of HELLP syndrome include hemolysis, elevated liver enzymes, and low platelet count. Thus, a variety of tests can be performed on blood from a subject to determine the level of hemolysis, the concentration of any of a variety of liver enzymes, and the platelet level in the blood. For example, the presence of schistocytes and/or elevated free hemoglobin, bilirubin, or serum LDH levels is an indication of intravascular hemolysis. Routine laboratory testing can be used to determine the platelet count as well as the blood level of liver enzymes such as, but not limited to, aspartate aminotransferase (AST) and alanine transaminase (ALT). Suitable methods for identifying a subject as having HELLP syndrome are also described in, e.g., Sibai et al. (1993), *supra;* Martin et al. (1990), *supra;* Padden (1999), *supra;* and Gleicher and Buttino (1998) "Principles & Practice of Medical Therapy in Pregnancy," 3rd Edition, Appleton & Lange (ISBN 083857677X).

Methods for identifying a subject as having, suspected of having, or at risk for developing PNH are known in the art of medicine. The laboratory evaluation of hemolysis normally includes hematologic, serologic, and urine tests. Hematologic tests include an examination of the blood smear for morphologic abnormalities of red blood cells (RBC), and the measurement of the reticulocyte count in whole blood (to determine bone marrow compensation for RBC loss). Serologic tests include lactate dehydrogenase (LDH; widely performed), and free hemoglobin (not widely performed) as a direct measure of hemolysis. LDH levels, in the absence of tissue damage in other organs, can be useful in the diagnosis and monitoring of patients with hemolysis. Other serologic tests include bilirubin or haptoglobin, as measures of breakdown products or scavenging reserve, respectively. Urine tests include bilirubin, hemosiderin, and free hemoglobin, and are generally used to measure gross severity of hemolysis and for differentiation of intravascular vs. extravascular etiologies of hemolysis rather than routine monitoring of hemolysis. Further, RBC numbers, RBC hemoglobin, and hematocrit are generally performed to determine the extent of any accompanying anemia.

Suitable methods for identifying the subject as having MG can be qualitative or quantitative. For example, a medical practitioner can examine the status of a subject's motor functions using a physical examination. Other qualitative tests include, e.g., an ice-pack test, wherein an ice pack is applied to a subject's eye (in a case of ocular MG) to determine if one or more symptoms (e.g., ptosis) are improved by cold (see, e.g., Sethi et al. (1987) Neurology 37(8):1383-1385). Other tests include, e.g., the "sleep test," which is based on the tendency for MG symptoms to improve following rest. In some embodiments, quantitative or semi-quantitative tests can be employed by a medical practitioner to determine if a subject has, is suspected of having, or is at risk for developing, MG. For example, a medical practitioner can perform a test to detect the presence or amount of MG-associated autoantibodies in a serum sample obtained from a subject. MG-associated autoantibodies include, e.g., antibodies that bind to, and modulate the activity of, acetylcholine receptor (AChR), muscle-specific receptor tyrosine kinase (MuSK), and/or striational protein. (See, e.g., Conti-Fine et al. (2006), *supra).* Suitable assays useful for detecting the presence or amount of an MG-associated antibody in a biological sample are known in the art and described in, e.g., Hoch et al. (2001) Nat Med 7:365-368; Vincent et al. (2004) Semin Neurol. 24:125-133; McConville et al. (2004) Ann. Neurol. 55:580-584; Boneva et al. (2006) J Neuroimmunol. 177:119-131; and Romi et al. (2005) Arch Neurol. 62:442-446.

Additional methods for diagnosing MG include, e.g., electrodiagnostic tests (e.g., single-fiber electromyography) and the Tensilon (or edrophonium) test, which involves injecting a subject with the acetylcholinesterase inhibitor edrophonium and monitoring the subject for an improvement in one or more symptoms. See, e.g., Pascuzzi (2003) Semin Neurol 23(1):83-88; Katirji et al. (2002) Neurol Clin 20:557-586; and "Guidelines in Electrodiagnostic Medicine. American Association of Electrodiagnostic Medicine," Muscle Nerve 15:229-253.

A subject can be identified as having CAD using an assay to detect the presence or amount (titer) of agglutinating autoantibodies that bind to the I antigen on red blood cells. The antibodies can be monoclonal (e.g., monoclonal IgM or IgA) or polyclonal. Suitable methods for detecting these antibodies are described in, e.g., Christenson and Dacie (1957) Br J Haematol 3:153-164 and Christenson et al. (1957) Br J Haematol 3:262-275. A subject can also be diagnosed as having CAD using one or more of a complete blood cell count (CBC), urinalysis, biochemical studies, and a Coombs test to test for hemolysis in blood. For example, biochemical studies can be used to detect elevated lactase dehydrogenase levels, elevated unconjugated bilirubin levels, low haptoglobin levels, and/or the presence of free plasma hemoglobin, all of which can be indicative of acute hemolysis. Other tests that can be used to detect CAD include detecting complement levels in the serum. For example, due to consumption during the acute phase of hemolysis, measured plasma complement levels (e.g., C2, C3, and C4) are decreased in CAD.

Typical (or infectious) HUS, unlike aHUS, is often identifiable by a prodrome of diarrhea, often bloody in nature, which results from infection with a shiga-toxin producing microorganism. A subject can be identified as having typical HUS when shiga toxins and/or serum antibodies against shiga toxin or LPS are detected in the stool of an individual. Suitable methods for testing for anti-shiga toxin antibodies or LPS are known in the art. For example, methods for detecting antibodies that bind to shiga toxins Stx1 and Stx2 or LPS in humans are described in, e.g., Ludwig et al. (2001) J Clin. Microbiol. 39(6):2272-2279.

In some embodiments, a bispecific antibody described herein can be administered to a subject as a monotherapy. Alternatively, as described above, the antibody can be administered to a subject as a combination therapy with another treatment, e.g., another treatment for DDD, TTP, aHUS, PNH, RA, HELLP, MG, CAD, CAPS, tHUS, or any other complement-associated disorder known in the art or described herein. For example, the combination therapy can include administering to the subject (e.g., a human patient) one or more additional agents (e.g., anti-coagulants, anti-hypertensives, or corticosteroids) that provide a therapeutic benefit to the subject who has, or is at risk of developing, DDD. In some embodiments, the combination therapy can include administering to the subject (e.g., a human patient) a bispecific antibody and an immunosuppressive agent such as Remicade® for use in treating RA. In some embodiments, the bispecific antibody and the one or more additional active agents are administered at the same time. In other embodiments, a bispecific antibody is administered first in time and the one or more additional active agents are administered second in time. In some embodiments, the one or more additional active agents are administered first in time and the bispecific antibody is administered second in time.

A bispecific antibody described herein can replace or augment a previously or currently administered therapy. For example, upon treating with an antibody that binds to C5a and C5b, administration of the one or more additional active agents can cease or diminish, e.g., be administered at lower levels. In some embodiments, administration of the previous therapy can be maintained. In some embodiments, a previous therapy will be maintained until the level of the bispecific antibody reaches a level sufficient to provide a therapeutic effect. The two therapies can be administered in combination.

Monitoring a subject (e.g., a human patient) for an improvement in a complement-associated disorder, as defined herein, means evaluating the subject for a change in a disease parameter, e.g., an improvement in one or more symptoms of the disease. Such symptoms include any of the symptoms of complement-associated disorders known in the art and/or described herein. In some embodiments, the evaluation is performed at least 1 hour, e.g., at least 2, 4, 6, 8, 12, 24, or 48 hours, or at least 1 day, 2 days, 4 days, 10 days, 13 days, 20 days or more, or at least 1 week, 2 weeks, 4 weeks, 10 weeks, 13 weeks, 20 weeks or more, after an administration. The subject can be evaluated in one or more of the following periods: prior to beginning of treatment; during the treatment; or after one or more elements of the treatment have been administered. Evaluating can include evaluating the need for further treatment, e.g., evaluating whether a dosage, frequency of administration, or duration of treatment should be altered. It can also include evaluating the need to add or drop a selected therapeutic modality, e.g., adding or dropping any of the treatments for any of the complement-associated disorders described herein.

*Ex vivo* **approaches.** An *ex vivo* strategy for treating or preventing a complement-associated disorder (e.g., an AP-associated disorder or a CP-associated disorder) can involve transfecting or transducing one or more cells obtained from a subject with a polynucleotide encoding a bispecific antibody described herein. For example, the cells can be transfected with a single vector encoding a heavy and light chain of an antibody that binds to C5a and C5b, or the cells can be transfected with a first vector encoding a heavy chain and a second vector encoding a light chain of the antibody.

The transfected or transduced cells are then returned to the subject. The cells can be any of a wide range of types including, without limitation, hemopoietic cells (e.g., bone marrow cells, macrophages, monocytes, dendritic cells, T cells, or B cells), fibroblasts, epithelial cells, endothelial cells, keratinocytes, or muscle cells. Such cells can act as a source (e.g., sustained or periodic source) of the bispecific antibody for as long as they survive in the subject. In some embodiments, the vectors and/or cells can be configured for inducible or repressible expression of the bispecific antibody (see, e.g., Schockett et al. (1996) Proc Natl Acad Sci USA 93: 5173-5176 and U.S. Patent No. 7,056,897).

Preferably, the cells are obtained from the subject (autologous), but can potentially be obtained from a subject of the same species other than the subject (allogeneic).

Suitable methods for obtaining cells from a subject and transducing or transfecting the cells are known in the art of molecular biology. For example, the transduction step can be accomplished by any standard means used for *ex vivo* gene therapy, including calcium phosphate, lipofection, electroporation, viral infection (see above), and biolistic gene transfer. (See, e.g., Sambrook et al. (*supra*) and Ausubel et al. (1992) "Current Protocols in Molecular Biology," Greene Publishing Associates.) Alternatively, liposomes or polymeric microparticles can be used. Cells that have been successfully transduced can be selected, for example, for expression of the coding sequence or of a drug resistance gene.

Kits

The disclosure also features articles of manufacture or kits, which include a container with a label; and a composition containing one or more bispecific antibodies. For example, the kit can contain one or more of any of the bispecific antibodies described herein. The label indicates that the composition is to be administered to a subject (e.g., a human) having, suspected of having, or at risk for developing, a complement-associated disorder (e.g., an AP- or CP-associated disorder) such as, but not limited to, DDD, aHUS, TTP, HELLP, RA, PNH, AMD, tHUS, MG, CAD, CAPS, or any other complement pathway-associated disorder known in the art and/or described herein. The kit can, optionally, include a means for administering the composition to the subject. For example, the kits can include one or more syringes.

The kits may further include one or more additional active agents such as any of those described herein. For example, the kits can include one or more corticosteroids, anti-hypertensives, immunosuppressives, and anti-seizure agents.

The following examples are intended to illustrate, not limit, the invention.

Example 1. Treatment of thrombotic thrombocytopenic purpura using a bispecific antibody

A human patient is identified by a medical practitioner as having an inherited form of TTP. Once a week for four weeks the patient is administered a composition containing a bispecific antibody that binds to C5a and C5b by intravenous infusion. The patient and medical practitioner observe a substantial improvement in at least two known symptoms of TTP during the initial treatment. One week after the initial four week treatment, the patient receives intravenously administered "maintenance doses" of the antibody every two weeks until the medical practitioner determines that the TTP is in remission.

Example 2. Treatment of dense deposit disease using a bispecific antibody

A human patient presenting with DDD is intravenously administered every two weeks a composition containing a bispecific antibody that binds to C5aR1 and C5b. The patient and medical practitioner observe a substantial reduction in overall severity of the patient's DDD symptoms during the initial treatment. The patient is maintained on the same treatment regimen until the medical practitioner determines that the DDD is in remission.

Example 3. Effect of human C5 on the clearance of a humanized anti-C5 antibody in mice

The following experiments were performed to determine the effect of human C5 on the clearance of a humanized anti-C5 antibody in a humanized neonatal Fc receptor (hFcRn) mouse model which is lacking endogenous FcRn and is transgenic for hFcRn (mFcRn$^{-/-}$ hFcRn $^{+/+}$; Jackson Laboratories, Bar Harbor, Maine). The humanized FcRn model has been described in, e.g., Petkova et al. (2006) Int Immunology 18(12):1759-1769 and Oiao et al. (2008) Proc Natl Acad Sci USA 105(27):9337-9342. 100 $\mu$g of a humanized anti-human C5 antibody in 200 $\mu$L of phosphate buffered saline (PBS) was administered by intravenous (i.v.) injection to each of eight (8) hFcRn transgenic mice. Serum was collected from each of the mice at days one, three, seven, 14, 21, 28, and 35 following the administration. The concentration of the humanized antibody in the serum was measured by ELISA. Briefly, assay plates were coated with an anti-human $\kappa$ light chain capture antibody followed by washing to remove unbound capture antibody. The wells of the plate were then contacted with the serum samples under conditions that allow the humanized anti-human C5 antibody, if present in the serum, to bind to the capture antibody. The relative amount of humanized antibody bound to each well was detected using a detectably-labeled anti-human IgG antibody.

The half-life of the antibody in the mice was calculated using the ELISA measurements and the following equation (where T is the time evaluated, $A_0$ is the initial concentration of the antibody, and $A_t$ is the concentration of the antibody in serum determined at time T).

$$\text{Half-life } (T_{\frac{1}{2}}) = T \text{ x } [(\ln 2)/\ln(A_0/A_t)] \qquad \text{(Equation 1)}$$

The results of the experiment are depicted in Fig. 1. The half-life of the humanized anti-C5 antibody in the hFcRn mouse model was 12.56 $\pm$ 1.73 days.

To determine the effect of human C5 on the half-life of the humanized antibody using the hFcRn model, mice were administered in 250 $\mu$L PBS one of: (i) 50 $\mu$g of the humanized antibody complexed in a 1:4 molar ratio of antibody to human C5 (6 mice); (ii) 50 $\mu$g of the humanized antibody complexed in a 1:4 molar ratio of antibody to human C5 and an additional 200 $\mu$g of human C5 (in 200 $\mu$L of PBS) by i.v. injection (6 mice); (iii) 50 $\mu$g of the humanized antibody complexed in a 1:2 molar ratio of antibody to human C5 (6 mice); or (iv) 50 $\mu$g of the humanized antibody alone (6 mice). Serum was collected from the mice, as described above, at days one, three, seven, 14, 21, 28, and 35 following the administration and the half-life of the humanized antibody under each condition was determined as described above.

The half-life of the humanized anti-human C5 antibody, in the absence of human C5, was determined in this experiment to be 13.49 $\pm$ 0.93 days. In contrast, the half-life of the humanized antibody administered to the mice in a 1:2 ratio with human C5 was measured to be 9.58 $\pm$ 1.24 days. The half-life of the humanized antibody administered to the mice in a 1:4 ratio with human C5 was determined to be 5.77 $\pm$ 1.86 days. The additional administration of human C5 along with the 1:4 antibody-C5 complex to mice resulted in a half-life for the antibody of 4.55 $\pm$ 1.02 days.

These results indicate that the clearance of the humanized anti-C5 antibody in this mouse model is greatly influenced by the concentration of its antigen. In other words, the half-life of the antibody in this model is dependent on the amount of uncomplexed antibody. Human C5 is constitutively expressed and present in serum at a concentration of approximately 0.37 $\mu$M. Unlike C5, fragments C5a and C5b are present in blood at much lower concentrations and are often restricted to specific areas of local complement activation. The data presented herein indicate that a lower concentration of fragments C5a and C5b, as compared to C5, will favor a longer half-life for a bispecific antibody (e.g., an anti-C5a/C5b antibody) over an anti-C5 antibody in blood due to a reduced contribution of target-mediated antibody clearance. The data also indicate that a lower dose and/or lower frequency administration of an anti-C5a/C5b bispecific antibody, as compared to an anti-C5 antibody, can provide the same or greater inhibition of C5 in a human with a complement-associated disorder such as PNH or aHUS.

Example 4. Mathematical modeling of the clearance of a humanized anti-C5 antibody

Example 3 demonstrated that the presence of an excess of C5 over antibody leads to an approximate three-fold reduction in the half life of an anti-C5 monoclonal antibody. A simple mathematical modeling featuring target (C5) mediated clearance was developed and used to explore the potential mechanism behind this effect. The model that invokes the basic pathways of clearance of the humanized antibody in a human patient is shown in Fig. 2. Free antibody (A) and its antigen C5 (C) are in equilibrium with their cognate, complexed form. The rate constant for association of the antibody and C5 is represented by $k3$ and the rate constant for the dissociation of the complex is represented by $k4$. The antibody:C5 complex (CA) can be eliminated by immune complex clearance with a rate constant represented by $k6$. Free antibody is also eliminated as represented by a different rate constant $k5$. C5 is constitutively expressed with a rate constant of $k1$ and it is eliminated with a rate constant of $k2$.

The model was also based on a series of assumptions. First, the rate of C5 synthesis (rate constant $k1$) is constant and therefore zero order and in the presence of excess antibody the clearance of free C5 via rate constant $k2$ is negligible because the concentration of free C5 is negligible. In addition, the clearance of free antibody is assumed to be controlled by first order beta-elimination. Also assumed was that because the antibody:C5 complex does not accumulate, the rate of synthesis of C5 is limiting for the rate of complex elimination (via rate constant $k6$). Lastly, the model assumes that no free antibody is recycled from the immune complex. In other words, the complex is either dissociated or eliminated - it is assumed that the immune complex-mediated clearance (rate constant $k6$) is irreversible.

Based on these assumptions, a simplified pathway of antibody clearance was constructed (Fig. 3). Like the pathway depicted in Fig. 2, the simplified pathway consists of two modes of antibody clearance: (i) free antibody clearance with rate constant $k8$ and (ii) immune complex clearance with rate constant $k7$. The first order equation that governs the free antibody clearance is as follows (where A is the concentration of antibody at the time measured, $A_0$ is the initial concentration of antibody, and t is the time at which A is measured).

$$A = A_0 \times e^{(-k8t)} \qquad \text{(Equation 2)}$$

The zero order equation governing the immune complex clearance is represented by the following equation.

$$A = A_0 - k7t \qquad \text{(Equation 3)}$$

The integrated rate equation for the concurrent processes was determined to be

$$A = A_0 \times e^{(-k8t)} - k7t. \qquad \text{(Equation 4)}$$

It was assumed that $A_0$, the initial antibody concentration, is 400 $\mu$g/mL or 2.67 $\mu$M for a 150 kDa antibody. The physiological concentration of human C5 is 0.37 $\mu$M for a 190 kDa protein. One antibody has two antigen-combining sites and therefore binds to two C5 molecules. Breakthrough is obtained when the concentration of the free antibody is zero. See, e.g., Brodsky et al. (2008) Blood 111(4):1840-1847 and Hillmen et al. (2004) N Engl J Med 350(6):552-559.

As C5 is constitutively expressed and maintained at a constant concentration in the blood, the rate of synthesis of C5 protein is equal to its rate of clearance. The half-life of human C5 in blood is approximately 63 hours (see, e.g., Sissons et al. (1977) J Clin Invest 59:704-715). Thus, the rate constant, $k7$, for C5 clearance or production is approximately 0.185 $\mu$M/ (63 $\div$ 24) day$^{-1}$ or 0.07 $\mu$M day$^{-1}$.

To determine the rate constant $k8$ for the first order antibody clearance (Equation 2), the relationship between the rate constant and the half-life can be represented by the following equation:

$$T_{\frac{1}{2}} = \ln2/k8. \qquad \text{(Equation 5)}$$

It was assumed that the half life of the anti-C5 monoclonal antibody in the absence of C5 is the same as the value (12.56 days) obtained in FcRn mice (see Example 3). Thus, solving Equation 5 for $k8$, $k8$ is equal to $\ln2/T_{1/2}$ or $(0.693)/(12.56$ days) or 0.055 day$^{-1}$. The calculated influence of immune complex clearance on the half-life of a humanized anti-C5 antibody is set forth in Table 1.

Table 1.

| Antibody | Time (days) | $A_0 \times e^{(-k8t)}$ | -*k7t* | A |
|---|---|---|---|---|
| Anti-C5 Ab | 0 | 2.67 | 0 | 2.67 |
| | 5 | 2.03 | -0.35 | 1.68 |
| | 10 | 1.54 | -0.7 | 0.84 |
| | 15 | 1.17 | -1.05 | 0.12 |
| | 16 | 1.11 | 1.12 | 0 |

From Table 1, breakthrough is achieved by 16 days. In other words, the levels of an antibody with a half-life of 12.56 days is reduced to zero by 16 days through the effects of the C5 mediated clearance component, whereas in theory approximately 40% of the starting concentration of this antibody would have remained without the effects of target (C5) mediated clearance component. In fact, the rate of clearance predicted by the above-described model closely overlaps with the rate observed *in vivo.* Based on the model and the calculations described above, the contribution of target-mediated clearance (immune complex clearance) on the half-life of the humanized antibody in man is substantial. This model, as well as the *in vivo* data, strongly indicates that a lower concentration of fragments C5a and C5b, as compared to C5, will favor a longer half-life for a bispecific antibody (e.g., an anti-C5a/C5b antibody) over an anti-C5 antibody in blood due to a reduced contribution of target-mediated antibody clearance. Accordingly, a lower dose and/or lower frequency administration of an anti-C5a/C5b bispecific antibody, as compared to an anti-C5 antibody, is likely to provide the same or greater inhibition of C5 in a human with a complement-associated disorder.

## Claims

1. A bispecific antibody comprising: (a) a first antigen combining site that binds to C5a and (b) a second antigen combining site that binds to C5b, wherein the bispecific antibody bound to C5a inhibits the interaction between C5a and the C5a receptor; and wherein the bispecific antibody bound to C5b inhibits the assembly or lytic activity of the TCC; and wherein the bispecific antibody does not bind to full-length or mature C5.

2. The bispecific antibody according to claim 1, wherein the bispecific antibody inhibits the interaction between C5b and C6.

3. The bispecific antibody according to claim 1, wherein the bispecific antibody inhibits (a) C5a-dependent chemotaxis or (b) complement-dependent lysis in vitro.

4. The bispecific antibody according to any one of claims 1-3, wherein the bispecific antibody is a humanized, deimmunized, chimeric, or fully human antibody.

5. The bispecific antibody according to any one of claims 1-3, wherein the bispecific antibody is selected from the group consisting of a diabody, an intrabody, a single chain antibody, a dual variable domain immunoglobulin, and an F(ab')$_2$ fragment.

6. The bispecific antibody according to any one of claims 1-3, wherein the antibody is a single chain diabody, a tandem single chain Fv fragment, a tandem single chain diabody, or a fusion protein comprising a single chain diabody and at least a portion of an immunoglobulin heavy chain constant region.

7. A pharmaceutical composition comprising the bispecific antibody according to any one of claims 1-6 and a pharmaceutically-acceptable carrier.

8. The bispecific antibody according to any one of claims 1-7, for use in treating a patient afflicted with a complement-associated disorder.

9. The bispecific antibody according to claim 8 for use according to claim 8, wherein the complement-associated disorder is selected from the group consisting of rheumatoid arthritis, asthma, ischemia-reperfusion injury, atypical hemolytic uremic syndrome, thrombotic thrombocytopenic purpura, paroxysmal nocturnal hemoglobinuria, dense deposit disease, age-related macular degeneration, spontaneous fetal loss, Pauci-immune vasculitis, epidermolysis bullosa, recurrent fetal loss, multiple sclerosis, traumatic brain injury, myasthenia gravis, cold agglutinin disease, dermatomyositis, Graves' disease, Hashimoto's thyroiditis, type I diabetes, psoriasis, pemphigus, autoimmune hemolytic anemia, idiopathic thrombocytopenic purpura, Goodpasture syndrome, antiphospholipid syndrome, and catastrophic antiphospholipid syndrome.

## Patentansprüche

1. Bispezifischer Antikörper, umfassend: (a) eine erste Antigenkombinierungsstelle, die an C5a bindet, und (b) eine zweite Antigenkombinierungsstelle, die an C5b bindet, wobei der an C5a gebundene bispezifische Antikörper die Interaktion zwischen C5a und dem C5a-Rezeptor hemmt, und wobei der an C5b gebundene bispezifische Antikörper die Assemblierung oder die lytische Aktivität des TCC hemmt; und wobei der bispezifische Antikörper nicht an Volllängen- oder reife C5 bindet.

2. Bispezifischer Antikörper nach Anspruch 1, wobei der bispezifische Antikörper die Wechselwirkung zwischen C5b und C6 hemmt.

3. Bispezifischer Antikörper nach Anspruch 1, wobei der bispezifische Antikörper (a) C5a-abhängige Chemotaxis oder (b) komplementabhängige Lyse in vitro hemmt.

4. Bispezifischer Antikörper nach einem der Ansprüche 1-3, wobei es sich bei dem bispezifischen Antikörper um einen humanisierten, deimmunisierten, chimären oder voll-humanen Antikörper handelt.

5. Bispezifischer Antikörper nach einem der Ansprüche 1-3, wobei der bispezifische Antikörper aus der Gruppe bestehend aus einem Diabody, einem Intrabody, einem Einzelkettenantikörper, einem Dual-Variable-Domain-Immunglobulin und einem $F(ab')_2$-Fragment ausgewählt ist.

6. Bispezifischer Antikörper nach einem der Ansprüche 1-3, wobei es sich bei dem Antikörper um einen Einzelketten-Diabody, ein Tandem-Einzelketten-Fv-Fragment, einen Tandem-Einzelketten-Diabody oder ein Fusionsprotein umfassend einen Einzelketten-Diabody und zumindest einen Teil einer Immunglobulin-Schwerketten-Konstantregion handelt.

7. Pharmazeutische Zusammensetzung, umfassend den bispezifischen Antikörper nach einem der Ansprüche 1-6 und einen pharmazeutisch annehmbaren Träger.

8. Bispezifischer Antikörper nach einem der Ansprüche 1-7 zur Verwendung in der Behandlung eines Patienten, der an einer komplementassoziierten Erkrankung leidet.

9. Bispezifischer Antikörper nach Anspruch 8 zur Verwendung nach Anspruch 8, wobei die komplementassoziierte Erkrankung aus der Gruppe bestehend aus rheumatoider Arthritis, Asthma, Ischämie-Reperfusionsverletzung, atypischem hämolytischem Urämiesyndrom, thrombotisch-thrombocytopenischer Purpura, paroxysmaler nächtlicher Hämoglobinurie, Dense Deposit Disease, altersbedingter Makuladegeneration, spontanem Abortus, pauci-immuner Vaskulitis, Epidermolysis bullosa, rekurrierendem Abortus, multipler Sklerose, traumatischer Hirnverletzung, Myasthenia gravis, Kälteagglutinin-Krankheit, Dermatomyositis, Morbus Basedow, Hashimoto-Thyroiditis, Typ-I-Diabetes, Psoriasis, Pemphigus, autoimmunhämolytischer Anämie, idiopathischer thrombocytopenischer Purpura, Goodpasture-Syndrom, Antiphospholipidsyndrom und katastrophischem Antiphospolidsyndrom ausgewählt ist.

## Revendications

1. Anticorps bispécifique comprenant : (a) un premier site de combinaison d'antigène qui se lie à C5a et (b) un deuxième site de combinaison d'antigène qui se lie à C5b, l'anticorps bispécifique lié à C5a inhibant l'interaction entre C5a et le récepteur de C5a ; et l'anticorps bispécifique lié à C5b inhibant l'assemblage ou l'activité lytique du TCC ; et l'anticorps bispécifique ne se liant pas à C5 de pleine longueur ou mature.

**2.** Anticorps bispécifique selon la revendication 1, l'anticorps bispécifique inhibant l'interaction entre C5b et C6.

**3.** Anticorps bispécifique selon la revendication 1, l'anticorps bispécifique inhibant (a) un chimiotactisme dépendant de C5a ou (b) une lyse dépendante du complément *in vitro.*

**4.** Anticorps bispécifique selon l'une quelconque des revendications 1 à 3, l'anticorps bispécifique étant un anticorps humanisé, désimmunisé, chimérique ou totalement humain.

**5.** Anticorps bispécifique selon l'une quelconque des revendications 1 à 3, l'anticorps bispécifique étant choisi dans le groupe constitué d'un diabody, un intrabody, un anticorps monocaténaire, une immunoglobuline à double domaine variable, et un fragment $F(ab')_2$.

**6.** Anticorps bispécifique selon l'une quelconque des revendications 1 à 3, l'anticorps étant un diabody monocaténaire, un fragment Fv monocaténaire en tandem, un diabody monocaténaire en tandem ou une protéine de fusion comprenant un diabody monocaténaire et au moins une partie d'une région constante de chaîne lourde d'immunoglobuline.

**7.** Composition pharmaceutique comprenant l'anticorps bispécifique selon l'une quelconque des revendications 1 à 6 et un véhicule pharmaceutiquement acceptable.

**8.** Anticorps bispécifique selon l'une quelconque des revendications 1 à 7, pour utilisation dans le traitement d'un patient atteint d'un trouble associé au complément.

**9.** Anticorps bispécifique selon la revendication 8 pour utilisation selon la revendication 8, le trouble associé au complément étant choisi dans le groupe constitué de la polyarthrite rhumatoïde, l'asthme, une lésion d'ischémie-reperfusion, le syndrome hémolytique et urémique atypique, le purpura thrombotique thrombocytopénique, l'hémoglobinurie nocturne paroxystique, la glomérulonéphrite membranoproliférative, la dégénérescence maculaire liée à l'âge, une perte foetale spontanée, la vascularite pauci-immune, l'épidermolyse bulleuse, une perte foetale récurrente, la sclérose en plaques, une lésion cérébrale traumatique, la myasthénie grave, la maladie des agglutinines froides, la dermatomyosite, la maladie de Graves, la thyroïdite d'Hashimoto, le diabète de type I, le psoriasis, le pemphigus, l'anémie hémolytique auto-immune, le purpura thrombocytopénique idiopathique, le syndrome de Goodpasture, le syndrome des antiphospholipides, et le syndrome des antiphospholipides catastrophique.

Fig. 1

Fig. 2

C

mAb

$1^{st}$ order
$A = A_0 . e^{-k8t}$

$k8$

$k7$

Zero order:
$A = A_0 - k7t$

Free mAb
clearance

Immune complex
clearance

Integrated rate equation for the concurrent processes:

$$A = A_0 . e^{-k8t} - k7t$$

**Fig. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61219644 B
- US 61228001 B
- WO 0115731 A
- US 6866845 B
- US 4686100 A
- US 20060160726 A
- US 6355245 B
- US 20050244406 A
- US 7455837 B
- US 20070065433 A
- US 20070274989 A
- US 20070123466 A
- US 7371849 B
- WO 9627011 A
- US 4676980 A
- US 5534254 A
- US 7112324 B
- US 5525491 A
- US 5258498 A
- WO 08024188 A
- WO 07024715 A
- US 20070004909 A
- US 5530101 A
- US 6001329 A
- US 6933368 B

- US 20080202513 A
- US 7112341 B
- US 6019968 A
- WO 00061178 A
- WO 06122257 A
- US 20070235029 A
- WO 0069887 A
- US 5997848 A
- WO 04060407 A
- US 4868116 A
- US 4980286 A
- WO 8907136 A
- WO 8902468 A
- WO 8905345 A
- WO 9207573 A
- US 20080241223 A
- US 5501856 A
- US 4863457 A
- US 3710795 A
- EP 488401 A
- EP 430539 A
- US 20070172483 A
- US 7056897 B
- WO 61219644 A
- WO 61228001 A

### Non-patent literature cited in the description

- **MULLER-EBERHARD.** *Ann Rev Biochem,* 1988, vol. 57, 321-347
- **MEDICUS et al.** *J Exp Med,* 1976, vol. 144, 1076-1093
- **FEARON et al.** *J Exp Med,* 1975, vol. 142, 856-863
- **ISENMAN et al.** *J Immunol,* 1980, vol. 124, 326-331
- **SCHREIBER et al.** *Proc Natl Acad Sci USA,* 1978, vol. 75, 3948-3952
- **SISSONS et al.** *Proc Natl Acad Sci USA,* 1980, vol. 77, 559-562
- **COOPER et al.** *J Exp Med,* 1970, vol. 132, 775-793
- **HOLERS et al.** *Immunological Reviews,* 2008, vol. 223, 300-316
- **ROTHER et al.** *Nature Biotechnology,* 2007, vol. 25 (11), 1256-1264
- **WANG et al.** *Proc. Natl. Acad Sci. USA,* 1996, vol. 93, 8563-8568

- **WANG et al.** *Proc. Natl. Acad. Sci. USA,* 1995, vol. 92, 8955-8959
- **RINDER et al.** *J. Clin. Invest.,* 1995, vol. 96, 1564-1572
- **KROSHUS et al.** *Transplantation,* 1995, vol. 60, 1194-1202
- **HOMEISTER et al.** *J. Immunol.,* 1993, vol. 150, 1055-1064
- **WEISMAN et al.** *Science,* 1990, vol. 249, 146-151
- **AMSTERDAM et al.** *Am. J. Physiol,* 1995, vol. 268, H448-H457
- **RABINOVICI et al.** *J Immunol,* 1992, vol. 149, 1744-1750
- **HILIMEN et al.** *N Engl J Med.,* 21 September 2006, vol. 355 (12), 1233-43
- **RAWAL ; PANGBURN.** *J Immunol,* 2001, vol. 166 (4), 2635-2642
- **HILLMEN et al.** *N Engl J Med,* 2004, vol. 350 (6), 552

- **HAVILAND et al.** *J Immunol,* 1991, vol. 146 (1), 362-8
- **AMES et al.** *J Immunol,* 1994, vol. 152 (9), 4572-4581
- **INOUE.** *Complement Inflamm,* 1989, vol. 6 (3), 219-222
- **KABAT et al.** Sequences of Proteins of Immunological Interest. U.S. Department of Health and Human Services, 1991
- **CHOTHIA et al.** *Nature,* 1989, vol. 342, 877-883
- **WARD ; ZVAIFLER.** *J Clin Invest.,* 1971, vol. 50 (3), 606-16
- **WURZNER et al.** *Compliment Inflamm.,* 1991, vol. 8, 328-340
- **MARY ; BOULAY.** *Eur J Haematol,* 1993, vol. 51 (5), 282-287
- **KANEKO et al.** *Immunology,* 1995, vol. 86 (1), 149-154
- **GIANNINI et al.** *J Biol Chem,* 1995, vol. 270 (32), 19166-19172
- **WARD.** *J Mol Med,* 2009, vol. 87 (4), 375-378
- **CHEN et al.** *Nature,* 2007, vol. 446 (7132), 203-207
- **MÜLLER-EBERHARD.** *Biochem Soc Symp,* 1985, vol. 50, 235-246
- **YAMAMOTO ; GEWURZ.** *J Immunol,* 1978, vol. 120 (6), 2008-2015
- Experimental Immunochemistry. Springfield, 1961, vol. 135-240, 135-139
- **GERARD ; GERARD.** *Nature,* 1991, vol. 349 (6310), 614-617
- **BAO et al.** *Genomics,* 1992, vol. 13, 437-440
- **SORURI et al.** *Immunol Lett,* 2003, vol. 88 (1), 47-52
- **ZWIRNER et al.** *Mol Immunol,* 1999, vol. 36 (13-14), 877-884
- **KIAFARD et al.** *Immunobiology,* 2007, vol. 212 (2), 129-139
- **RITTIRSCH et al.** *Nature Med,* 2008, vol. 14, 551-557
- **GIRARDI et al.** *J Clin Invest,* 2003, vol. 112, 1644-1654
- **ATKINSON.** *J Clin Invest,* 2003, vol. 112, 1639-1641
- **MONK et al.** *Br J Pharm,* 2007, vol. 152, 429-448
- **HUBER-LANG et al.** *J Immunol,* 2005, vol. 174, 1104-1110
- **SCOLA et al.** *J Biol Chem,* 2007, vol. 282 (6), 3664-3671
- **CAIN ; MONK.** *J Biol Chem,* 2002, vol. 277 (9), 7165-7169
- **DISCIPIO.** *J Biol Chem,* 1992, vol. 267 (24), 17087-17094
- **PODACK et al.** *J Exp Med,* 1980, vol. 151, 301-303
- **PODACK et al.** *J Immunol,* 1978, vol. 120, 1841-1848
- **DISCIPIO et al.** *J Biol Chem,* 1988, vol. 263, 549-560
- **MOLLNES et al.** *Complement Inflamm,* 1989, vol. 6 (3), 223-235
- **HARLOW ; LANE.** Antibodies: A Laboratory Manual. Cold Spring Harbor Laboratory Press, 1988
- **BENNY K. C. ; LO.** Antibody Engineering: Methods and Protocols. Humana Press, 2004
- **BORREBAEK.** Antibody Engineering, A Practical Guide. W.H. Freeman and Co, 1992
- **BORREBAEK.** Antibody Engineering. Oxford University Press, 1995
- **JOHNE et al.** *J. Immunol. Meth,* 1993, vol. 160, 191-198
- **JONSSON et al.** *Ann. Biol. Clin.,* 1993, vol. 51, 19-26
- **JONSSON et al.** *Biotechniques,* 1991, vol. 11, 620-627
- **TODOROVSKA et al.** *J Immunol Methods,* 2001, vol. 248 (1), 47-66
- **HUDSON ; KORTT.** *J Immunol Methods,* 1999, vol. 231 (1), 177-189
- **POLJAK.** *Structure,* 1994, vol. 2 (12), 1121-1123
- **RONDON ; MARASCO.** *Annual Review of Microbiology,* 1997, vol. 51, 257-28 3
- **KONTERMANN.** *Acta Pharmacologica Sinica,* 2005, vol. 26 (1), 1-9
- **KUFER et al.** *Trends Biotechnol,* 2004, vol. 22, 238-244
- **KRIANGKUM et al.** *Biomol Eng,* 2001, vol. 18, 31-40
- **JEFFREY et al.** *Proc Natl Acad Sci USA,* 1993, vol. 90, 10310-10314
- **MUYLDERMANS.** *J Biotechnol,* 2001, vol. 74, 277-302
- **DESMYTER et al.** *J Biol Chem,* 2001, vol. 276, 26285-90
- **DUMOULIN et al.** *Nature,* 2003, vol. 424, 783-788
- **CHAN et al.** *Biochemistry,* 2008, vol. 47 (42), 11041-54
- **MILSTEIN ; CUELLO.** *Nature,* 1983, vol. 305, 537-539
- **SURESH et al.** *Methods in Enzymology,* 1986, vol. 121, 210
- **BRENNAN et al.** *Science,* 1985, vol. 229:, 81
- **SHALABY et al.** *J. Exp. ed.,* 1992, vol. 175, 217-225
- **KOSTELNY et al.** *J Immunol,* 1992, vol. 148 (5), 1547-1553
- **HOLLINGER et al.** *Proc Natl Acad Sci USA,* 1993, vol. 90, 6444-6448
- **GRUBER et al.** *J Immunol,* 1994, vol. 152, 5368
- **TUTT et al.** *J Immunol,* 1991, vol. 147, 60

- **SEGAL ; BAST.** *Curr Protocols Immunol,* 1995, 2.13.1-2.13.16
- **KOSTELNY et al.** *J Immunol,* 1992, vol. 148 (5), 1547-1553
- **DE KRUIF ; LOGTENBERG.** *J Biol Chem,* 1996, vol. 271 (13), 7630-7634
- **REN-HEIDENREICH et al.** *Cancer,* 2004, vol. 100, 1095-1103
- **KORN et al.** *Gene Med,* 2004, vol. 6, 642-651
- **GROSSE-HOVEST et al.** *Proc Natl Acad Sci USA,* 2004, vol. 101, 6858-6863
- **MALETZ et al.** *Int J Cancer,* 2001, vol. 93, 409-416
- **HAYDEN et al.** *Ther Immunol,* 1994, vol. 1, 3-15
- **HONEMANN et al.** *Leukemia,* 2004, vol. 18, 636-644
- **ZAPATA et al.** *Protein Eng.,* 1995, vol. 8 (10), 1057-1062
- **ZHU et al.** *Biotechnology,* 1996, vol. 14, 192-196
- **HELFRICH et al.** *Int J Cancer,* 1998, vol. 76, 232-239
- **BRÜSSELBACH et al.** *Tumor Targeting,* 1999, vol. 4, 115-123
- **KIPRIYANOV et al.** *J Mol Biol,* 1999, vol. 293, 41-56
- **NETTLEBECK et al.** *Mol Ther,* 2001, vol. 3, 882-891
- **WU et al.** *Nat Biotechnol,* 2007, vol. 25 (11), 1290-1297
- **JONES et al.** *Nature,* 1996, vol. 321, 522-525
- **RIECHMAN et al.** *Nature,* 1988, vol. 332, 323-327
- **ABBAS ; LICHTMAN ; POBER.** Cellular and Molecular Immunology. W.B. Saunders Company, 2000
- **WELSCHOF et al.** *J Immunol Methods,* 1995, vol. 179, 203-214
- **CHOTHIA et al.** *J Mol Biol,* 1992, vol. 227, 776-798
- **WILLIAMS et al.** *J Mol Biol,* 1996, vol. 264, 220-232
- **MARKS et al.** *Eur J Immunol,* 1991, vol. 21, 985-991
- **TOMLINSON et al.** *EMBO J.,* 1995, vol. 14, 4628-4638
- **SOUTO-CARNEIRO et al.** *J Immunol.,* 2004, vol. 172, 6790-6802
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **DUCHOSAL et al.** *Nature,* 1992, vol. 355, 258
- **MULLIGAN ; BERG.** *Proc. Natl. Acad. Sci. USA,* 1981, vol. 78, 2072
- **SOUTHERN ; BERG.** *Mol. Appl. Genet.,* 1982, vol. 1, 327
- **WIGLER et al.** *Cell,* 1979, vol. 16, 77
- **SARVER et al.** *Proc. Natl. Acad. Sci. USA,* 1982, vol. 79, 7147
- **DEANS et al.** *Proc. Natl. Acad. Sci. USA,* 1984, vol. 81, 1292
- **LUSKY ; BOTCHAN.** *Nature,* 1981, vol. 293, 79
- **HOUDEBINE.** *Curr Opin Biotechnol,* 2002, vol. 13 (6), 625-629
- **VAN KUIK-ROMEIJN et al.** *Transgenic Res,* 2000, vol. 9 (2), 155-159
- **POLLOCK et al.** *J Immunol Methods,* 1999, vol. 231 (1-2), 147-157
- **HOU et al.** *Cytokine,* 1998, vol. 10, 319-30
- Current Protocols in Molecular Biology, Wiley & Sons, and Molecular Cloning - A Laboratory Manual. Cold Spring Harbor Laboratory Press, 2001
- **KASZUBSKA et al.** *Protein Expression and Purification,* 2000, vol. 18, 213-220
- **SCOPES.** Protein Purification. Springer-Verlag, 1994
- **ROGERS et al.** *J. Nucl. Med.,* 1997, vol. 38, 1221-1229
- **WELCH ; REDVANLY.** Handbook of Radiopharmaceuticals: Radiochemistry and Applications. John Wiley and Sons, 2003
- **LEE et al.** *Bioconjug. Chem,* 1999, vol. 10 (6), 973-8
- **KINSTLER et al.** *Advanced Drug Deliveries Reviews,* 2002, vol. 54, 477-485
- **ROBERTS et al.** *Advanced Drug Delivery Reviews,* 2002, vol. 54, 459-476
- **WRIGHT et al.** *EMBO J,* 1991, vol. 10 (10), 2717-2723
- **CO et al.** *Mol Immunol,* 1993, vol. 30, 1361
- **BERGE et al.** *J Pharm Sci,* 1977, vol. 66, 1-19
- **GENNARO.** Remington: The Science and Practice of Pharmacy. Lippincott, Williams & Wilkins, 2000
- **ANSEL et al.** Pharmaceutical Dosage Forms and Drug Delivery Systems. Lippincott Williams & Wilkins, 1999
- **KIBBE.** Handbook of Pharmaceutical Excipients American Pharmaceutical Association. 2000
- **J.R. ROBINSON.** Sustained and Controlled Release Drug Delivery Systems. Marcel Dekker, Inc, 1978
- **EGLITIS et al.** *Science,* 1985, vol. 230, 1395-1398
- **DANOS ; MULLIGAN.** *Proc. Natl. Acad. Sci. USA,* 1988, vol. 85, 6460-6464
- **WILSON et al.** *Proc. Natl. Acad. Sci. USA,* 1988, vol. 85, 3014-3018
- **ARMENTANO et al.** *Proc. Natl. Acad. Sci. USA,* 1990, vol. 87, 6141-6145

- **HUBER et al.** *Proc. Natl. Acad. Sci. USA,* 1991, vol. 88, 8039-8043
- **FERRY et al.** *Proc. Natl. Acad. Sci. USA,* 1991, vol. 88, 8377-8381
- **CHOWDHURY et al.** *Science,* 1991, vol. 254, 1802-1805
- **VAN BEUSECHEM et al.** *Proc. Natl. Acad. Sci. USA,* 1992, vol. 89, 7640-7644
- **KAY et al.** *Human Gene Therapy,* 1992, vol. 3, 641-647
- **DAI et al.** *Proc. Natl. Acad. Sci. USA,* 1992, vol. 89, 10892-10895
- **HWU et al.** *J. Immunol.,* 1993, vol. 150, 4104-4115
- **BERKNER et al.** *BioTechniques,* 1988, vol. 6, 616
- **ROSENFELD et al.** *Science,* 1991, vol. 252, 431-434
- **ROSENFELD et al.** *Cell,* 1992, vol. 68, 143-155
- **FLOTTE et al.** *Am J Respir Cell Mol Biol,* 1992, vol. 7, 349-356
- **SAMULSKI et al.** *J Virol,* 1989, vol. 63, 3822-3828
- **MCLAUGHLIN et al.** *J Virol,* 1989, vol. 62, 1963-1973
- **MIHU et al.** *J Gasrointestin Liver Dis,* 2007, vol. 16 (4), 419-424
- **VAN GURP et al.** *Am J Transplantation,* 2008, vol. 8 (8), 1711-1718
- **HANOUSKA et al.** *Clin Cancer Res,* 2007, vol. 13 (2), 523-531
- **HETHERINGTON et al.** *Antimicrobial Agents and Chemotherapy,* 2006, vol. 50 (10), 3499-3500
- **APPEL et al.** *J Am. Soc. Nephrol.,* 2005, vol. 16, 1392-1404
- **HOLERS.** *Immunological Reviews,* 2008, vol. 223, 300-316
- **HOLERS ; THURMAN.** *Molecular Immunology,* 2004, vol. 41, 147-152
- **LICHT et al.** *Kidney Int.,* 2006, vol. 70, 42-50
- The role of complement in membranoproliferative glomerulonephritis. **ZIPFEL et al.** Complement and Kidney Disease. Springer, 2006, 199-221
- **AULT et al.** *J Biol. Chew,* 1997, vol. 272, 25168-75
- **ABRERA-ABELEDA et al.** *J Med. Genet,* 2006, vol. 43, 582-589
- **POZNANSKY et al.** *J Immunol.,* 1989, vol. 143, 1254-1258
- **JANSEN et al.** *Kidney Int.,* 1998, vol. 53, 331-349
- **HEGASY et al.** *Am J Pathol,* 2002, vol. 161, 2027-2034
- **LEVY et al.** *Nature,* 2001, vol. 413, 488-494
- **KOKAME et al.** *Semin. Hematol,* 2004, vol. 41, 34-40
- **LICHT et al.** *Kidney Int.,* 2004, vol. 66, 955-958
- **NORIS et al.** *J. Am. Soc. Nephrol,* 2005, vol. 16, 1177-1183
- **AVERY et al.** *American Journal of Hematology,* 1998, vol. 58, 148-149
- **GORDON et al.** *Seminars in Hematology,* 1997, vol. 34 (2), 140-147
- **WARWICKER et al.** *Kidney Int.,* 1998, vol. 53, 836-844
- **RICHARDS et al.** *Am J Hum Genet,* 2001, vol. 68, 485-490
- **CAPRIOLI et al.** *Am Soc Nephrol,* 2001, vol. 12, 297-307
- **NEUMAN et al.** *J Med Genet,* 2003, vol. 40, 676-681
- **RICHARDS et al.** *Proc Natl Acad Sci USA,* 2006, vol. 100, 12966-12971
- **FREMEAUX-BACCHI et al.** *J Am Soc Nephrol,* 2005, vol. 17, 2017-2025
- **ESPARZA-GORDILLO et al.** *Hum Mol Genet,* 2005, vol. 14, 703-712
- **GOICOECHEA DE JORGE et al.** *Proc Natl Acad Sci USA,* 2007, vol. 104 (1), 240-245
- **BLOM et al.** *J Immunol.,* 2008, vol. 180 (9), 6385-91
- **FREMEAUX-BACCHI et al.** *J Medical Genet,* 2004, vol. 41, e84
- **CONSTANTINESCU et al.** *Am J Kidney Dis,* 2004, vol. 43, 976-982
- **GEORGE.** *Curr Opin Hematol,* 2003, vol. 10, 339-344
- **GOTTSCHALL et al.** *Am J Hematol,* 1994, vol. 47, 283-289
- **VALAVAARA et al.** *Cancer,* 1985, vol. 55, 47-50
- **MIRALBELL et al.** *J Clin Oncol,* 1996, vol. 14, 579-585
- **DRAGON-DUREY et al.** *J Am Soc Nephrol,* 2005, vol. 16, 555-63
- **BECKER et al.** *Clin Infect Dis,* 2004, vol. 39, S267-S275
- **SAHIN et al.** *Nagoya Med J,* 2001, vol. 44 (3), 145-152
- **SULLIVAN et al.** *Am J Obstet Gynecol,* 1994, vol. 171, 940-943
- **PADDEN et al.** *Am Fam Physician,* 1999, vol. 60 (3), 829-836
- **GERTZ.** *Hematology,* 2006, vol. 1, 19-23
- **HORWITZ et al.** *Blood,* 1977, vol. 50, 195-202
- **FINLAND ; BARNES.** *AMA Arch Intern Med,* 1958, vol. 191, 462-466
- **WANG et al.** *Acta Paediatr Taiwan,* 2004, vol. 45, 293-295
- **MICHAUX et al.** *Ann Hematol,* 1998, vol. 76, 201-204
- **CHANG et al.** *Cancer Genet Cytogenet,* 2004, vol. 152, 66-69

- **DROSOS et al.** *Clin Exp Rheumatol,* 1993, vol. 11 (4), 387-91
- **KAESER et al.** *Acta Neurol Scand Suppl.,* 1984, vol. 100, 39-47
- **SOLTÉSZ et al.** *Haematologia (Budep),* 2000, vol. 30 (4), 303-311
- **IDEGUCHI et al.** *Lupus,* 2007, vol. 16 (1), 59-64
- **MANNER et al.** *Am J Med. Sci.,* 2008, vol. 335 (5), 394-7
- **MIESBACH et al.** *Autoimmune Rev.,* 2006, vol. 6 (2), 94-7
- **GÓMEZ-PUERTA et al.** *Autoimmune Rev.,* 2006, vol. 6 (2), 85-8
- **GÓMEZ-PUERTA et al.** *Semin. Arthritis Rheum,* 2006, vol. 35 (5), 322-32
- **KASAMON et al.** *Haematologia,* 2005, vol. 90 (3), 50-53
- **ATHERSON et al.** *Medicine,* 1998, vol. 77 (3), 195-207
- **CANPOLAT et al.** *Clin Pediatr,* 2008, vol. 47 (6), 593-7
- **TOMSEN.** *Am J Obstet Gynecol,* 1995, vol. 172, 1876-1890
- **SIBAI.** *Am J Obstet Gynecol,* 1986, vol. 162, 311-316
- **AULT et al.** *J Biol. Chem.,* 1997, vol. 272, 25168-75
- **ABRERA-ABELEDA et al.** *J Med Genet,* 2006, vol. 43, 582-589
- **POZNANSKY et al.** *J Immunol,* 1989, vol. 143, 1254-1258
- **WALKER et al.** *Mod. Pathol.,* 2007, vol. 20, 605-616
- **HABIB et al.** *Kidney Int.,* 1975, vol. 7, 204-215
- **SCHWERTZ et al.** *Pediatr Allergy Immunol,* 2001, vol. 12, 166-172
- **COLVILLE et al.** *Am J Kidney Dis.,* 2003, vol. 42, E2-5
- *Curr Opin Hematol,* 2007, vol. 14 (3), 277-283
- **TSAI.** *J. Am. Soc. Nephrol,* 2003, vol. 14, 1072-1081
- **FURLAN et al.** *New Engl J Med,* 1998, vol. 339, 1578-1584
- **MATSUMOTO et al.** *Blood,* 2004, vol. 103, 1305-1310
- **MORI et al.** *Transfusion,* 2002, vol. 42, 572-580
- **DONG et al.** *Am J Hematol,* 2008, vol. 83 (10), 815-817
- **KAPLAN et al.** Hemolytic Uremic Syndrome and Thrombotic Thrombocytopenic Purpura. Informa Health Care, 1992
- **ZIPFEL.** Complement and Kidney Disease. Springer, 2005
- **BRESLIN et al.** *Clin Am Soc Nephrol,* 2006, vol. 1, 88-99
- **GOICOECHEA DE JORGE et al.** *Proc Natl Acad Sci USA,* 2007, vol. 104, 240-245
- **VAN VENROOIJ et al.** *Ann NY Acad Sci,* 2008, vol. 1143, 268-285
- **HABIB et al.** *Immunol Invest,* 2007, vol. 37 (8), 849-857
- **BENUCCI et al.** *Clin Rheumatol,* 2008, vol. 27 (1), 91-95
- **JULKUNEN et al.** *Scan J Rheumatol,* 2005, vol. 34 (2), 122-124
- **MIYAWAKI et al.** *J Rheumatol,* 2005, vol. 32 (8), 1488-1494
- **GLEICHER ; BUTTINO.** Principles & Practice of Medical Therapy in Pregnancy. Appleton & Lange, 1998
- **SETHI et al.** *Neurology,* 1987, vol. 37 (8), 1383-1385
- **HOCH et al.** *Nat Med,* 2001, vol. 7, 365-368
- **VINCENT et al.** *Semin Neurol.,* 2004, vol. 24, 125-133
- **MCCONVILLE et al.** *Ann. Neurol.,* 2004, vol. 55, 580-584
- **BONEVA et al.** *J Neuroimmunol,* 2006, vol. 177, 119-131
- **ROMI et al.** *Arch Neurol.,* 2005, vol. 62, 442-446
- **PASCUZZI.** *Semin Neurol,* 2003, vol. 23 (1), 83-88
- **KATIRJI et al.** *Neurol Clin,* 2002, vol. 20, 557-586
- Guidelines in Electrodiagnostic Medicine. American Association of Electrodiagnostic Medicine. Muscle Nerve. vol. 15, 229-253
- **CHRISTENSON ; DACIE.** *Br J Haematol,* 1957, vol. 3, 153-164
- **CHRISTENSON et al.** *Br J Haematol,* 1957, vol. 3, 262-275
- **LUDWIG et al.** *J Clin. Microbiol,* 2001, vol. 39 (6), 2272-2279
- **SCHOCKETT et al.** *Proc Natl Acad Sci USA,* 1996, vol. 93, 5173-5176
- **AUSUBEL et al.** Current Protocols in Molecular Biology. Greene Publishing Associates, 1992
- **PETKOVA et al.** *Int Immunology,* 2006, vol. 18 (12), 1759-1769
- **OIAO et al.** *Proc Natl Acad Sci USA,* 2008, vol. 105 (27), 9337-9342
- **BRODSKY et al.** *Blood,* 2008, vol. 111 (4), 1840-1847
- **HILLMEN et al.** *N Engl J Med,* 2004, vol. 350 (6), 552-559
- **SISSONS et al.** *J Clin Invest,* 1977, vol. 59, 704-715